# EUROPEAN PATENT APPLICATION

(11) **EP 4 717 179 A1**
(43) Date of publication of application: **01.04.2026**
(21) Application number: 25204605.7
(22) Date of filing: 25.09.2025
(51) Int. Cl.: A61B 6/00, A61B 6/42, A61B 6/51

(54) **PROCESSING DEVICE, IMAGE COMBINATION METHOD, PROGRAM, AND RECORDING MEDIUM**

(30) Priority: 30.09.2024 JP 2024171252
(71) Applicant: J. MORITA MFG. CORP., Fushimi-ku Kyoto-shi Kyoto 612-8533 (JP)
(72) Inventor: Sugihara, Yoshito, Kyoto, 612-8533 (JP); Yoshikawa, Hideki, Kyoto, 612-8533 (JP); Hikosaka, Miho, Kyoto, 612-8533 (JP)
(74) Representative: Gulde & Partner

(57) **Abstract**

A technology is provided that allows for easy and appropriate registration when combining a plurality of radiation images read from a plurality of imaging plates used for intra-oral radiography. A covering member covers a plurality of imaging plates used for intra-oral radiography and is placed in the oral cavity together with the plurality of imaging plates. The covering member includes at least one mark for registration when combining a plurality of radiation images respectively read from the plurality of imaging plates. When the plurality of imaging plates are irradiated with radiation through the covering member, a mark image corresponding to the at least one mark is included in the plurality of radiation images.

## Description

### Technical Field

The present disclosure relates to an imaging plate used for intra-oral radiography.

### Background Art

Patent Literature 1 discloses a technique for performing imaging in the oral cavity using a plurality of imaging plates.

### Citation List

### Patent Literature

[Patent Literature 1] Japanese Unexamined Patent Application Publication No. 2011-212401

### Summary of Invention

### Technical Problem

When a plurality of imaging plates are used simultaneously for imaging in the oral cavity, a plurality of radiation images respectively read from the plurality of imaging plates may be combined to generate a composite image, and the generated composite image may be displayed.

Furthermore, in conventional intra-oral imaging using a plurality of imaging plates, when generating a composite image from a plurality of pieces of image data, it was necessary for a program to comprehensively search for feature points of each tooth, which is the subject, and to perform an image data combination (synthesis) process using these feature points as clues.

Such processing required a complex program and tended to take a long time. Furthermore, when the subject's oral cavity is an edentulous jaw, for example, there are few anatomical cues, resulting in a scarcity of feature points, which made the combination process extremely difficult.

Furthermore, in dental radiography using an imaging plate, when performing imaging over a relatively wide range in the oral cavity (for example, occlusal radiography), if a large size is selected for the imaging plate to be used, a reading device equipped with a larger sensor than usual is required for reading the large-sized imaging plate.

The present disclosure aims to provide a technology capable of easily, quickly, and appropriately performing registration when combining a plurality of radiation images read from a plurality of imaging plates used in intra-oral radiography, and also aims to provide a technology that can handle image processing by various imaging methods using a commonly used, conventional imaging plate reading device.

### Solution to Problem

One aspect of a covering member covers a plurality of imaging plates used for intra-oral radiography and is placed in the oral cavity together with the plurality of imaging plates.

The one aspect of the covering member includes at least one mark for registration when combining a plurality of radiation images respectively read from the plurality of imaging plates.

When the plurality of imaging plates are irradiated with radiation through the covering member, a mark image corresponding to the at least one mark is included in the plurality of radiation images.

One aspect of a processing device includes an image processing unit that performs registration of a plurality of radiation images read from the plurality of imaging plates, based on a mark image included in the plurality of radiation images, and combines the plurality of radiation images after the registration.

One aspect of a program is a program for causing a computer device to execute a process of performing registration of a plurality of radiation images read from the plurality of imaging plates, based on a mark image included in the plurality of radiation images, and a process of combining the plurality of radiation images after the registration.

One aspect of an image combination method performs registration of a plurality of radiation images read from the plurality of imaging plates, based on a mark image included in the plurality of radiation images, and combines the plurality of radiation images after the registration.

Registration when combining a plurality of radiation images can be performed easily, quickly, and appropriately based on a mark image included in the plurality of radiation images. Furthermore, for example, it becomes possible to realize diverse imaging corresponding to various cases by using imaging plates of a relatively small size.

### Brief Description of Drawings

FIG. 1 is a schematic diagram illustrating an example of an imaging plate assembly.
FIG. 2 is a schematic diagram illustrating an example of an imaging plate assembly.
FIG. 3 is a schematic diagram illustrating an example of an imaging plate assembly.
FIG. 4 is a schematic diagram illustrating an example of an imaging plate assembly.
FIG. 5 is a schematic diagram illustrating an example of an imaging plate assembly.
FIG. 6 is a schematic diagram illustrating an example of an imaging plate assembly.
FIG. 7 is a schematic diagram illustrating an example of an imaging plate assembly.
FIG. 8 is a schematic diagram illustrating an example of an imaging plate assembly.
FIG. 9 is a schematic diagram illustrating an example of an imaging plate assembly.
FIG. 10 is a schematic diagram illustrating an example of a reading device.
FIG. 11 is a schematic diagram illustrating an example of a reading device.
FIG. 12 is a schematic diagram illustrating an example of a reading device.
FIG. 13 is a schematic diagram illustrating an example of a reading device.
FIG. 14 is a schematic diagram illustrating an example of the configuration of a control unit.
FIG. 15 is a schematic diagram for explaining an example of the operation of the image processing unit.
FIG. 16 is a schematic diagram illustrating an example of a holding member.
FIG. 17 is a schematic diagram illustrating an example of a radiation image recorded on an imaging plate.
FIG. 18 is a schematic diagram illustrating an example of a read radiation image.
FIG. 19 is a schematic diagram illustrating an example of a first layout diagram.
FIG. 20 is a schematic diagram for explaining an example of the operation of the image processing unit.
FIG. 21 is a schematic diagram for explaining an example of the operation of the image processing unit.
FIG. 22 is a schematic diagram for explaining an example of a composite image.
FIG. 23 is a schematic diagram illustrating an example of a radiation image recorded on an imaging plate.
FIG. 24 is a schematic diagram for explaining an example of the operation of the image processing unit.
FIG. 25 is a schematic diagram for explaining an example of a composite image.
FIG. 26 is a schematic diagram illustrating an example of a holding member.
FIG. 27 is a schematic diagram illustrating an example of a holding member.
FIG. 28 is a schematic diagram illustrating an example of a holding member.
FIG. 29 is a schematic diagram illustrating an example of a holding member.
FIG. 30 is a schematic diagram illustrating an example of a radiation image recorded on an imaging plate.
FIG. 31 is a schematic diagram for explaining an example of the operation of the image processing unit.
FIG. 32 is a schematic diagram illustrating an example of a holding member.
FIG. 33 is a schematic diagram illustrating an example of a holding member.
FIG. 34 is a schematic diagram illustrating an example of a holding member.
FIG. 35 is a schematic diagram illustrating an example of a holding member.
FIG. 36 is a schematic diagram illustrating an example of a second layout diagram.
FIG. 37 is a schematic diagram for explaining an example of the operation of the image processing unit.
FIG. 38 is a schematic diagram illustrating an example of a read radiation image.
FIG. 39 is a schematic diagram illustrating an example of a holding member.
FIG. 40 is a schematic diagram for explaining an example of the operation of the image processing unit.
FIG. 41 is a schematic diagram illustrating an example of a holding member.
FIG. 42 is a schematic diagram illustrating an example of a read radiation image.
FIG. 43 is a schematic diagram for explaining an example of the operation of the image processing unit.
FIG. 44 is a schematic diagram for explaining an example of the operation of the image processing unit.
FIG. 45 is a schematic diagram illustrating an example of a holding member.
FIG. 46 is a schematic diagram illustrating an example of a holding member.
FIG. 47 is a schematic diagram for explaining an example of the operation of the image processing unit.
FIG. 48 is a schematic diagram illustrating an example of registration of read radiation images.
FIG. 49 is a schematic diagram illustrating an example of a holding member.
FIG. 50 is a schematic diagram illustrating an example of a read radiation image.
FIG. 51 is a schematic diagram illustrating an example of a holding member.
FIG. 52 is a schematic diagram illustrating an example of a holding member.
FIG. 53 is a schematic diagram illustrating an example of a holding member.
FIG. 54 is a schematic diagram illustrating an example of a holding member.
FIG. 55 is a schematic diagram illustrating an example of a holding member.
FIG. 56 is a schematic diagram illustrating an example of a holding member.
FIG. 57 is a schematic diagram illustrating an example of a holding member.
FIG. 58 is a schematic diagram illustrating an example of a holding member.
FIG. 59 is a schematic diagram illustrating an example of a holding member.
FIG. 60 is a schematic diagram illustrating an example of a holding member.
FIG. 61 is a schematic diagram illustrating a display example of a composite image.
FIG. 62 is a schematic diagram illustrating an arrangement example of imaging plates.
FIG. 63 is a schematic diagram illustrating an example of a radiation image recorded on an imaging plate.
FIG. 64 is a schematic diagram illustrating an example of a read radiation image.
FIG. 65 is a schematic diagram illustrating an example of a composite image.
FIG. 66 is a schematic diagram illustrating an example of a holding member.
FIG. 67 is a schematic diagram illustrating an example of a holding member.
FIG. 68 is a schematic diagram illustrating an example of a read radiation image.
FIG. 69 is a schematic diagram illustrating an example of a read radiation image.
FIG. 70 is a schematic diagram illustrating an example of a holding member.
FIG. 71 is a schematic diagram illustrating an example of a holding member.
FIG. 72 is a schematic diagram illustrating an example of a read radiation image.
FIG. 73 is a schematic diagram illustrating an example of a read radiation image.
FIG. 74 is a schematic diagram illustrating an example of a holding member.
FIG. 75 is a schematic diagram illustrating an example of a holding member.
FIG. 76 is a schematic diagram illustrating an example of a holding member.
FIG. 77 is a schematic diagram illustrating an example of an imaging plate assembly.
FIG. 78 is a schematic diagram illustrating an example of an imaging plate assembly.
FIG. 79 is a schematic diagram illustrating an example of an imaging plate assembly.
FIG. 80 is a schematic diagram illustrating an example of a state where a reading device and a display unit are connected.
FIG. 81 is a schematic diagram illustrating an example of a state where a reading device and a processing device are connected.

### Description of Embodiments

### <Example of Configuration of Imaging Plate Assembly>

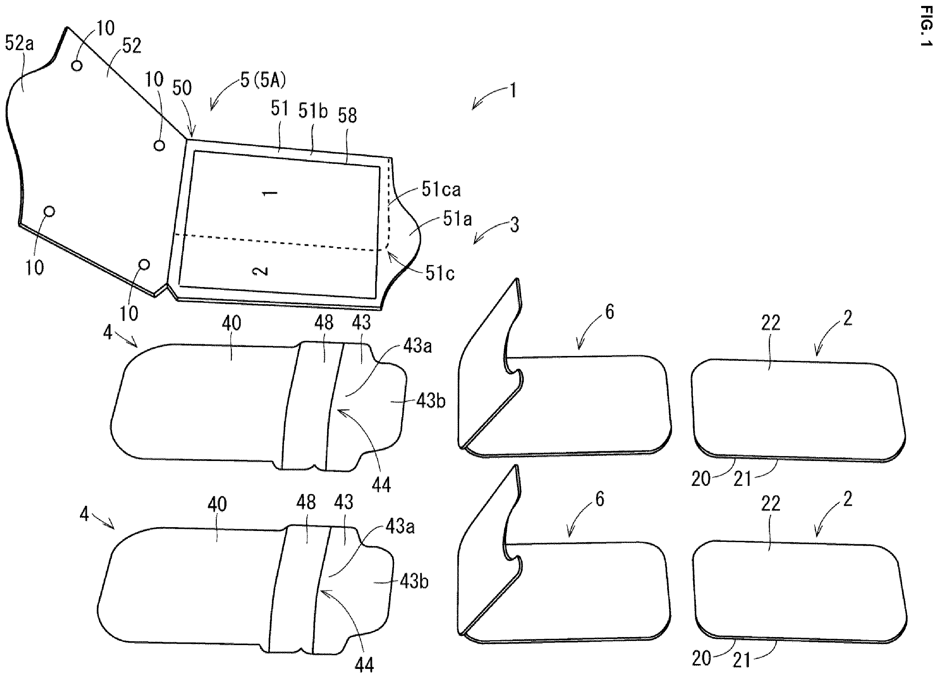

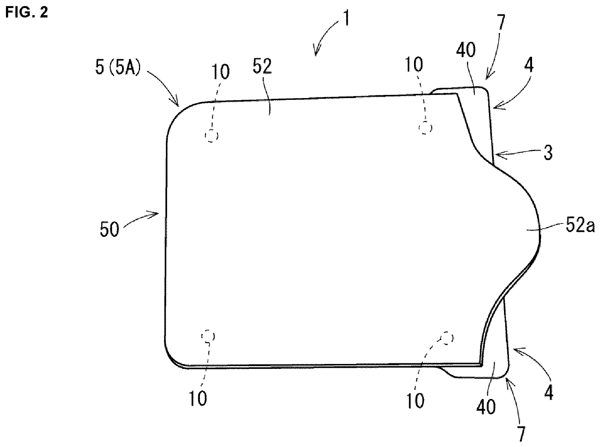

FIGS. 1 and 2 are schematic views illustrating an example of an imaging plate assembly 1 (also referred to as an IP assembly 1) comprising a plurality of imaging plates 2. FIG. 1 illustrates an example of the IP assembly 1 before assembly, and FIG. 2 illustrates an example of the IP assembly 1 after assembly. The IP assembly 1 is, for example, placed in a person's oral cavity and is used for imaging of the oral cavity. The IP assembly 1 is used, for example, in occlusal radiography. Note that the use of the IP assembly 1 is not limited to this.

As shown in FIGS. 1 and 2, an IP assembly 1 comprises, for example, a pluralit y of imaging plates 2 used for imaging of the oral cavity, and an imaging plate protection assembly 3 capable of accommodating the plurality of imaging plates 2. In the example of FIGS. 1 and 2, the number of the imaging plates 2 is two, but the number of the imagin g plates 2 may be three or more. Note that the IP assembly 1 can also be used in a sta te of accommodating a single imaging plate 2.

An imaging plate protection assembly 3 (also referred to as a protection assembly 3) comprises, for example, a plurality of waterproof bags 4 for respectively and individually accommodating a plurality of imaging plates 2, and a holding member 5 for holding the plurality of imaging plates 2 accommodated in the plurality of waterproof bags 4 at a predetermined position. Furthermore, the protection assembly 3 also comprises, for example, a plurality of protection covers 6 for respectively covering the plurality of imaging plates 2. A waterproof bag 4 accommodates an imaging plate 2 covered by a protection cover 6.

The imaging plate 2 has a flat shape with a radiation image forming layer 20 and is a recording medium for recording a radiation image. The imaging plate 2 has a front surface where the radiation image forming layer 20 is located, and a back surface opposite to the front surface. The imaging plate 2 has, for example, a substantially rectangular flat shape with rounded corners. The radiation image forming layer 20 is a layer that accumulates the energy of irradiated radiation and emits stimulated luminescence corresponding to the accumulated energy. The imaging plate 2 has a film formed of a resin. The film has a front surface on which the radiation image forming layer 20 is provided, and a back surface opposite to the front surface. The radiation image forming layer 20 is composed of, for example, a stimulable phosphor coated on the front surface of the film. As the radiation applied to the radiation image forming layer 20, for example, an X-ray is adopted. When an X-ray from an X-ray generator, which is a radiation source, passes through an object (also referred to as a subject) and irradiates the imaging plate 2, energy corresponding to the intensity of the X-ray on the imaging plate 2 is accumulated in the radiation image forming layer 20. Since the intensity of the X-ray is based on the distribution of an X-ray absorbing region in the object, the distribution of the energy accumulated in the radiation image forming layer 20 is a radiation image of the object by the X-ray. In this way, the imaging plate 2 records, for example, a radiation image by an X-ray as a latent image.

FIG. 2 shows an example of a state in which the plurality of imaging plates 2 respectively accommodated in the plurality of waterproof bags 4 are held by the holding member 5. An IP assembly 1, in the state of FIG. 2, is placed, for example, in a human oral cavity. There are, for example, a plurality of types of sizes for the imaging plate 2. The type and number of the imaging plates 2 used for radiography are appropriately selected according to the object.

In dental radiography using an imaging plate, generally, a small-sized imaging plate tends to be used more often than a large-sized imaging plate. This tendency is due to the background that there are many issues and high technical hurdles in scanning a large-sized imaging plate using a general detector conventionally used in the field of dental radiography. Conversely, when mounting a detector capable of scanning a large-sized imaging plate, the device becomes large or the cost of the device itself becomes high, or it becomes over-specified for detecting a commonly used (small-sized) imaging plate. Therefore, if the same radiography as when using a large-sized imaging plate can be performed using a small-sized imaging plate, various dental radiographies become possible even with a conventional device, and its applications will be expanded.

Note that the small-sized imaging plate and the large-sized imaging plate mentioned here are expressions for convenience to show the distinction between a size that can be read by a normal scan with a conventionally used general detector and a size that cannot. For example, the small-sized imaging plate includes an imaging plate of a size called "size 0", an imaging plate of a size called "size 1", an imaging plate of a size called "size 2", and an imaging plate of a size called "size 3". Also, for example, the large-sized imaging plate includes an imaging plate of a size larger than "size 3". In dental radiography, a "size 0" imaging plate is generally used for standard radiography of children, etc., and a "size 1" imaging plate is generally used for bitewing radiography of children, etc. A "size 2" imaging plate is generally used for standard radiography of adults, etc., and a "size 3" imaging plate is generally used for bitewing radiography of adults, etc. The large-sized imaging plate includes imaging plates such as "size 4" and "size 5", and is generally used for occlusal radiography of children and adults in dental radiography.

When a plurality of imaging plates 2 are used, as in the IP assembly 1 disclosed in the embodiment of FIG. 1 and the like, large-area occlusal radiography, which conventionally required preparing an imaging plate of a size larger than size 3, becomes possible even with a small-sized imaging plate.

In intra-oral radiography, with the IP assembly 1 placed in the oral cavity, radiation is applied to the radiation image forming layer 20 of each imaging plate 2 included in the IP assembly 1. On the radiation image forming layer 20 of each imaging plate 2 of the IP assembly 1, a subject (in other words, an object) in the oral cavity is recorded. On the radiation image forming layer 20, for example, at least one tooth (for example, a dentition forming a part of a dental arch), a soft tissue such as a gingiva, and a jawbone are recorded as the subject. Note that when the subject includes an edentulous jaw, no tooth is recorded on the radiation image forming layer 20. Further, when the IP assembly 1 including the plurality of imaging plates 2 is placed in the oral cavity, not all of the IP assembly 1 may fit in the oral cavity, and a part of the IP assembly 1 may be located outside the oral cavity. That is, a part of the holding member 5 on the outside of the IP assembly 1 may be located outside the oral cavity.

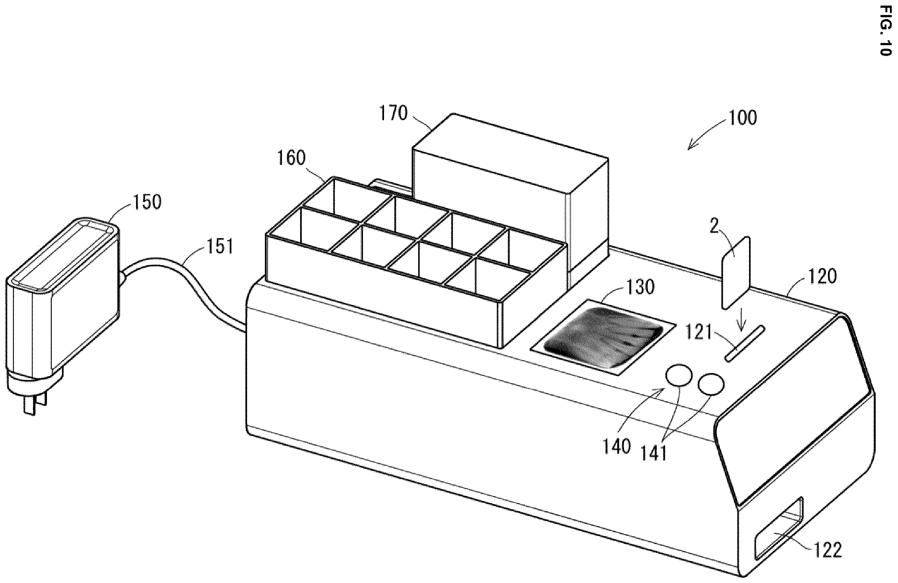

The radiation image recorded on the imaging plate 2 is read by a reading device 100 (see FIG. 10) to be described later. Into the reading device 100, for example, each imaging plate 2 included in the IP assembly 1 is inserted one by one. The reading device 100 reads the radiation image from the radiation image forming layer 20 of the inserted imaging plate 2. In a radiation image read from each imaging plate 2 (also referred to as a read radiation image), a subject such as a dentition forming a part of a dental arch is captured. The reading device 100 combines (in other words, synthesizes) a plurality of radiation images read from the plurality of imaging plates 2 included in the IP assembly 1 to generate a single composite image showing the state of the oral cavity. In the composite image, a subject such as a dental arch is captured. The composite image is sometimes called an occlusal image. The reading device 100 causes, for example, a display unit included in the reading device 100 to display the generated composite image. Hereinafter, the front surface of the imaging plate 2 is referred to as a reading surface 21. Furthermore, the back surface of the imaging plate 2 is referred to as a non-reading surface 22.

The protection cover 6 is a member that protects the imaging plate 2. The protection cover 6 has, for example, a structure in which a sheet-like member is bent, and can be opened and closed. The protection cover 6 can cover the imaging plate 2 by sandwiching it. For example, in the protection cover 6, at least a portion that comes into contact with the reading surface 21 of the imaging plate 2 is made of a radiation-transmissive material. Furthermore, the protection cover 6 is made of, for example, a material having a light-shielding property against visible light. The protection cover 6 is made of, for example, paper.

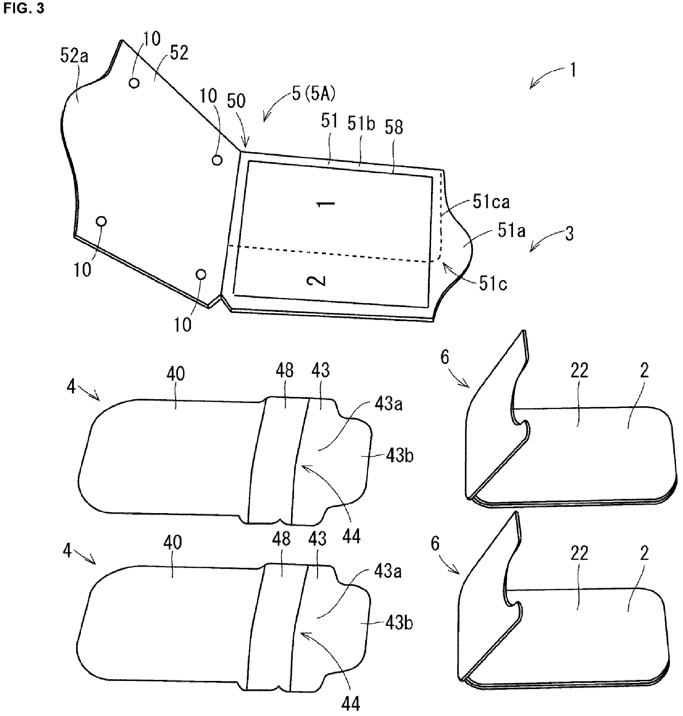

For example, the protection cover 6 is in an open state as shown in FIG. 1 when no external force is applied to it. On the other hand, when the protection cover 6 is sandwiched by fingers from the outside, the protection cover 6 enters a closed state, and the imaging plate 2 is sandwiched by the protection cover 6. FIG. 3 is a schematic diagram showing an example of a state in which the imaging plate 2 is placed on an inner surface of the protection cover 6 in the open state.

Thus, by being covered with the protection cover 6, the imaging plate 2 becomes less likely to be mechanically damaged. For example, during dental radiography, when the IP assembly 1 placed in the oral cavity is bitten by a person's teeth, or when it is bent along a dentition and comes into contact with the dentition, the imaging plate 2 becomes less likely to be damaged. The protection cover 6 is also called, for example, a bite cover, an IP cover, or a carton.

Further, since the imaging plate 2 is covered with the protection cover 6 made of a material such as paper that hardly transmits visible light, visible light or external light emitted from a surrounding lighting device (for example, a fluorescent lamp or an LED (light-emitting diode)) is less likely to hit the imaging plate 2. Therefore, information of the radiation image is less likely to be lost from the imaging plate 2.

The configuration of the protection cover 6 is not limited to the above example. For example, the protection cover 6 may be in a closed state when no external force is applied. Furthermore, the protection cover 6 may be made of a material other than paper. Furthermore, the protection assembly 3 may not include the protection cover 6.

The waterproof bag 4 is a protective material for the imaging plate 2 and the protection cover 6. The waterproof bag 4 accommodates the imaging plate 2 in a state of being covered by the protection cover 6. Like the protection cover 6, the waterproof bag 4 can also be said to be a covering member that covers the imaging plate 2.

The waterproof bag 4 is configured such that a fluid such as a body fluid (saliva, blood, virus, bacteria, etc.) does not penetrate or flow into the waterproof bag 4, so that such fluids do not adhere to the imaging plate 2 and the protection cover 6 placed in the oral cavity during radiography. Further, when washing the entire waterproof bag 4 after radiography, the waterproof bag 4 is configured to prevent a disinfectant or the like from entering the inside of the waterproof bag 4. The waterproof bag 4 is also called, for example, a protection bag, an IP protection bag, or a hygienic pouch.

The waterproof bag 4 comprises, for example, a bag-shaped main body portion 40 having waterproofness, and an adhesive member 48 for sealing the main body portion 40. The adhesive member 48 is provided on the main body portion 40. The adhesive member 48 is made of, for example, a radiation-transmissive material. The adhesive member 48 is composed of, for example, a double-sided tape. The adhesive member 48 has an adhesive surface to which a part of the main body portion 40 is attached. In an unused waterproof bag 4, for example, the adhesive surface of the adhesive member 48 is covered with a release paper or a release film (also called a protective sheet).

The main body portion 40 is, for example, a hygienic member and is made of a biocompatible material. Furthermore, the main body portion 40 is made of, for example, a radiation-transmissive material. The main body portion 40 is made of, for example, a soft material such as a resin. The main body portion 40 may be made of a resin such as PVC (Polyvinyl Chloride) or EVA (Ethylene-Vinyl Acetate Copolymer).

The main body portion 40 has an opening 44 through which the imaging plate 2 is inserted and removed, and a lid portion 43 capable of closing the opening 44. The lid portion 43 has a portion to be attached 43a that is attached to the adhesive surface of the adhesive member 48, and a tab portion 43b. An operator assembling the IP assembly 1 holds the tab portion 43b of the lid portion 43 with fingers, bends the lid portion 43 toward the adhesive member 48, and attaches the portion to be attached 43a to the adhesive surface of the adhesive member 48.

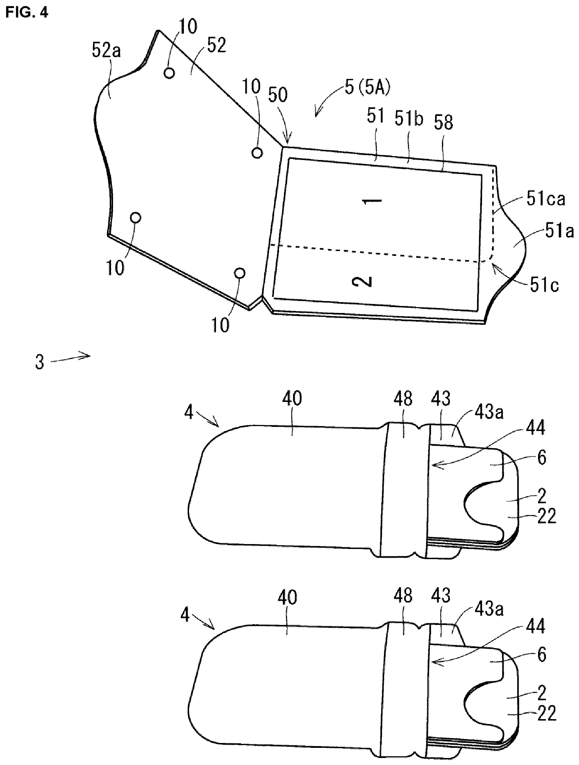

The imaging plate 2 is, for example, sandwiched by the protection cover 6, inserted into the main body portion 40 through the opening 44, and accommodated therein. The operator holds the protection cover 6 (see FIG. 3) with the imaging plate 2 placed on its inner surface by sandwiching it from the outside with fingers, and inserts the imaging plate 2 and the protection cover 6 into the main body portion 40 through the opening 44. FIG. 4 is a schematic diagram showing an example of a state in which the imaging plate 2 and the protection cover 6 are inserted into the main body portion 40 through the opening 44.

After the imaging plate 2 and the protection cover 6 are accommodated in the main body portion 40, the release paper or release film covering the adhesive surface of the adhesive member 48 is removed. Then, the operator holds the tab portion 43b of the lid portion 43 with fingers, bends the lid portion 43 toward the adhesive member 48, and attaches the portion to be attached 43a of the lid portion 43 to the adhesive surface of the adhesive member 48. Thereby, the opening 44 is covered by the lid portion 43, and the main body portion 40 is sealed in a watertight manner. As a result, the assembly of an assembly 7 (also referred to as a unit assembly 7), which consists of the imaging plate 2, the protection cover 6 covering it, and the waterproof bag 4 accommodating the imaging plate 2 and the protection cover 6, is completed.

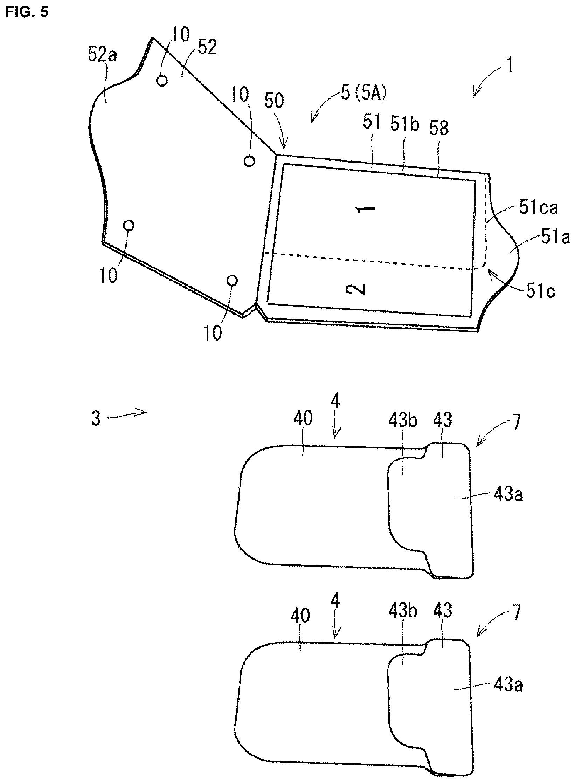

FIG. 5 is a schematic diagram showing an example of a state in which the opening 44 is covered by the lid portion 43 and the main body portion 40 is sealed. Two unit assemblies 7 are shown in FIG. 5. The operator assembles a plurality of unit assemblies 7. If the protection assembly 3 does not include the protection cover 6, the unit assembly 7 does not include the protection cover 6.

Thus, by being accommodated in the waterproof bag 4, the imaging plate 2 is less likely to be contaminated with body fluids such as saliva or blood when the IP assembly 1 is placed in the oral cavity. This makes cross-infection less likely to occur.

Note that, instead of the adhesive member 48, for example, a curable adhesive may be used. Furthermore, the lid portion 43 may be bent and welded to the main body portion 40. Furthermore, the waterproof bag 4 may not include the lid portion 43 and may be composed only of the bag-shaped main body portion 40. In this case, the adhesive member 48 may be provided on the inner surface of the main body portion 40, a vicinity of the opening 44 of the main body portion 40 may be welded by heat, or the opening 44 may be closed by a zipper-type structure like a freezer bag.

At least a part of the main body portion 40 of the waterproof bag 4 may have a light-shielding property against visible light. In this case, for example, since visible light or external light emitted from a surrounding lighting device is less likely to hit the imaging plate 2, information of the radiation image is less likely to be lost from the imaging plate 2. As a method for providing at least a part of the main body portion 40 with a light-shielding property against visible light, for example, the surface of at least a part of the main body portion 40 may be colored black. In this case, for example, of the main body portion 40, at least one of an outer surface or an inner surface of a portion facing the reading surface 21 of the imaging plate 2 in the main body portion 40 may be black. This makes it difficult for ambient visible light to hit the imaging plate 2, particularly the radiation image forming layer 20. Further, of the main body portion 40, at least one of an outer surface or an inner surface of a portion facing the non-reading surface 22 of the imaging plate 2 in the main body portion 40 may be black. Even in this case, ambient visible light is less likely to hit the imaging plate 2. Further, at least one of both surfaces of the lid portion 43 may be black. In this case, ambient visible light is less likely to hit the imaging plate 2. It is preferable that the assembly work of the unit assembly 7 is performed such that at least the light-shielding portion of the waterproof bag 4 and the reading surface 21 of the imaging plate 2 face each other.

The holding member 5 holds the plurality of unit assemblies 7 at a predetermined position. The holding member 5 is also called, for example, a positioning tool, an IP positioning tool, or a container. The holding member 5 holds the plurality of unit assemblies 7 such that, for example, the plurality of unit assemblies 7 are arranged in a line in a predetermined direction. **It** can also be said that the holding member 5 holds the plurality of imaging plates 2 such that the plurality of imaging plates 2 are arranged in a line in the predetermined direction. Furthermore, it can also be said that the holding member 5 holds the plurality of waterproof bags 4 such that the plurality of waterproof bags 4 are arranged in a line in the predetermined direction. The holding member 5 holds the plurality of unit assemblies 7 so as to cover them. It can also be said that the holding member 5 holds the plurality of imaging plates 2 so as to cover them. The holding member 5 can also be said to be a covering member that covers the plurality of imaging plates 2. Hereinafter, the plurality of imaging plates 2 held by the holding member 5 may be referred to as a combined imaging plate. The combined imaging plate can also be regarded as one large imaging plate composed of the plurality of imaging plates 2.

The holding member 5 holds the plurality of imaging plates 2 such that two adjacent imaging plates 2 partially overlap. For example, the holding member 5 holds the plurality of imaging plates 2 such that short-side end portions (in other words, long-side end portions) of two adjacent imaging plates 2 overlap each other. Two imaging plates 2 held by the holding member 5 are arranged along their short-side direction.

In this disclosure, a plurality of types of the holding member 5 will be described. Hereinafter, an example of the holding member 5 shown in FIGS. 1 to 5 is referred to as a holding member 5A.

The holding member 5A is openable and closable. FIG. 1 shows the holding member 5A in an open state, and FIG. 2 shows the holding member 5A in a closed state. The holding member 5A comprises, for example, a base member 50 that covers the plurality of unit assemblies 7, and an attachment portion 58 for attaching the plurality of unit assemblies 7 to the base member 50. The base member 50 covers the plurality of waterproof bags 4. The base member 50 can also be referred to as a main body portion.

The base member 50 has, for example, a structure in which a sheet-like member is bent, and can be opened and closed. The base member 50 has, for example, a fixed portion 51 where the plurality of unit assemblies 7 are placed and fixed, and a cover portion 52 that covers the plurality of unit assemblies 7 fixed to the fixed portion 51. The plurality of unit assemblies 7 are fixed to the fixed portion 51 by the attachment portion 58. The waterproof bags 4 of the plurality of unit assemblies 7 are fixed to the fixed portion 51. The cover portion 52 covers the plurality of waterproof bags 4 fixed to the fixed portion 51.

Each of the fixed portion 51 and the cover portion 52 has, for example, a sheet-like shape. The cover portion 52 is connected to the fixed portion 51 and can be opened and closed relative to the fixed portion 51 with a connection point with the fixed portion 51 as a base point (in other words, an opening/closing axis).

When no external force is applied to the base member 50, for example, the cover portion 52 is in a relatively open state with respect to the fixed portion 51, and the base member 50 is in an open state. Then, when an external force is applied to at least one of the fixed portion 51 and the cover portion 52 such that the fixed portion 51 and the cover portion 52 approach each other, the base member 50 enters a closed state, and the plurality of unit assemblies 7 are sandwiched between the fixed portion 51 and the cover portion 52. For example, when the fixed portion 51 and the cover portion 52 are sandwiched by fingers from their outside, the base member 50 enters a closed state. In a state where the plurality of unit assemblies 7 are sandwiched between the fixed portion 51 and the cover portion 52, the base member 50 faces both surfaces (that is, the reading surface 21 and the non-reading surface 22) of the imaging plate 2 of the plurality of unit assemblies 7. Note that the base member 50 may be in a closed state when no external force is applied.

The fixed portion 51 and the cover portion 52 respectively have a pair of tab portions 51a and 52a. The tab portion 51a is provided at an end of the fixed portion 51 opposite to a connection end with the cover portion 52. The tab portion 52a is provided at an end of the cover portion 52 opposite to a connection end with the fixed portion 51. When the base member 50 is in the closed state, the pair of tab portions 51a and 52a face each other. The operator can maintain the closed state of the base member 50 by holding the pair of tab portions 51a and 52a with fingers from their outside. When the base member 50 is in the closed state with the unit assembly 7 fixed to the fixed portion 51, the imaging plate 2 of the unit assembly 7 is not located between the pair of tab portions 51a and 52a. Note that the holding member 5 may not include the tab portions 51a and 52a.

The base member 50 is, for example, a hygienic member and is made of a biocompatible material (for example, a material compliant with ISO standard 10993-1, etc.). Furthermore, the base member 50 is made of, for example, a radiation-transmissive material. The base member 50 is made of, for example, a soft material such as a resin. The base member 50 may be made of a resin such as PVC or EVA. The base member 50 may be made of the same material as the main body portion 40 of the waterproof bag 4, or may be made of a different material from the main body portion 40. Furthermore, the base member 50 may be made of paper instead of a resin. Furthermore, the base member 50 may be made of paper and a resin. In this case, for example, the base member 50 may be configured by coating a base material (also referred to as a main body) made of paper with a resin such as vinyl. As the material for the base member 50, various other materials with high radiation transmissivity, such as a carbon fiber material, can be used.

On an inner surface 51b of the fixed portion 51, that is, a surface to be fixed 51b to which the plurality of unit assemblies 7 are fixed, placement location information 51c indicating the placement locations of the plurality of unit assemblies 7 is shown by printing or the like. The placement location information 51c indicates the placement locations of the plurality of waterproof bags 4 respectively accommodating the plurality of imaging plates 2. The placement location information 51c can also be said to indicate a layout of the plurality of unit assemblies 7 on the surface to be fixed 51b, and can also be said to indicate a layout of the plurality of imaging plates 2 on the surface to be fixed 51b.

In the example of FIGS. 1 to 5, the placement location information 51c includes numbers 1 and 2 indicating that two unit assemblies 7 are to be placed on the surface to be fixed 51b. The arrangement direction of the numbers 1 and 2 indicates the arrangement direction of the two unit assemblies 7. A location indicated by the number 1 indicates an approximate placement location of one unit assembly 7, and a location indicated by the number 2 indicates an approximate placement location of the other unit assembly 7. It can also be said that the location indicated by the number 1 indicates an approximate placement location of the imaging plate 2 of the one unit assembly 7, and the location indicated by the number 2 indicates an approximate placement location of the imaging plate 2 of the other unit assembly 7.

Furthermore, the placement location information 51c also includes a dotted line 51ca that indicates the detailed placement location of one of the unit assemblies 7. The dotted line 51ca is shown around the number 1. The dotted line 51ca has a shape corresponding to the shape of the unit assembly 7. It can also be said that the dotted line 51ca has a shape corresponding to the shape of the imaging plate 2. It can also be said that the dotted line 51ca is information indicating the detailed placement location of the imaging plate 2 of one of the unit assemblies 7.

Note that the configuration of the placement location information 51c is not limited to the above example. The placement location information 51c may indicate the positions of three or more unit assemblies 7, or it may indicate the arrangement direction of three or more unit assemblies 7. In other words, the placement location information 51c may indicate the layout of three or more unit assemblies 7 on the surface to be fixed 51b. Furthermore, the placement location information 51c may be indicated using lines other than dotted lines. Furthermore, the placement location information 51c may be indicated using at least one of multiple types of marks such as lines, symbols, characters, and figures. The placement location information 51c may have any configuration as long as it can at least show a user the arrangement (in other words, the layout) of each imaging plate 2 that is suitable for the radiography method in which the IP assembly 1 is used.

The attachment portion 58 for attaching the fixed portion 51 and the plurality of waterproof bags 4 to each other is made of, for example, a radiation-transmissive material. Before the plurality of unit assemblies 7 are fixed to the fixed portion 51, that is, in an unused holding member 5A, the attachment portion 58 is provided, for example, on the surface to be fixed 51b. The attachment portion 58 is, for example, an adhesive member. As this adhesive member, for example, a translucent or transparent double-sided tape is adopted. In an unused holding member 5A, a release paper or a release film may be attached to the attachment portion 58. The attachment portion 58 is provided on at least a part of the surface to be fixed 51b. In the example of FIGS. 1 to 5, the attachment portion 58 is provided on most of the surface to be fixed 51b. The placement location information 51c is visible through the attachment portion 58. Each of the plurality of unit assemblies 7 is, for example, detachable from the surface to be fixed 51b.

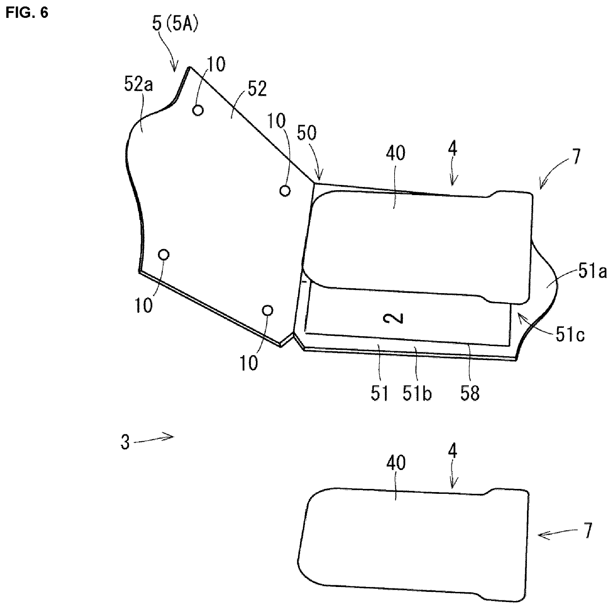

For example, when two unit assemblies 7 are fixed to the fixed portion 51 of the holding member 5A, first, one unit assembly 7 is placed and fixed at a location indicated by the number 1 on the surface to be fixed 51b. Specifically, the one unit assembly 7 is placed and fixed in a region indicated by the dotted line 51ca around the number 1 on the surface to be fixed 51b. In detail, the one unit assembly 7 is placed in a substantially rectangular region surrounded by the dotted line 51ca shown on the surface to be fixed 51b and an outer edge of the surface to be fixed 51b connected to the dotted line 51ca. FIG. 6 is a schematic diagram showing an example of this state. The unit assembly 7 is fixed to the surface to be fixed 51b such that, for example, the non-reading surface 22 of the imaging plate 2 included in the unit assembly 7 is positioned on the side of the surface to be fixed 51b. Further, the unit assembly 7 is fixed to the surface to be fixed 51b such that, for example, a bottom of the waterproof bag 4 of the unit assembly 7 is positioned on a side of a connection end between the cover portion 52 and the fixed portion 51, and the lid portion 43 of the waterproof bag 4 is positioned on a side of the tab portion 51a.

Here, on the surface to be fixed 51b, a direction along a direction from the tab portion 51a toward the connection end between the cover portion 52 and the fixed portion 51 (a substantially left and right direction in FIG. 1) is defined as a vertical direction of the surface to be fixed 51b, and a direction perpendicular to the vertical direction is defined as a horizontal direction of the surface to be fixed 51b. When the unit assembly 7 is placed in the region indicated by the dotted line 51ca on the surface to be fixed 51b, a longitudinal direction of the unit assembly 7 becomes aligned with the vertical direction of the surface to be fixed 51b. Therefore, a longitudinal direction of the imaging plate 2 becomes aligned with the vertical direction of the surface to be fixed 51b.

After the one unit assembly 7 is fixed to the surface to be fixed 51b, the other unit assembly 7 is placed and fixed on the surface to be fixed 51b. Specifically, the other unit assembly 7 is placed and fixed at a location indicated by the number 2 on the surface to be fixed 51b. At this time, similar to the one unit assembly 7 already fixed to the surface to be fixed 51b, the other unit assembly 7 is placed on the surface to be fixed 51b such that a longitudinal direction of the other unit assembly 7 is aligned with the vertical direction of the surface to be fixed 51b.

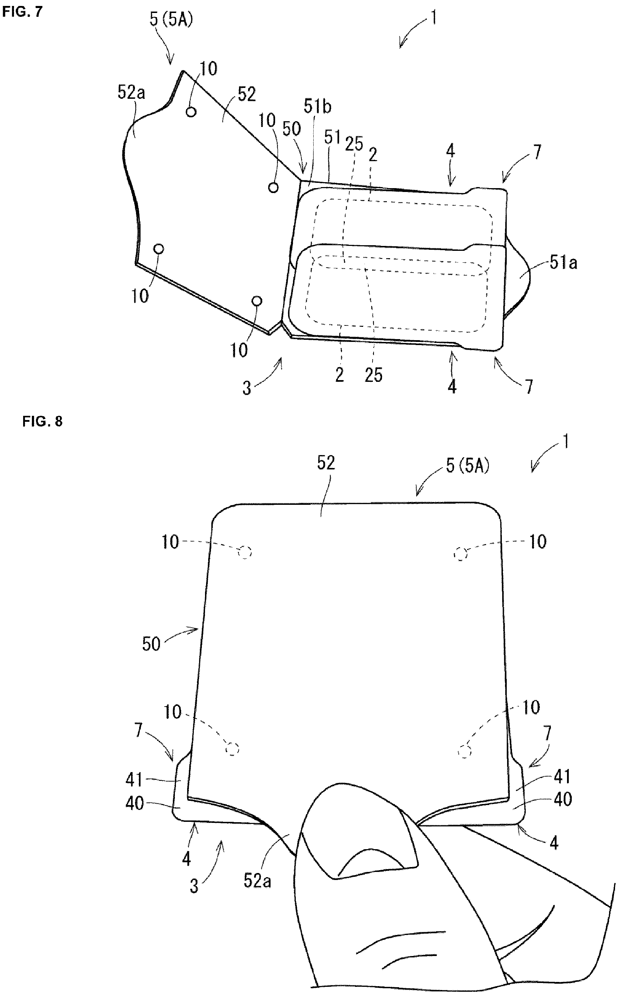

When the plurality of unit assemblies 7 are fixed to the surface to be fixed 51b as described above, the assembly of the IP assembly 1 is completed. FIG. 7 is a schematic diagram showing an example of the completed IP assembly 1.

In FIG. 7, the imaging plate 2 is shown by a dashed line. The two unit assemblies 7 are fixed to the fixed portion 51 such that the two imaging plates 2 respectively included in the two unit assemblies 7 partially overlap each other. For example, the two unit assemblies 7 are fixed by the attachment portion 58 to the surface to be fixed 51b such that one end portion 25 in a short-side direction (in other words, one long-side end portion 25) of the imaging plate 2 of the unit assembly that is fixed second is positioned on one end portion 25 in a short-side direction (in other words, one long-side end portion 25) of the imaging plate 2 of the unit assembly that is fixed first. The one end portion 25 of the imaging plate 2 can be said to be an overlapping portion that overlaps with another imaging plate 2. The two imaging plates 2 are basically stacked by an operator such that their long sides are parallel and their short sides are aligned in a straight line.

As can be understood from the above description, it can be said that the dotted line 51ca shown on the surface to be fixed 51b is information indicating that the unit assembly 7 should be placed on the surface to be fixed 51b such that a longitudinal direction of the unit assembly 7 is aligned with a vertical direction of the surface to be fixed 51b. That is, it can be said that the dotted line 51ca is information (also referred to as orientation information) indicating an orientation that the plurality of imaging plates 2 should take with respect to the holding member 5A. The operator assembling the IP assembly 1 can identify, from the dotted line 51ca shown on the holding member 5A, in what orientation the plurality of imaging plates 2 should be placed with respect to the holding member 5A. In other words, the operator can identify an orientation to be set for the plurality of imaging plates 2 with respect to the holding member 5A from the orientation information shown on the holding member 5A. This makes it easier for the operator to set the orientation of the plurality of imaging plates 2 with respect to the holding member 5A to an appropriate orientation.

Further, it can be said that the numbers 1 and 2 shown on the surface to be fixed 51b are information (also referred to as stacking order information) indicating a stacking order that the two imaging plates 2 covered by the holding member 5 should take. The operator assembling the IP assembly 1 can identify how the stacking order of the two imaging plates 2 should be from the numbers 1 and 2. From another perspective, the operator can identify, from the numbers 1 and 2, in what order the two imaging plates 2 should be placed on the surface to be fixed 51b. It can also be said that the numbers 1 and 2 are information indicating in what order the two imaging plates 2 should be placed (in other words, fixed) on the surface to be fixed 51b. The operator can identify the stacking order to be set for the plurality of imaging plates 2 in the holding member 5A from the stacking order information shown on the holding member 5A. This makes it easier for the operator to set the stacking order of the two imaging plates 2 to an appropriate stacking order.

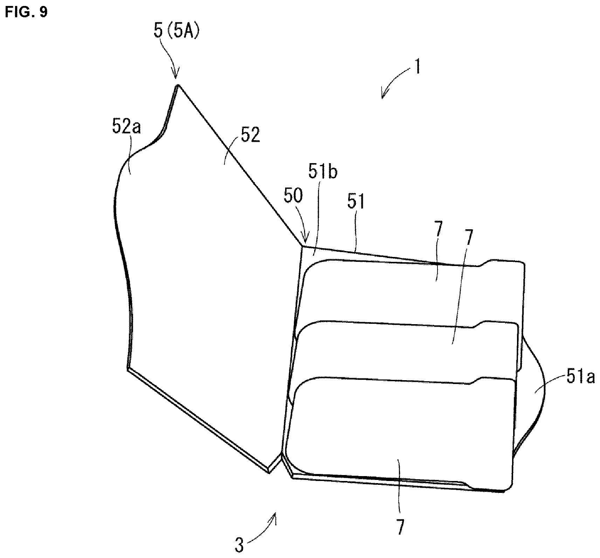

Note that even when three or more unit assemblies 7 are fixed to the fixed portion 51, each unit assembly 7 is fixed to the fixed portion 51 in order. For example, after a first unit assembly 7 is fixed to the fixed portion 51, a second unit assembly 7 is fixed to the fixed portion 51 such that one end portion in a short-side direction of an imaging plate 2 of the second unit assembly 7 overlaps one end portion in a short-side direction of an imaging plate 2 of the first unit assembly 7. Next, a third unit assembly 7 is fixed to the fixed portion 51 such that one end portion in a short-side direction of an imaging plate 2 of the third unit assembly 7 overlaps the other end portion in the short-side direction of the imaging plate 2 of the second unit assembly 7. Thereafter, similarly, a fourth and subsequent unit assemblies 7 are fixed to the fixed portion 51 in order. FIG. 9 is a schematic diagram showing an example of a state in which three unit assemblies 7 are fixed to the fixed portion 51. In the example of FIG. 9, one end portion of another imaging plate is positioned on one end portion of a central imaging plate 2, and one end portion of yet another imaging plate is positioned under the other end portion of the central imaging plate 2.

Note that a method of stacking the imaging plates 2 of the plurality of unit assemblies 7 when three or more unit assemblies 7 are fixed to the fixed portion 51 is not limited to the example of FIG. 9. For example, one end portion in a short-side direction of each of two other imaging plates 2 may be respectively positioned on both end portions in a short-side direction of a certain one imaging plate 2. Further, one end portion in a short-side direction of each of two other imaging plates 2 may be respectively positioned under both end portions in a short-side direction of a certain one imaging plate 2. Further, the plurality of unit assemblies 7 may be fixed to the fixed portion 51 such that two adjacent imaging plates 2 do not overlap each other. That is, the holding member 5A may hold the plurality of unit assemblies 7 such that two adjacent imaging plates 2 do not overlap each other.

The configuration of the attachment portion 58 of the holding member 5A is not limited to the above example. For example, the attachment portion 58 may be composed of a plurality of adhesive members (for example, double-sided tape or a coating-type adhesive) respectively provided on the plurality of waterproof bags 4 to be fixed to the fixed portion 51. Further, the attachment portion 58 may be composed of a plurality of adhesive members respectively provided on the plurality of waterproof bags 4 and an adhesive member provided on the surface to be fixed 51b. When the attachment portion 58 is provided only on the plurality of waterproof bags 4, when fixing the waterproof bag 4 containing the imaging plate 2 to the holding member 5A, it is preferable that a mounting orientation of the imaging plate 2, that is, an orientation of a reading surface 21 and a non-reading surface 22, is less likely to be mistaken. For example, by providing an adhesive member or the like on a portion of the main body portion 40 of the waterproof bag 4 on the side of the non-reading surface 22, the imaging plate 2 can be reliably guided to a correct mounting state with respect to its front and back surfaces.

Further, at least one of the plurality of waterproof bags 4 fixed to the fixed portion 51 may be detachable from the fixed portion 51. In this case, for example, the attachment portion 58 may include a hook-and-loop fastener. Specifically, the attachment portion 58 may include, for example, a first hook-and-loop fastener provided on the waterproof bag 4, and a second hook-and-loop fastener provided on the surface to be fixed 51b, which engages with the first hook-and-loop fastener.

Further, at least one of the plurality of waterproof bags 4 fixed to the fixed portion 51 may be non-detachable from the fixed portion 51. For example, the attachment portion 58 may include a curable adhesive, and the waterproof bag 4 and the fixed portion 51 may be attached to each other with the adhesive.

When the assembled IP assembly 1 is placed in a person's oral cavity for radiography, as shown in FIG. 8, a radiographer (also referred to as a radiography operator) holds a pair of tab portions 51a and 52a of the base member 50 of the holding member 5A with fingers from their outside. Then, the radiographer, while holding the pair of tab portions 51a and 52a, inserts the IP assembly 1 into an oral cavity of a person to be radiographed from a side opposite to the pair of tab portions 51a and 52a. The person to be radiographed maintains a closed state of the base member 50 by biting the base member 50 of the holding member 5A in the oral cavity. The radiation image forming layer 20 of each imaging plate 2 held by the holding member 5A in the closed state faces the cover portion 52 of the holding member 5A. After the IP assembly 1 is placed in the oral cavity, radiography is performed by irradiating the IP assembly 1 with radiation from a side of the cover portion 52.

When a maxillary dental arch is radiographed, the IP assembly 1 is placed in the oral cavity such that the cover portion 52 of the holding member 5A faces the maxillary dental arch. Thereby, each imaging plate 2 of the IP assembly 1 is placed in the oral cavity such that its reading surface 21 (in other words, the radiation image forming layer 20) faces the maxillary dental arch. Then, the IP assembly 1 in the oral cavity is irradiated with radiation from above a maxilla. The radiation passes sequentially through the maxilla, the cover portion 52 of the holding member 5A, the waterproof bag 4, and the protection cover 6 to irradiate the radiation image forming layer 20 of each imaging plate 2. Thereby, a radiation image of the maxillary dental arch or the like is recorded over the entire radiation image forming layers 20 of the plurality of imaging plates 2 included in the IP assembly 1. In an overlapping portion of the two imaging plates 2 held by the holding member 5A, radiation that passed through an upper imaging plate irradiates a lower imaging plate. A part of the maxillary dental arch or the like is recorded on each imaging plate 2. In the IP assembly 1 in the oral cavity, the lower imaging plate 2 fixed first to the surface to be fixed 51b (in other words, the imaging plate 2 fixed at the location of the number 1 on the surface to be fixed 51b) is located on a left cheek side, and the upper imaging plate 2 fixed later to the surface to be fixed 51b (in other words, the imaging plate 2 fixed at the location of the number 2 on the surface to be fixed 51b) is located on a right cheek side.

On the other hand, when a mandibular dental arch is radiographed, the IP assembly 1 is placed in the oral cavity such that the cover portion 52 of the holding member 5A faces the mandibular dental arch. Thereby, each imaging plate 2 of the IP assembly 1 is placed in the oral cavity such that its reading surface 21 faces the mandibular dental arch. Then, the IP assembly 1 in the oral cavity is irradiated with radiation from below a mandible. The radiation passes sequentially through the mandible, the cover portion 52 of the holding member 5A, the waterproof bag 4, and the protection cover 6 to irradiate the radiation image forming layer 20 of each imaging plate 2. Thereby, a radiation image of the mandibular dental arch or the like is recorded over the entire radiation image forming layers 20 of the plurality of imaging plates 2 included in the IP assembly 1. A part of the mandibular dental arch or the like is recorded on each imaging plate 2. In the IP assembly 1 in the oral cavity, the lower imaging plate 2 fixed first to the surface to be fixed 51b is located on a right cheek side, and the upper imaging plate 2 fixed later to the surface to be fixed 51b is located on a left cheek side.

Thus, in this example, the holding member 5 that holds the plurality of imaging plates 2 is inserted into the oral cavity of the person to be radiographed from a side opposite to the pair of tab portions 51a and 52a. Therefore, in the oral cavity, the plurality of imaging plates are placed such that their longitudinal directions are aligned with a front-rear direction (in other words, a depth direction) in the oral cavity. Since the pair of tab portions 51a and 52a determines an insertion orientation of the holding member 5A into the oral cavity, it can be said that the pair of tab portions 51a and 52a indicates the insertion orientation of the holding member 5A into the oral cavity. The radiographer can identify the insertion orientation of the holding member 5A into the oral cavity from the pair of tab portions 51a and 52a. That is, the radiographer can identify, from the pair of tab portions 51a and 52a, in what orientation the holding member 5A should be inserted into the oral cavity. This makes it easier for the radiographer to insert the holding member 5A into the oral cavity in an appropriate orientation.

When the radiography is finished, the IP assembly 1 is removed from the oral cavity of the person to be radiographed. Next, for example, the plurality of unit assemblies 7 are removed from the fixed portion 51 of the holding member 5A. Next, the imaging plate 2 covered by the protection cover 6 is taken out from the waterproof bag 4 of each unit assembly 7.

The imaging plate 2 taken out from the waterproof bag 4 is carried to an insertion port 121 (see FIG. 10) of the reading device 100 while being sandwiched by the protection cover 6. After that, the protection cover 6 is opened and the imaging plate 2 is inserted into the insertion port 121. The reading device 100 reads a radiation image from the inserted imaging plate 2. Similarly, a radiation image is read from each imaging plate 2 included in the IP assembly 1. The reading device 100 combines a plurality of radiation images respectively read from the plurality of imaging plates 2 included in the IP assembly 1 to generate a composite image (in other words, an occlusal image). Then, the reading device 100 displays the generated composite image. In the composite image, for example, a maxillary dental arch or a mandibular dental arch is captured.

Note that when radiography is performed using a single imaging plate 2 because a radiography range is narrow or the like, a single unit assembly 7 is placed in the oral cavity, and the one unit assembly 7 in the oral cavity is irradiated with radiation. Hereinafter, radiography using one unit assembly 7 is referred to as single IP radiography.

As shown in FIGS. 1 to 8, the holding member 5 includes at least one mark 10 for registration, which is used when combining a plurality of radiation images respectively read from the plurality of imaging plates 2 covered by the holding member 5. The mark 10 is made of a radiation-shielding material. The mark 10 does not completely shield radiation but transmits some radiation.

The mark 10 is provided on a portion of the holding member 5 in the oral cavity that faces the radiation image forming layer 20 of each imaging plate 2 covered by the holding member 5. It can be said that the mark 10 is provided on a portion of the holding member 5 where radiation is incident. It can also be said that the mark 10 is provided on a portion of the holding member 5 in the oral cavity that is on a radiation source side with respect to the imaging plate 2.

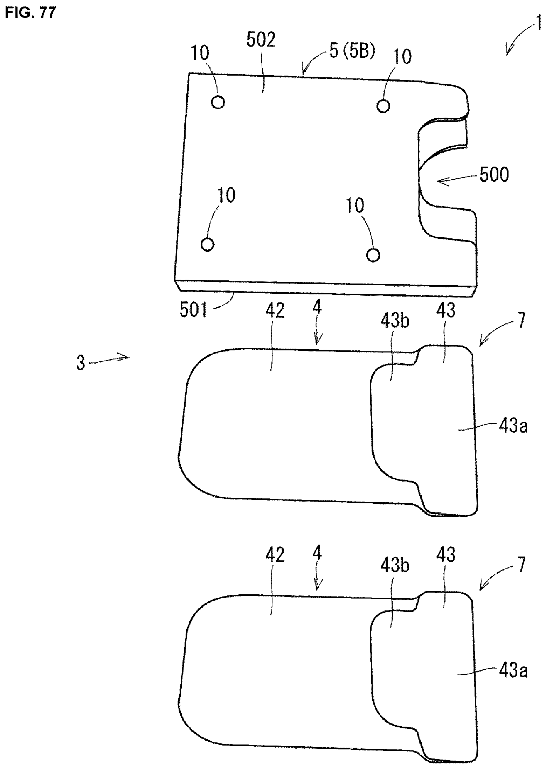

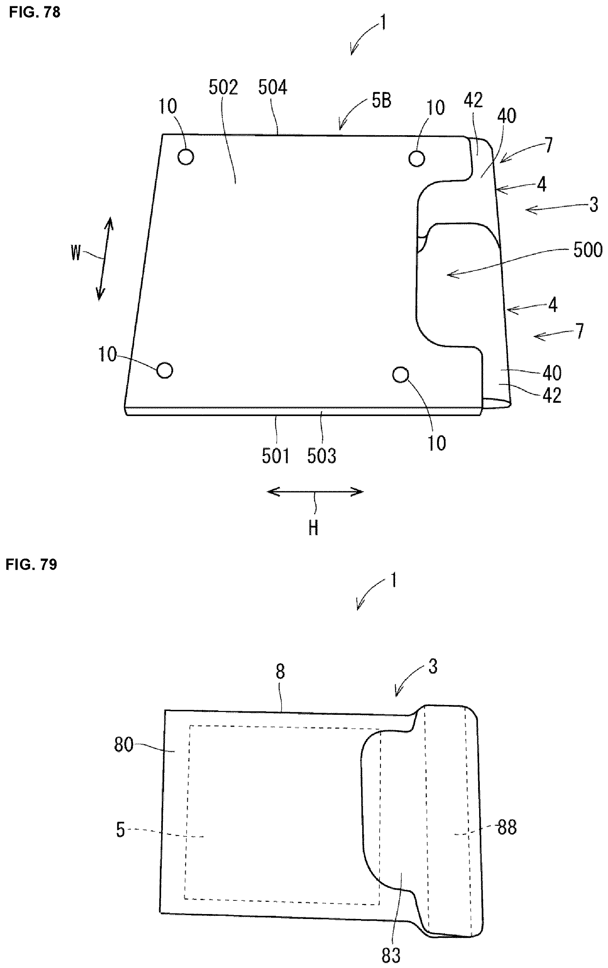

In the holding member 5A shown in FIGS. 1 and 2 and the like, the mark 10 is provided, for example, on an inner surface of the cover portion 52 that faces the radiation image forming layer 20 of the imaging plate 2 when the holding member 5A is closed. The mark 10 is provided on a surface of the holding member 5A of the IP assembly 1 in the oral cavity that faces the radiation image forming layer 20 of the imaging plate 2. Note that the mark 10 may be provided on an outer surface of the cover portion 52. The same applies to a holding member 5B shown in FIGS. 77 and 78 to be described later.

When intra-oral radiography is performed using the IP assembly 1 in the oral cavity, radiation passes through the cover portion 52 of the holding member 5A and irradiates the imaging plate 2. At this time, transmission of radiation is blocked by the mark 10 in a portion of the cover portion 52 where the mark 10 is provided. As a result, a transmission amount of radiation in the portion of the cover portion 52 where the mark 10 is provided is significantly reduced compared to a transmission amount of radiation in a portion of the cover portion 52 where the mark 10 is not provided.

When radiation is applied to the radiation image forming layers 20 of the plurality of imaging plates 2 through the holding member 5, a mark image corresponding to the at least one mark 10 provided on the holding member 5 is recorded on the plurality of imaging plates 2. Thereby, a plurality of radiation images read from the plurality of imaging plates 2 include a mark image corresponding to the at least one mark 10 provided on the holding member 5. When combining the plurality of radiation images read from the plurality of imaging plates 2, the reading device 100 performs a registration process of registering the plurality of radiation images based on the mark images included in the plurality of radiation images.

Hereinafter, the mark 10 may be referred to as a registration mark 10. Further, a mark image corresponding to the mark 10 included in a radiation image may be referred to as a registration mark image. It can be said that the registration mark image is an image showing the registration mark 10, or an image in which the registration mark 10 is captured. The registration mark 10 and the registration mark image will be described in detail later.

### <Example of Configuration of Reading Device>

FIG. 10 is a schematic diagram illustrating an example of a configuration of the reading device 100. The reading device 100 can also be said to be a processing device. As shown in FIG. 10, the reading device 100 includes, for example, a housing 120. A configuration for reading a radiation image from the imaging plate 2 is accommodated in the housing 120. This configuration will be described later.

The housing 120 is provided with an insertion port 121 and an outlet 122. The insertion port 121 is provided, for example, on an upper surface of the housing 120. A user of the reading device 100 can insert the imaging plate 2 into the housing 120 through the insertion port 121. In the example of FIG. 10, the imaging plate 2 is inserted into the insertion port 121 from one end side in a longitudinal direction (in other words, a short side) of the imaging plate 2. Note that the imaging plate 2 may be inserted into the insertion port 121 from one end side in a short-side direction (in other words, a long side) of the imaging plate 2.

A radiation image is read from the imaging plate 2 in the housing 120. The outlet 122 is provided, for example, in a lower portion of one side surface of the housing 120. The imaging plate 2 from which the radiation image has been read (also referred to as a read imaging plate 2) is discharged to the outlet 122. The user of the reading device 100 can collect the read imaging plate 2 through the outlet 122.

The housing 120 is provided with, for example, an operation unit 140 that accepts an operation from a user. The operation unit 140 includes, for example, a plurality of operation buttons 141. Each operation button 141 is, for example, a hardware button. The plurality of operation buttons 141 include, for example, a power button and a start button for instructing the start of reading. The operation unit 140 may include a touch sensor that detects a user's touch operation.

The housing 120 is provided with, for example, a display unit 130. The display unit 130 is composed of, for example, a liquid crystal display panel or an organic EL (electro-luminescence) display panel. The display unit 130 can display various types of information such as characters, symbols, figures, and images. The display unit 130 may display, for example, a radiation image read from the imaging plate 2, that is, a read radiation image. The display unit 130 may include a display unit such as an indicator lamp in addition to a form such as a display panel.

For example, plate storage cases 160 and 170 capable of accommodating the imaging plate 2 are placed on the upper surface of the housing 120.

From the housing 120, a cable 151 of an AC adapter 150 extends outward. Power is supplied from the AC adapter 150 to each component of the reading device 100. The reading device 100 may include not only the AC adapter 150 but also a battery that supplies power to each component of the reading device 100. Alternatively, the reading device 100 may include a battery instead of the AC adapter 150.

### <Example of Mechanism inside Housing>

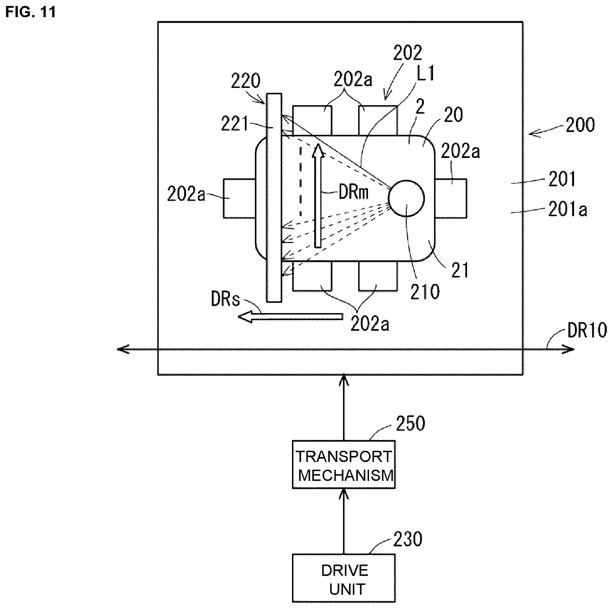

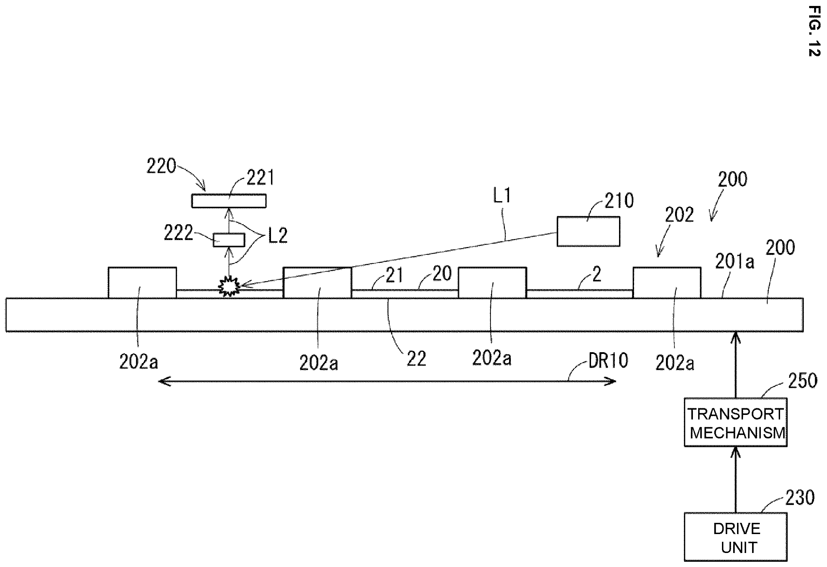

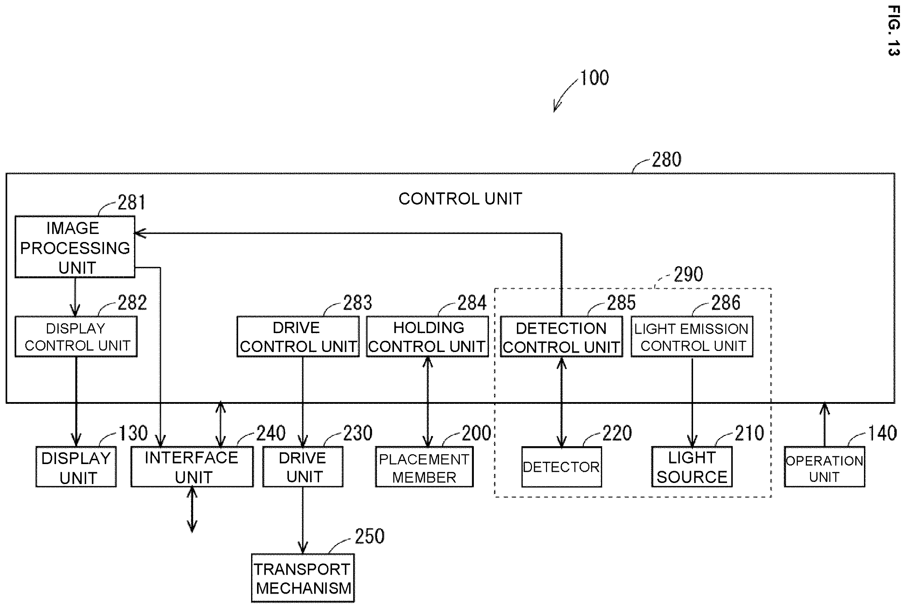

FIGS. 11 and 12 are schematic diagrams illustrating an example of a configuration inside the housing 120. FIG. 12 shows an example of a state where a structure shown in FIG. 11 is viewed from a lower side of FIG. 11. FIG. 13 is a block diagram mainly illustrating an example of a configuration of a control unit 280 included in the reading device 100.

As shown in FIGS. 11 to 13, the reading device 100 includes, for example, a placement member 200 on which the imaging plate 2 is placed, a light source 210, a detector 220, a drive unit 230, an interface unit 240, a control unit 280, and a transport mechanism 250. These configurations are provided in the housing 120.

### <Regarding Control Unit>

The control unit 280 can integrally manage operations of the reading device 100 and can also be said to be, for example, a control circuit. The control unit 280 can control, for example, the display unit 130, the placement member 200, the light source 210, the detector 220, the drive unit 230, and the interface unit 240. Further, the control unit 280 can perform processing according to a user operation accepted by the operation unit 140.

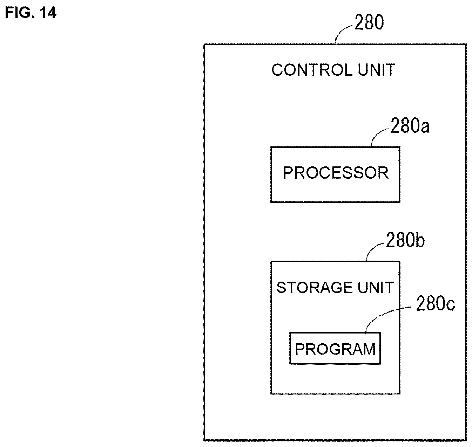

As shown in FIG. 14, the control unit 280 includes, for example, at least one processor 280a and a storage unit (also referred to as a storage circuit) 280b. The at least one processor 280a may include a CPU (Central Processing Unit) or a processor other than a CPU. In the control unit 280, various functions described below are realized by the at least one processor 280a executing a program 280c in the storage unit 280b. The control unit 280 can also be referred to as a computer device.

In the control unit 280, by the at least one processor 280a executing the program 280c in the storage unit 280b, an image processing unit 281, a display control unit 282, a drive control unit 283, a holding control unit 284, a detection control unit 285, and a light emission control unit 286 are formed as functional blocks, for example.

The image processing unit 281 can, for example, perform image processing on an image indicated by an image signal described later, which is output from the detector 220. The display control unit 282 can control the display of the display unit 130. The drive control unit 283 can control the drive unit 230. The holding control unit 284 can control the holding of the imaging plate 2 on the placement member 200. The detection control unit 285 can control the detector 220. The light emission control unit 286 can control the light source 210.

Note that some functions of the control unit 280 or all functions of the control unit 280 may be realized by a hardware circuit that does not require software (in other words, a program) to realize the functions. For example, some functions of the image processing unit 281 or all functions of the image processing unit 281 may be realized by a hardware circuit that does not require software to realize the functions. The same applies to the display control unit 282, the drive control unit 283, the holding control unit 284, the detection control unit 285, and the light emission control unit 286. Further, at least one of the image processing unit 281, the display control unit 282, the drive control unit 283, the holding control unit 284, the detection control unit 285, and the light emission control unit 286 may be configured by a circuit independent of other configurations.

### <Regarding Interface Unit>

The interface unit 240 can communicate with a device external to the housing 120 (hereinafter, also referred to as an external device) and can also be said to be, for example, an interface circuit, a communication circuit, or a communication unit. The external device may include a personal computer, a mobile phone such as a smartphone, or another computer device (for example, a tablet terminal). Further, the external device may include a data recording medium (for example, a flash memory) detachable from the reading device 100. The interface unit 240 can receive a signal from the external device and input the received signal to the control unit 280. Further, the interface unit 240 can transmit a signal from the control unit 280 to the external device. For example, the interface unit 240 can transmit an image signal representing an image subjected to image processing in the image processing unit 281 of the control unit 280 to the external device. The interface unit 240 may perform wired communication or wireless communication with the external device. Communication between the interface unit 240 and the external device may comply with Ethernet, USB (Universal Serial Bus), Wi-Fi, or other standards.

### <Regarding Placement Member>

The imaging plate 2 inserted from the insertion slot 121 of the housing 120 is placed on the placement member 200. The placement member 200 can, for example, hold the placed imaging plate 2. The placement member 200 includes, for example, a placement plate 201 (also referred to as a stage) on which the imaging plate 2 is placed, and a fixing portion 202 that fixes a position of the imaging plate 2 placed on the placement plate 201.

The placement plate 201 includes a main surface 201a (also referred to as a placement surface 201a) on which the imaging plate 2 is placed from its non-reading surface 22 side, and a back surface opposite to the main surface 201a.

The fixing portion 202 has, for example, a plurality of fixing portions 202a that approach a peripheral portion of the imaging plate 2. The fixing portion 202 can also be referred to as a fixing member. As shown in FIG. 10, the plurality of fixing portions 202a approach the peripheral portion of the imaging plate 2 so as to surround it. Thereby, a position (that is, a relative position) and an orientation (that is, a relative orientation) of the imaging plate 2 with respect to the placement plate 201 are fixed.

Each fixing portion 202a can move between an approaching position where it approaches the imaging plate 2 supported by the placement plate 201 and a separated position where it is separated from the imaging plate 2 supported by the placement plate 201, under the control of the holding control unit 284. The imaging plate 2 is inserted into the housing 120 from the insertion port 121 and placed on the placement plate 201 in a state where each fixing portion 202a is in the separated position. After that, by each fixing portion 202a moving from the separated position to the approaching position, the position and orientation of the imaging plate 2 are fixed by the fixing portion 202. That is, the imaging plate 2 is held.

### <Regarding Drive Unit and Transport Mechanism>

The transport mechanism 250 can transport the placement member 200 along a predetermined direction DR10 by being driven by the drive unit 230. Thereby, the imaging plate 2 placed on the placement member 200 can also move along the predetermined direction DR10. It can also be said that the transport mechanism 250 can move the imaging plate 2 along the predetermined direction DR10 via the placement member 200. The drive unit 230 is controlled by the drive control unit 283. The drive unit 230 includes, for example, a motor. The transport mechanism 250 is driven by a motor included in the drive unit 230. Rotation of the motor included in the drive unit 230 is controlled by the drive control unit 283. The predetermined direction DR10 is, for example, a direction along a longitudinal direction of the imaging plate 2 placed on the placement member 200. Therefore, the imaging plate 2 moves in a direction along the longitudinal direction of the imaging plate 2 as the placement member 200 moves along the predetermined direction DR10. Hereinafter, the predetermined direction DR10 may be referred to as a transport direction DR10 or a moving direction DR10.

### <Regarding Light Source and Detector>

In this example, as shown in FIG. 13, an optical measuring instrument 290 that reads a radiographic image from the radiation image forming layer 20 of the imaging plate 2 is configured by the light source 210, the light emission control unit 286 that controls it, the detector 220, and the detection control unit 285 that controls it. The light source 210, the detector 220, the detection control unit 285, and the light emission control unit 286 constituting the optical measuring instrument 290 may be accommodated in a single case and unitized, or may not be accommodated in a single case.

The light source 210 can irradiate the imaging plate 2 held by the placement member 200 with excitation light L1 for exciting the radiation image forming layer 20. The light source 210 emits the excitation light L1 toward the placement surface 201a of the placement member 200. The light source 210 can scan the excitation light L1 in one direction (also referred to as a main scanning direction DRm) on the imaging plate 2. The main scanning direction DRm is a direction perpendicular to the moving direction DR10 of the placement member 200. The main scanning direction DRm is a direction along a short-side direction of the imaging plate 2 placed on the placement member 200.

The excitation light L1 is, for example, a laser beam of visible light. The excitation light L1 may be, for example, a red laser beam or a laser beam of another color. When the radiation image forming layer 20 is irradiated with the excitation light L1, the radiation image forming layer 20 emits light according to the distribution of energy accumulated in the radiation image forming layer 20, and stimulated luminescence L2 (see FIG. 12) is emitted from the radiation image forming layer 20. The stimulated luminescence L2 is, for example, blue visible light. The detector 220 detects the stimulated luminescence L2 from the imaging plate 2 and outputs an electric signal corresponding to the intensity of the detected stimulated luminescence L2.

The light source 210 has, for example, a laser generation unit that generates and outputs the excitation light L1, and a laser scanning unit that scans the excitation light L1 on the imaging plate 2 in a main scanning direction DRm. The laser generation unit includes, for example, a semiconductor laser oscillator and is controlled by the light emission control unit 286. The laser generation unit may include a laser diode or another semiconductor laser. The laser scanning unit includes, for example, a MEMS (Micro Electro Mechanical Systems) mirror that reflects the excitation light L1 from the laser generation unit toward the radiation image forming layer 20 of the imaging plate 2. The MEMS mirror changes a reflection angle of the excitation light L1 under the control of the light emission control unit 286 such that an irradiation point of the excitation light L1 on the radiation image forming layer 20 moves in the main scanning direction DRm. The laser scanning unit may include another mirror such as a galvanometer mirror instead of the MEMS mirror.

The detector 220 includes an optical filter 222 on which the stimulated luminescence L2 from the imaging plate 2 is entered, and a sensor 221 that detects the stimulated luminescence L2 emitted from the optical filter 222. The sensor 221 is controlled by the detection control unit 285. The optical filter 222 is located between the sensor 221 and the main surface 201a of the placement plate 201. The stimulated luminescence L2 from the imaging plate 2 first enters the optical filter 222, and the filtered stimulated luminescence L2 exits the optical filter 222 and enters the sensor 221.

The sensor 221 can detect the stimulated luminescence L2 that has passed through the optical filter 222 and output an electric signal corresponding to the intensity of the detected stimulated luminescence L2. The sensor 221 may be composed of, for example, a plurality of photodiodes, or may be composed of a photomultiplier tube.

In the reading device 100, when a process of reading a radiation image from the imaging plate 2 (also referred to as a reading process) is performed, the placement member 200 on which the imaging plate 2 is placed is transported to a reading start position by the transport mechanism 250 driven by the drive unit 230. Then, the optical measuring instrument 290 starts the reading process.

In the reading process, the light source 210 repeatedly executes a process (also referred to as a main scanning direction scan) of scanning the excitation light L1 on the imaging plate 2 in the main scanning direction DRm under the control of the light emission control unit 286. On the other hand, in the reading process, the transport mechanism 250 moves the placement member 200, on which the imaging plate 2 is placed, in one direction DRs (also referred to as a sub-scanning direction DRs) along the transport direction DR10. The sub-scanning direction DRs is a direction perpendicular to the main scanning direction DRm. While the placement member 200 is moving in the sub-scanning direction DRs, the main scanning direction scan is repeatedly executed, whereby the radiation image forming layer 20 of the imaging plate 2 is irradiated with the excitation light L1 with a two-dimensional spread, and the radiation image forming layer 20 is raster-scanned. Thereby, in the reading process, the entire area of the radiation image forming layer 20 is sequentially irradiated with the excitation light L1, and the entire area of the radiation image forming layer 20 is scanned with the excitation light L1. While the radiation image forming layer 20 is being raster-scanned by the excitation light L1, the sensor 221 of the detector 220 detects the stimulated luminescence L2 sequentially arriving from the radiation image forming layer 20 in response to the raster scan, whereby the radiation image is read from the radiation image forming layer 20.

The sensor 221 outputs an image signal representing a read radiation image to the detection control unit 285 as a detection result of the stimulated luminescence L2 during the raster scan of the excitation light L1. This image signal includes intensity values (in other words, pixel values) of a plurality of pixels representing the read radiation image. The sensor 221 outputs, for example, a grayscale image signal.

The optical measuring instrument 290 reads the radiation image recorded on the imaging plate 2 along a longitudinal direction of the imaging plate 2. That is, the optical measuring instrument 290 reads the radiation image recorded on the imaging plate 2 from one end side in the longitudinal direction of the imaging plate 2 (in other words, one short side) toward the other end side in the longitudinal direction of the imaging plate 2 (in other words, the other short side).

An outer shape of the radiation image read from the imaging plate 2 by the optical measuring instrument 290 is the same as an outer shape of the imaging plate 2. The outer shape of the read radiation image is, for example, a substantial rectangle with rounded corners. One end in the longitudinal direction of the read radiation image (in other words, one short side) becomes a reading start side in the optical measuring instrument 290, and the other end in the longitudinal direction of the read radiation image (in other words, the other short side) becomes a reading end side in the optical measuring instrument 290. Note that one long side of the read radiation image may be the reading start side in the optical measuring instrument 290, and the other long side of the read radiation image may be the reading end side in the optical measuring instrument 290.

Hereinafter, for convenience of explanation, the imaging plate 2 and the read radiation image will be described assuming that an outer shape of the imaging plate 2 and an outer shape of the read radiation image are rectangular.

The scanning of the excitation light L1 may be performed two-dimensionally, and the main scanning direction DRm and the sub-scanning direction DRs do not have to be perpendicular to each other. For example, the main scanning direction DRm and the sub-scanning direction DRs may intersect at an angle other than perpendicular. Further, one or both of the main scanning direction DRm and the sub-scanning direction DRs may be set to a curved direction.

### <Regarding Image Processing Unit>

The image processing unit 281 of the control unit 280 acquires an image signal output from the sensor 221 via the detection control unit 285. Then, the image processing unit 281 performs predetermined image processing on a read radiographic image indicated by the acquired image signal, that is, a radiation image read by the optical measuring instrument 290. A radiographic image can also be said to be a type of image.

Here, the reading device 100 includes, as operation modes, a combination mode (also referred to as a synthesis mode) for combining a plurality of read radiation images, and a normal mode in which a plurality of read radiation images are not combined. The reading device 100 switches the operation mode, for example, according to an operation accepted by the operation unit 140. When inserting the imaging plate 2 of one unit assembly 7 used in single IP radiography into the reading device 100, a user performs a predetermined operation on the operation unit 140 to set the operation mode of the reading device 100 to the normal mode. In the normal mode, the image processing unit 281 performs predetermined image processing on the read radiation image indicated by the image signal output from the sensor 221. Then, the display control unit 282 causes the display unit 130 to display the read radiation image that has been subjected to image processing by the image processing unit 281.

On the other hand, when inserting the plurality of imaging plates 2 included in the IP assembly 1 used in occlusal radiography into the reading device 100, the user performs a predetermined operation on the operation unit 140 to set the operation mode of the reading device 100 to the combination mode. When the user sets the operation mode of the reading device 100 to the combination mode, the user continuously and sequentially inserts the plurality of imaging plates 2 included in the IP assembly 1 into the insertion port 121 of the reading device 100. Here, a layout of the plurality of imaging plates 2 on the holding member 5 is referred to as an IP layout. When inserting the plurality of imaging plates 2 into the insertion port 121 in order, the user inserts the imaging plates 2 into the insertion port 121 sequentially, for example, from a topmost imaging plate 2 in the IP layout (that is, the imaging plate 2 fixed last to the holding member 5) to a bottommost imaging plate 2 in the IP layout (that is, the imaging plate 2 fixed first to the holding member 5). When two imaging plates 2 are held by the holding member 5A as in the example of FIG. 7, the user first inserts the imaging plate 2 placed at the location of the number 2 on the surface to be fixed 51b (that is, the topmost imaging plate 2 in the IP layout) into the insertion port 121. After that, the user inserts the imaging plate 2 placed at the location of the number 1 on the surface to be fixed 51b (that is, the bottommost imaging plate 2 in the IP layout) into the insertion port 121.

Hereinafter, the number of imaging plates 2 included in the IP assembly 1 is represented by N (where N is an integer of 2 or more). Further, of the N imaging plates 2 included in the IP assembly 1, an n-th (1 ≤ n ≤ N) imaging plate 2 from the top of the IP layout may be referred to as an n-th imaging plate 2. In the example of FIG. 7, the imaging plate 2 placed at the location of the number 2 on the surface to be fixed 51b becomes a 1-th imaging plate 2, and the imaging plate 2 placed at the location of the number 1 on the surface to be fixed 51b becomes a 2-th imaging plate 2. The n-th imaging plate 2 and an (n+1)-th imaging plate 2 are adjacent to each other in the IP layout. That is, the n-th imaging plate 2 and the (n+1)-th imaging plate 2 are fixed to the surface to be fixed 51b so as to be adjacent to each other. It can also be said that n represents an order in which the N imaging plates 2 are inserted into the reading device 100.

The user inserts the N imaging plates 2 into the insertion port 121 from the same direction. Here, when the IP assembly 1 is placed in the oral cavity, a short side of the imaging plate 2 of the IP assembly 1 that is located on a posterior side of the oral cavity (in other words, a deep side or posterior side of the oral cavity) is referred to as an intra-oral posterior short side. Further, when the IP assembly 1 is placed in the oral cavity, a short side of the imaging plate 2 of the IP assembly 1 that is located on an anterior side of the oral cavity (in other words, an anterior side) is referred to as an intra-oral anterior short side. Each imaging plate 2 of the IP assembly 1 is inserted into the insertion port 121 from, for example, the intra-oral posterior short side. Note that each imaging plate 2 of the IP assembly 1 may be inserted into the insertion port 121 from the intra-oral anterior short side.

The optical measuring instrument 290 of the reading device 100 reads a radiation image from the imaging plate 2 each time the imaging plate 2 is inserted. When the imaging plate 2 is inserted into the reading device 100 from the intra-oral posterior short side, the optical measuring instrument 290 reads the radiation image from the intra-oral posterior short side of the imaging plate 2. That is, the intra-oral posterior short side of the imaging plate 2 becomes a reading start side of the radiation image. The image processing unit 281 performs a registration process of registering the N read radiation images based on registration mark images included in the N read radiation images read in order by the optical measuring instrument 290. After that, the image processing unit 281 combines the N read radiation images after the registration. Thereby, a composite image (that is, an occlusal image) in which a subject such as a dental arch (in other words, an object) is captured is generated. The image processing unit 281 performs predetermined image processing on the generated composite image. The display control unit 282 causes the display unit 130 to display the composite image that has been subjected to image processing by the image processing unit 281.

The image processing unit 281 combines the N read radiation images according to the IP layout of the N imaging plates 2. The image processing unit 281 combines two read radiation images respectively read from two imaging plates 2 that are adjacent to each other in the IP layout.

Here, two read radiation images respectively read from two imaging plates 2 that are adjacent to each other in the IP layout are referred to as a pair of radiation images to be combined. Further, a read radiation image read from the n-th imaging plate 2 is referred to as an n-th radiation image. The n-th radiation image is an n-th read radiation image read by the reading device 100 among the N read radiation images.

For the N read radiation images, there are (N-1) pairs of radiation images to be combined. For example, when N=3, there are two pairs of radiation images to be combined: a pair of radiation images to be combined consisting of a 1-th radiation image and a 2-th radiation image, and a pair of radiation images to be combined consisting of the 2-th radiation image and a 3-th radiation image. Further, when N=4, there are three pairs of radiation images to be combined: a pair of radiation images to be combined consisting of a 1-th radiation image and a 2-th radiation image, a pair of radiation images to be combined consisting of the 2-th radiation image and a 3-th radiation image, and a pair of radiation images to be combined consisting of the 3-th radiation image and a 4-th radiation image.

The image processing unit 281 combines two read radiation images constituting one pair of radiation images to be combined, for each of the (N-1) pairs of radiation images to be combined. Thereby, the N read radiation images are combined to generate a composite image.

For example, a case where N=3 is considered. In this case, the image processing unit 281 combines a 1-th radiation image (in other words, a first read radiation image) and a 2-th radiation image (in other words, a second read radiation image), and combines the 2-th radiation image and a 3-th radiation image (in other words, a third read radiation image) to combine the three radiation images. When N=3, it can also be said that the image processing unit 281 generates a composite image by combining the 1-th radiation image and the 3-th radiation image with the 2-th radiation image.

As another example, a case where N=4 is considered. In this case, the image processing unit 281 combines a 1-th radiation image and a 2-th radiation image, combines the 2-th radiation image and a 3-th radiation image, and combines the 3-th radiation image and a 4-th radiation image, to combine the four radiation images.

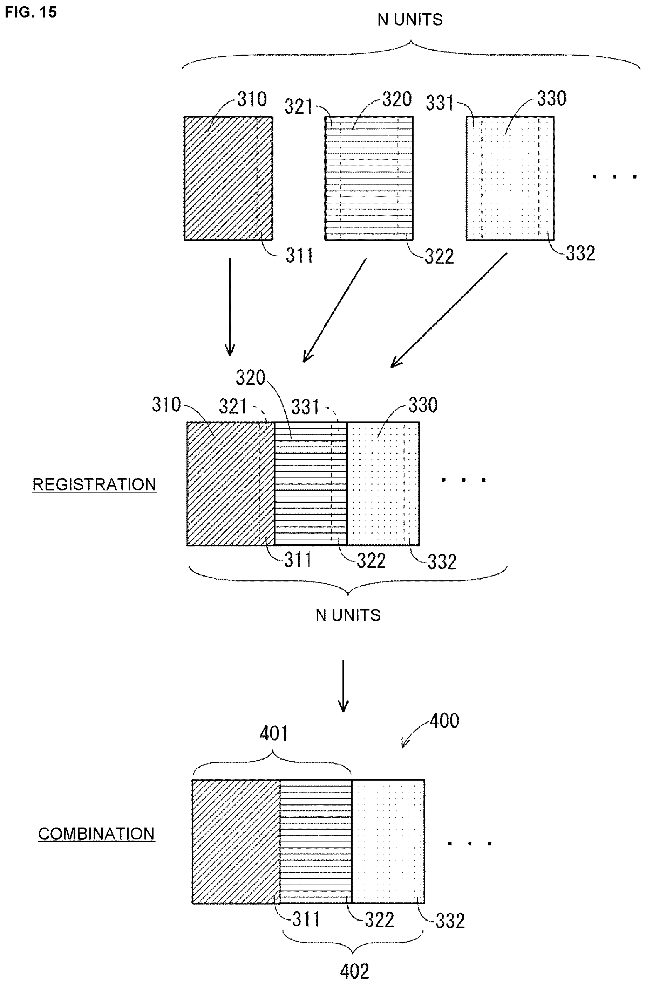

FIG. 15 is a schematic diagram for explaining an example of a method for combining N read radiation images. An example of a method for combining N read radiation images will be described below with reference to FIG. 15.

The 1-th imaging plate 2 has an overlapping portion (also referred to as a 2-th IP overlapping portion) that overlaps with a 2-th imaging plate 2 in the IP layout. One end portion 25 in a short-side direction (see FIG. 7) of the first imaging plate 2 that overlaps with the 2-th imaging plate 2 becomes the 2-th IP overlapping portion.

The N-th imaging plate 2 has an overlapping portion (also referred to as an (N-1)-th IP overlapping portion) that overlaps with an (N-1)-th imaging plate 2 in the IP layout. When 2 ≤ n ≤ N-1, the n-th imaging plate 2 has an overlapping portion (also referred to as an (n-1)-th IP overlapping portion) that overlaps with an (n-1)-th imaging plate 2 in the IP layout, and an overlapping portion (also referred to as an (n+1)-th IP overlapping portion) that overlaps with an (n+1)-th imaging plate 2 in the IP layout.

As shown on an upper side of FIG. 15, a 1-th radiation image 310 has a 2-th IP overlapping portion corresponding image 311 corresponding to a 2-th IP overlapping portion of the 1-th imaging plate 2. The 2-th IP overlapping portion corresponding image 311 is an image read from the 2-th IP overlapping portion of the 1-th imaging plate 2. One end portion in a short-side direction of the 1-th radiation image 310 (in other words, one long-side end portion) constitutes the 2-th IP overlapping portion corresponding image 311.

An N-th radiation image has an (N-1)-th IP overlapping portion corresponding image corresponding to an (N-1)-th IP overlapping portion of the N-th imaging plate 2. The (N-1)-th IP overlapping portion corresponding image is an image read from the (N-1)-th IP overlapping portion of the N-th imaging plate 2. One end portion in a short-side direction of the N-th radiation image constitutes the (N-1)-th IP overlapping portion corresponding image.

When 2 ≤ n ≤ N-1, an n-th radiation image has an (n-1)-th IP overlapping portion corresponding image and an (n+1)-th IP overlapping portion corresponding image, corresponding respectively to an (n-1)-th IP overlapping portion and an (n+1)-th IP overlapping portion of the n-th imaging plate 2. The (n-1)-th IP overlapping portion corresponding image is an image read from the (n-1)-th IP overlapping portion of the n-th imaging plate 2. The (n+1)-th IP overlapping portion corresponding image is an image read from the (n+1)-th IP overlapping portion of the n-th imaging plate 2. One end portion in a short-side direction of the n-th radiation image constitutes the (n-1)-th IP overlapping portion corresponding image, and the other end portion in the short-side direction of the n-th radiation image constitutes the (n+1)-th IP overlapping portion corresponding image. For example, a 2-th radiation image 320 has a 1-th IP overlapping portion corresponding image 321 and a 3-th IP overlapping portion corresponding image 322, as shown on the upper side of FIG. 15. Further, a 3-th radiation image 330 has a 2-th IP overlapping portion corresponding image 331 and a 4-th IP overlapping portion corresponding image 332.

The (n-1)-th IP overlapping portion corresponding image of the n-th radiation image and the n-th IP overlapping portion corresponding image of the (n-1)-th radiation image show the same part of the subject. Therefore, it can be said that the (n-1)-th IP overlapping portion corresponding image of the n-th radiation image and the n-th IP overlapping portion corresponding image of the (n-1)-th radiation image are an overlapping portion (in other words, an overlapping image) that overlaps between the n-th radiation image and the (n-1)-th radiation image. For example, it can be said that the 1-th IP overlapping portion corresponding image 321 of the 2-th radiation image 320 and the 2-th IP overlapping portion corresponding image 311 of the 1-th radiation image 310 are an overlapping portion between the 2-th radiation image 320 and the 1-th radiation image 310.

Further, the (n+1)-th IP overlapping portion corresponding image of the n-th radiation image and the n-th IP overlapping portion corresponding image of the (n+1)-th radiation image show the same part of the subject. Therefore, it can be said that the (n+1)-th IP overlapping portion corresponding image of the n-th radiation image and the n-th IP overlapping portion corresponding image of the (n+1)-th radiation image are an overlapping portion that overlaps between the n-th radiation image and the (n+1)-th radiation image. For example, it can be said that the 3-th IP overlapping portion corresponding image 322 of the 2-th radiation image 320 and the 2-th IP overlapping portion corresponding image 331 of the 3-th radiation image 330 are an overlapping portion between the 2-th radiation image 320 and the 3-th radiation image 330.

The image processing unit 281 performs registration of the N radiation images by performing registration of two radiation images constituting one pair of radiation images to be combined for each of the (N-1) pairs of radiation images to be combined, such that the two radiation images partially overlap. Then, the image processing unit 281 combines the N radiation images after registration to generate a composite image in which a subject such as a dental arch is captured.

When registering the n-th radiation image and the (n+1)-th radiation image constituting one pair of radiation images to be combined, the image processing unit 281 registers the n-th radiation image and the (n+1)-th radiation image such that the (n+1)-th IP overlapping portion corresponding image of the n-th radiation image and the n-th IP overlapping portion corresponding image of the (n+1)-th radiation image overlap each other. At this time, the image processing unit 281 basically registers the n-th radiation image and the (n+1)-th radiation image such that the n-th radiation image and the (n+1)-th radiation image overlap only by the (n+1)-th IP overlapping portion corresponding image and the n-th IP overlapping portion corresponding image. Further, in the n-th imaging plate 2 and the (n+1)-th imaging plate 2, the image processing unit 281 registers the n-th radiation image and the (n+1)-th radiation image such that the (n+1)-th IP overlapping portion corresponding image of the n-th radiation image read from the upper n-th imaging plate 2 is positioned above the n-th IP overlapping portion corresponding image of the (n+1)-th radiation image read from the lower (n+1)-th imaging plate 2. Note that the n-th radiation image and the (n+1)-th radiation image may be registered such that the (n+1)-th IP overlapping portion corresponding image of the n-th radiation image is positioned below the n-th IP overlapping portion corresponding image of the (n+1)-th radiation image.

For example, a case where a 1-th radiation image 310 and a 2-th radiation image 320 constituting one pair of radiation images to be combined are registered is considered. In this case, as shown in a middle part of FIG. 15, the image processing unit 281 registers the 1-th radiation image 310 and the 2-th radiation image 320 such that a 2-th IP overlapping portion corresponding image 311 (in other words, an overlapping portion) of the 1-th radiation image 310 and a 1-th IP overlapping portion corresponding image 321 (in other words, an overlapping portion) of the 2-th radiation image 320 overlap each other. At this time, the image processing unit 281 basically registers the 1-th radiation image 310 and the 2-th radiation image 320 such that the 1-th radiation image 310 and the 2-th radiation image 320 overlap only by the 2-th IP overlapping portion corresponding image 311 and the 1-th IP overlapping portion corresponding image 321. Further, the image processing unit 281 registers, for example, the 1-th radiation image 310 and the 2-th radiation image 320 such that the 2-th IP overlapping portion corresponding image 311 of the 1-th radiation image 310 read from the upper 1-th imaging plate 2 is positioned above the 1-th IP overlapping portion corresponding image 321 of the 2-th radiation image 320 read from the lower 2-th imaging plate 2.

As another example, a case where a 2-th radiation image 320 and a 3-th radiation image 330 constituting one pair of radiation images to be combined are registered is considered. In this case, as shown in the middle part of FIG. 15, the image processing unit 281 registers the 2-th radiation image 320 and the 3-th radiation image 330 such that a 3-th IP overlapping portion corresponding image 322 of the 2-th radiation image 320 and a 2-th IP overlapping portion corresponding image 331 of the 3-th radiation image 330 overlap each other. At this time, the image processing unit 281 basically registers the 2-th radiation image 320 and the 3-th radiation image 330 such that the 2-th radiation image 320 and the 3-th radiation image 330 overlap only by the 3-th IP overlapping portion corresponding image 322 and the 2-th IP overlapping portion corresponding image 331. Further, the image processing unit 281 registers, for example, the 2-th radiation image 320 and the 3-th radiation image 330 such that the 3-th IP overlapping portion corresponding image 322 of the 2-th radiation image 320 read from the upper 2-th imaging plate 2 is positioned above the 2-th IP overlapping portion corresponding image 331 of the 3-th radiation image 330 read from the lower 3-th imaging plate 2.

As described above, the image processing unit 281 performs registration of the N radiation images by performing registration of two read radiation images constituting one pair of radiation images to be combined for each of the (N-1) pairs of radiation images to be combined, such that the two read radiation images partially overlap. The N read radiation images are registered such that they are arranged in a line along a short-side direction of the radiation images in their numerical order, and long-side end portions of two adjacent radiation images overlap each other. When performing registration of two read radiation images constituting one pair of radiation images to be combined, the image processing unit 281 performs registration of the two read radiation images based on at least one registration mark image included in each of the two read radiation images. A specific example of registration using a registration mark image will be described in detail later.

When the registration of the N read radiation images is completed (see the middle part of FIG. 15), the image processing unit 281 combines the N read radiation images after registration to generate a composite image 400 shown on a lower side of FIG. 15. Specifically, after performing registration of the N read radiation images, the image processing unit 281 combines the N read radiation images after registration by combining two read radiation images constituting one pair of radiation images to be combined, for each of the (N-1) pairs of radiation images to be combined.

When combining the n-th radiation image and the (n+1)-th radiation image constituting one pair of radiation images to be combined, the image processing unit 281 deletes, for example, the lower n-th IP overlapping portion corresponding image from the (n+1)-th radiation image, from among the (n+1)-th IP overlapping portion corresponding image of the n-th radiation image and the n-th IP overlapping portion corresponding image of the (n+1)-th radiation image, which overlap each other. Then, the image processing unit 281 connects the (n+1)-th radiation image, from which the n-th IP overlapping portion corresponding image has been deleted, and the n-th radiation image. That is, an edge on the n-th radiation image side of the (n+1)-th radiation image, from which the n-th IP overlapping portion corresponding image has been deleted, and an edge on the (n+1)-th radiation image side of the n-th radiation image are connected. Thereby, the n-th radiation image and the (n+1)-th radiation image are combined. A combined image obtained by combining the n-th radiation image and the (n+1)-th radiation image includes the (n+1)-th IP overlapping portion corresponding image of the n-th radiation image, and does not include the n-th IP overlapping portion corresponding image of the (n+1)-th radiation image.

For example, a case where a 1-th radiation image 310 and a 2-th radiation image 320 constituting one pair of radiation images to be combined are combined is considered. In this case, the image processing unit 281 deletes, for example, the lower 1-th IP overlapping portion corresponding image 321 from the 2-th radiation image 320, from among the 2-th IP overlapping portion corresponding image 311 of the 1-th radiation image 310 and the 1-th IP overlapping portion corresponding image 321 of the 2-th radiation image 320, which overlap each other. Then, the image processing unit 281 connects the 1-th radiation image 310 and the 2-th radiation image 320 from which the 1-th IP overlapping portion corresponding image 321 has been deleted. Thereby, the 1-th radiation image 310 and the 2-th radiation image 320 are combined. As shown on the lower side of FIG. 15, a combined image 401 obtained by combining the 1-th radiation image 310 and the 2-th radiation image 320 includes the 2-th IP overlapping portion corresponding image 311 of the 1-th radiation image 310, and does not include the 1-th IP overlapping portion corresponding image 321 of the 2-th radiation image 320.

Further, a case where a 2-th radiation image 320 and a 3-th radiation image 330 constituting one pair of radiation images to be combined are combined is considered. In this case, the image processing unit 281 deletes the lower 2-th IP overlapping portion corresponding image 331 from the 3-th radiation image 330, from among the 3-th IP overlapping portion corresponding image 322 of the 2-th radiation image 320 and the 2-th IP overlapping portion corresponding image 331 of the 3-th radiation image 330. Then, the image processing unit 281 connects the 3-th radiation image 330, from which the 2-th IP overlapping portion corresponding image 331 has been deleted, and the 2-th radiation image 320. Thereby, the 2-th radiation image 320 and the 3-th radiation image 330 are combined. As shown on the lower side of FIG. 15, a combined image 402 obtained by combining the 2-th radiation image 320 and the 3-th radiation image 330 includes the 3-th IP overlapping portion corresponding image 322 of the 2-th radiation image 320, and does not include the 2-th IP overlapping portion corresponding image 331 of the 3-th radiation image 330.

As described above, the image processing unit 281 combines two read radiation images constituting one pair of radiation images to be combined for each of the (N-1) pairs of radiation images to be combined, to generate a composite image 400 in which a radiographed dental arch (a maxillary dental arch or a mandibular dental arch) is captured. Note that when N=2, the image processing unit 281 combines the 1-th radiation image 310 and the 2-th radiation image 320, and uses the resulting combined image 401 as the composite image 400.

Note that when combining the n-th radiation image and the (n+1)-th radiation image constituting one pair of radiation images to be combined, the image processing unit 281 may delete the upper (n+1)-th IP overlapping portion corresponding image from the n-th radiation image, from among the (n+1)-th IP overlapping portion corresponding image of the n-th radiation image and the n-th IP overlapping portion corresponding image of the (n+1)-th radiation image, which overlap each other. In this case, the image processing unit 281 connects the n-th radiation image, from which the (n+1)-th IP overlapping portion corresponding image has been deleted, and the (n+1)-th radiation image, to combine the n-th radiation image and the (n+1)-th radiation image. A combined image obtained by combining the n-th radiation image and the (n+1)-th radiation image does not include the (n+1)-th IP overlapping portion corresponding image of the n-th radiation image, but includes the n-th IP overlapping portion corresponding image of the (n+1)-th radiation image.

In the reading device 100, the imaging plate 2 from which a radiation image has been read may be discharged to the outlet 122 of the housing 120 after the radiation image has been erased from the imaging plate 2. In this case, the reading device 100 may include an erasing light source that irradiates the imaging plate 2 with erasing light for erasing the radiation image from the imaging plate 2. The erasing light may be white light, red visible light, or visible light of another color. The erasing light source may be an LED (Light Emitting Diode), a halogen lamp, or another light source.

When the radiation image is erased from the imaging plate 2, upon completion of the reading process, the transport mechanism 250 moves the placement member 200 to an erasing position by being driven by the drive unit 230. Next, the erasing light source irradiates the entire area of the radiation image forming layer 20 of the imaging plate 2 with erasing light under the control of the control unit 280. Thereby, the radiation image is erased from the imaging plate 2. After that, the imaging plate 2 from which the radiation image has been erased is discharged to the outlet 122.

In the above example, the reading device 100 switched the operation mode according to the operation accepted by the operation unit 140, but the method of switching the operation mode of the reading device 100 is not limited to this. For example, an external device (for example, a personal computer or a tablet terminal) connected to the reading device 100 may accept an instruction to switch the operation mode of the reading device 100 from a user, and notify the reading device 100 of the instruction to switch the operation mode. Further, an initial setting of the operation mode of the reading device 100 may be the normal mode, and switching between the normal mode and the synthesis mode may be arbitrarily performed according to a switching instruction from the user.

Further, the reading device 100 may combine a plurality of read radiation images read from a plurality of imaging plates in the normal mode, according to an instruction from a user. For example, the reading device 100 causes an external device to display a plurality of read radiation images read from a plurality of imaging plates in the normal mode. A user performs a predetermined operation on the external device to select a plurality of read radiation images to be combined from among the plurality of read radiation images displayed on the external device. The external device notifies the reading device 100 of the selected plurality of read radiation images to be combined. The reading device 100 synthesizes the plurality of read radiation images to be combined notified from the external device.

Further, the reading device 100 may display a plurality of read radiation images read from a plurality of imaging plates in the normal mode on the display unit 130, and a user may perform a predetermined operation on the operation unit 140 to select a plurality of read radiation images to be combined from among the plurality of read radiation images displayed on the display unit 130. Then, the reading device 100 may combine the selected plurality of read radiation images to be combined.

With the above configuration, even if there is a setting error in the operation mode, for example, a plurality of occlusally radiographed read radiation images can be selected from a group of read radiation images read in the normal mode, and can be synthesized later as read radiation images to be combined.

### <Regarding Registration Mark>

As described above, the registration mark 10 is made of a radiation shielding material. The radiation shielding material constituting the registration mark 10 may be, for example, a high radiation absorbing material. The material of the registration mark 10 may be, for example, copper, tungsten, titanium, or iron. The mark 10 does not completely shield radiation, but transmits some radiation. Note that the mark 10 may completely shield radiation.

Any method may be used to form the registration mark 10 on the holding member 5. For example, the registration mark 10 may be printed on the holding member 5. Further, the registration mark 10 may be made of a sticker material and attached to the holding member 5, or the registration mark 10 may be formed by metal foil stamping. Further, the registration mark 10 may be detachable from the holding member 5.

The image processing unit 281 can easily and appropriately register the plurality of read radiation images based on a mark image corresponding to the registration mark 10 included in the plurality of read radiation images. A plurality of examples of the registration mark 10 and a registration process using it will be described below. Hereinafter, a plurality of examples will be described mainly focusing on a case where N=2, that is, a case where the holding member 5A holds two imaging plates 2 as in the example of FIG. 7. Further, hereinafter, unless otherwise specified, the holding member 5A refers to the holding member 5A in a closed state, as shown in FIGS. 2 and 8. Further, a vertical direction of the holding member 5A means a direction along a vertical direction (a left and right direction in FIG. 2) of the surface to be fixed 51b of the holding member 5A in the closed state, and a horizontal direction of the holding member 5A means a direction along a horizontal direction (an up and down direction in FIG. 2) of the surface to be fixed 51b of the holding member 5A in the closed state. In the holding member 5A, the plurality of imaging plates 2 are placed such that their longitudinal directions are aligned with the vertical direction of the holding member 5A. It can also be said that the vertical direction of the holding member 5A is a direction along a front-rear direction of the oral cavity when the holding member 5A is placed in the oral cavity.

### <First Example (Use of Non-overlapping Portion Marks)>

In this example, the image processing unit 281 performs registration of the n-th radiation image and the (n+1)-th radiation image based on a mark image corresponding to a non-overlapping portion mark 10 (also referred to as an n-th IP non-overlapping portion mark 10) for recording a mark image in a portion of the n-th imaging plate 2 that does not overlap with the (n+1)-th imaging plate 2, and a mark image corresponding to a non-overlapping portion mark 10 (also referred to as an (n+1)-th IP non-overlapping portion mark 10) for recording a mark image in a portion of the (n+1)-th imaging plate 2 that does not overlap with the n-th imaging plate 2. For example, the image processing unit 281 performs registration of the n-th radiation image and the (n+1)-th radiation image based on the position of the mark image corresponding to the n-th IP non-overlapping portion mark 10 and the position of the mark image corresponding to the (n+1)-th IP non-overlapping portion mark 10.

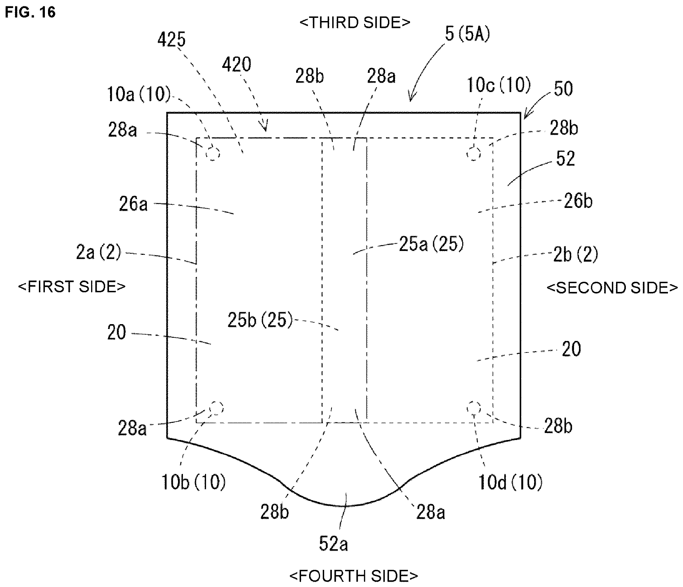

FIG. 16 is a schematic diagram showing an example of a holding member 5A and a 1-th imaging plate 2 and a 2-th imaging plate 2 held thereby. In the example of FIG. 16, a combined imaging plate 420 is composed of a 1-th imaging plate 2 and a 2-th imaging plate 2. Hereinafter, the 1-th imaging plate 2 may be referred to as a 1-th imaging plate 2a or an imaging plate 2a, and the 2-th imaging plate 2 may be referred to as a 2-th imaging plate 2b or an imaging plate 2b. In FIG. 16, the imaging plate 2a is shown by a dash-dotted line, and the imaging plate 2b is shown by a dashed line.

One end portion 25 in a short-side direction of the imaging plate 2a that overlaps with the imaging plate 2b becomes a 2-th IP overlapping portion. Further, one end portion 25 in a short-side direction of the imaging plate 2b that overlaps with the imaging plate 2a becomes a 1-th IP overlapping portion. Hereinafter, the 2-th IP overlapping portion may be referred to as an overlapping portion 25a, and the 1-th IP overlapping portion may be referred to as an overlapping portion 25b.

Hereinafter, for convenience of explanation, a first side and a second side of the holding member 5A mean a left side and a right side, respectively, of the holding member 5A when viewed from a side of the cover portion 52 with a tab portion 52a on a lower side (in other words, with a connection point between the cover portion 52 and the fixed portion 51 on an upper side), as shown in FIG. 16. Further, a side of the tab portion 52a of the holding member 5A may be referred to as a fourth side of the holding member 5A. Further, a side opposite to the fourth side of the holding member 5A (that is, a side of the connection point between the cover portion 52 and the fixed portion 51) may be referred to as a third side of the holding member 5A.

The holding member 5A is placed in the oral cavity such that the third side of the holding member 5A is located on a posterior side of the oral cavity (in other words, a posterior side), and the fourth side of the holding member 5A is located on an anterior side of the oral cavity (in other words, an anterior side). Then, when a maxillary dental arch is radiographed, the holding member 5A is placed in the oral cavity such that the cover portion 52 is located on an upper side of the oral cavity and the fixed portion 51 is located on a lower side of the oral cavity, so that the first side of the holding member 5A in the oral cavity is located on a right cheek side, and the second side of the holding member 5A in the oral cavity is located on a left cheek side. On the other hand, when a mandibular dental arch is radiographed, the holding member 5A is placed in the oral cavity such that the cover portion 52 is located on a lower side of the oral cavity and the fixed portion 51 is located on an upper side of the oral cavity, so that the first side of the holding member 5A in the oral cavity is located on a left cheek side, and the second side of the holding member 5A in the oral cavity is located on a right cheek side.

Further, hereinafter, for convenience of explanation, a first side and a second side of the imaging plate 2 mean a left side and a right side, respectively, of the imaging plate 2 when the holding member 5A is plan-viewed from a side of the cover portion 52 with a tab portion 52a of the holding member 5A holding the imaging plate 2 on a lower side (in other words, with a connection point between the cover portion 52 and the fixed portion 51 on an upper side), as shown in FIG. 16. In this example, one long side of the imaging plate 2 becomes the first side, and the other long side of the imaging plate 2 becomes the second side.

Further, for convenience of explanation, a third side of the imaging plate 2 means a side of a connection point between the cover portion 52 and the fixed portion 51 when the imaging plate 2 is held by the holding member 5A. Further, for convenience of explanation, a fourth side of the imaging plate 2 means a side of the tab portion 52a when the imaging plate 2 is held by the holding member 5A. In this example, one short side of the imaging plate 2 becomes the third side, and the other short side of the imaging plate 2 becomes the fourth side.

The imaging plate 2 is placed in the oral cavity such that the third side of the imaging plate 2 is located on a posterior side of the oral cavity (in other words, a posterior side), and the fourth side of the imaging plate 2 is located on an anterior side of the oral cavity (in other words, an anterior side). When a maxillary dental arch is radiographed, the imaging plate 2 is placed in the oral cavity such that the first side of the imaging plate 2 is located on a right cheek side, and the second side of the imaging plate 2 is located on a left cheek side. On the other hand, when a mandibular dental arch is radiographed, the imaging plate 2 is placed in the oral cavity such that the first side of the imaging plate 2 is located on a left cheek side, and the second side of the imaging plate 2 is located on a right cheek side.

As described above, in this example, the reading device 100 reads a radiation image from an intra-oral posterior short side of the imaging plate 2. Therefore, the third side of the imaging plate 2 becomes a reading start side of the radiation image.

As shown in FIG. 16, the imaging plate 2a has an overlapping portion 25a that overlaps with the imaging plate 2b, and a non-overlapping portion 26a that does not overlap with the imaging plate 2b. Further, the imaging plate 2b has an overlapping portion 25b that overlaps with the imaging plate 2a, and a non-overlapping portion 26b that does not overlap with the imaging plate 2a.

The holding member 5A includes, for example, a plurality of marks 10. The plurality of marks 10 include, for example, circular marks 10a, 10b, 10c, and 10d. The marks 10a and 10b are marks 10 for recording a mark image on the non-overlapping portion 26a of the imaging plate 2a. The marks 10c and 10d are marks 10 for recording a mark image on the non-overlapping portion 26b of the imaging plate 2b.

The marks 10a and 10b for recording a mark image on the non-overlapping portion 26a of the imaging plate 2a can also be referred to as non-overlapping portion marks 10. Further, the marks 10c and 10d for recording a mark image on the non-overlapping portion 26b of the imaging plate 2b can also be referred to as non-overlapping portion marks 10. The marks 10a and 10b are 1-th IP non-overlapping portion marks 10, and the marks 10c and 10d are 2-th IP non-overlapping portion marks 10.

The marks 10a and 10b are provided on a portion of the holding member 5A that faces the radiation image forming layer 20 of the imaging plate 2a. For example, the marks 10a and 10b are provided on an inner surface of a portion of the cover portion 52 of the holding member 5A that faces the radiation image forming layer 20 of the imaging plate 2a.

The marks 10a and 10b are provided on a portion of the holding member 5A that faces an end portion (in other words, an edge portion, a peripheral end portion, or a peripheral edge portion) 425 of a combined imaging plate 420 composed of the imaging plate 2a and the imaging plate 2b held by the holding member 5A. The end portion 425 of the combined imaging plate 420 can be said to be an end portion of one large imaging plate composed of the imaging plate 2a and the imaging plate 2b held by the holding member 5A. For example, the marks 10a and 10b are provided on an inner surface of a portion of the cover portion 52 that faces the end portion 425 of the combined imaging plate 420.

The imaging plate 2a has four corner portions 28a. The four corner portions 28a are included in the end portion 425 of the combined imaging plate 420. Of the four corner portions 28a, two corner portions 28a on a first side are included in the non-overlapping portion 26a, and two corner portions 28a on a second side are included in the overlapping portion 25a.

The cover portion 52 has four first facing portions that respectively face the four corner portions 28a of the imaging plate 2a. The mark 10a is provided, for example, on an inner surface of a first facing portion on a third side, among two first facing portions on the first side of the cover portion 52. The mark 10b is provided, for example, on an inner surface of a first facing portion on a fourth side, among the two first facing portions on the first side of the cover portion 52.

The marks 10c and 10d are provided on a portion of the holding member 5A that faces the radiation image forming layer 20 of the imaging plate 2b. For example, the marks 10c and 10d are provided on an inner surface of a portion that faces the radiation image forming layer 20 of the imaging plate 2b, of the cover portion 52 of the holding member 5A.

The marks 10c and 10d are provided on a portion that faces the end portion 425 of the combined imaging plate 420, of the holding member 5A. For example, the marks 10c and 10d are provided on an inner surface of a portion that faces the end portion 425 of the combined imaging plate 420, of the cover portion 52.

The imaging plate 2b has four corner portions 28b. The four corner portions 28b are included in the end portion 425 of the combined imaging plate 420. Of the four corner portions 28b, two corner portions 28b on a first side are included in the non-overlapping portion 26b, and two corner portions 28a on a second side are included in the overlapping portion 25b.

The cover portion 52 has four second facing portions that respectively face the four corner portions 28b of the imaging plate 2b. The mark 10c is provided, for example, on an inner surface of a second facing portion on a third side, among two second facing portions on a second side of the cover portion 52. The mark 10d is provided, for example, on an inner surface of a second facing portion on a fourth side, among the two second facing portions on the second side of the cover portion 52.

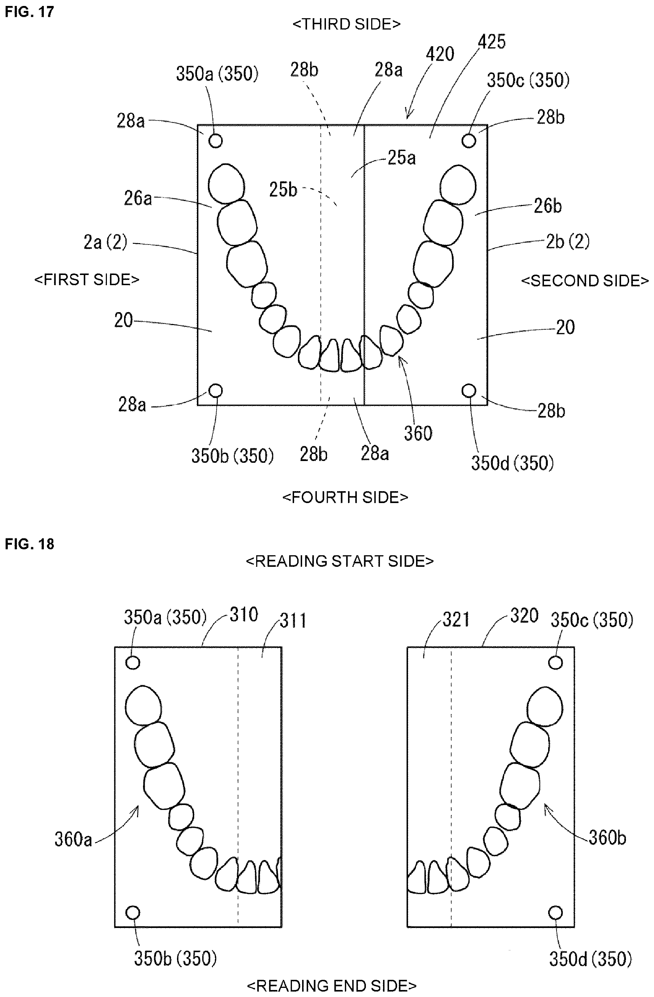

FIG. 17 is a diagram schematically showing an example of a radiation image recorded on the combined imaging plate 420 shown in FIG. 16. In FIG. 17, for convenience of explanation, an example of a radiation image recorded on the combined imaging plate 420 is shown by a solid line. FIG. 17 schematically shows a radiation image when a subject includes a dentition. Further, in FIG. 17, illustration of tissues other than teeth (for example, soft tissues such as gingiva and hard tissues such as jawbone) captured in an actual radiation image is omitted. The same applies to a diagram schematically showing an example of a radiation image recorded on the combined imaging plate 420, which will be described later.

A radiation image recorded on the combined imaging plate 420 (in other words, one large imaging plate) includes four mark images 350 respectively corresponding to four marks 10 included in the holding member 5A. The mark image 350 is an image representing the mark 10, and can also be said to be an image in which the mark 10 is captured. A shape of the mark image 350 is the same as a shape of the mark 10 corresponding to the mark image 350. Further, the radiation image recorded on the combined imaging plate 420 includes a dental arch image 360 representing a dental arch. It can also be said that the dental arch image 360 is an image in which a dental arch as a subject is captured. Further, the dental arch image 360 can also be said to be a subject image or an object image. The mark image 350 has a shape that can be distinguished from a subject image.

The four mark images 350 include mark images 350a, 350b, 350c, and 350d corresponding respectively to marks 10a, 10b, 10c, and 10d. The mark images 350a and 350b are recorded on the non-overlapping portion 26a of the imaging plate 2a. The mark images 350c and 350d are recorded on the non-overlapping portion 26b of the imaging plate 2b.

The mark images 350a, 350b, 350c, and 350d are recorded on the end portion 425 of the combined imaging plate 420. The marks 10a, 10b, 10c, and 10d corresponding to the mark images 350a, 350b, 350c, and 350d can also be referred to as end portion marks 10 for recording a mark image on the end portion 425 of the combined imaging plate 420.

The mark image 350a is recorded on a corner portion 28a on a third side, among two corner portions 28a on a first side of the imaging plate 2a, which are included in the end portion 425 of the combined imaging plate 420. The mark image 350b is recorded on a corner portion 28a on a fourth side, among the two corner portions 28a on the first side of the imaging plate 2a. The marks 10a and 10b corresponding to the mark images 350a and 350b can also be referred to as corner portion marks 10 for recording a mark image on the corner portion 28a of the imaging plate 2a.

The mark image 350c is recorded on a corner portion 28b on a third side, among two corner portions 28b on a second side of the imaging plate 2b, which are included in the end portion 425 of the combined imaging plate 420. The mark image 350d is recorded on a corner portion 28b on a fourth side, among the two corner portions 28b on the second side of the imaging plate 2b. The marks 10c and 10d corresponding to the mark images 350c and 350d can also be referred to as corner portion marks 10 for recording a mark image on the corner portion 28b of the imaging plate 2b.

In this example, the plurality of marks 10 included in the holding member 5A are configured such that all of a plurality of mark images 350 respectively corresponding to the plurality of marks 10 are recorded on the end portion 425 of the combined imaging plate 420. In the example of FIG. 17, all of the mark images 350a, 350b, 350c, and 350d included in the radiation image recorded on the combined imaging plate 420 are recorded on the end portion 425 of the combined imaging plate 420.

As can be understood from the above description, the mark 10 is provided on a portion of the holding member 5 that faces a portion of the radiation image forming layer 20 of the imaging plate 2 where a mark image corresponding to the mark 10 is recorded.

As in this example, when the plurality of mark images 350 recorded on the combined imaging plate 420 include a mark image 350 recorded on the end portion 425 of the combined imaging plate 420, the mark image 350 is less likely to overlap with a dental arch image 360 recorded on the combined imaging plate 420. That is, the mark image 350 is less likely to overlap with a subject image recorded on the combined imaging plate 420. Therefore, it becomes easier to obtain an appropriate subject image.

Further, as in this example, when the plurality of mark images 350 recorded on the combined imaging plate 420 include a mark image 350 recorded on a corner portion of the imaging plate 2, the mark image 350 is less likely to overlap with a subject image recorded on the combined imaging plate 420, and it becomes easier to obtain an appropriate subject image.

Further, as in this example, when all of a plurality of mark images 350 respectively corresponding to a plurality of marks 10 included in the holding member 5A are recorded on the end portion 425 of the combined imaging plate 420, the mark image 350 is less likely to overlap with a subject image recorded on the combined imaging plate 420, and it becomes easier to obtain an appropriate subject image (in other words, an object image). Thus, it is preferable that the mark image 350 is placed at a position that does not overlap with a region to be observed in the subject image. That is, it is preferable that the mark image 350 is placed at a position that does not interfere with interpretation.

FIG. 18 is a diagram schematically showing an example of a 1-th radiation image 310 and a 2-th radiation image 320 read from the imaging plates 2a and 2b shown in FIG. 17. Hereinafter, a 2-th IP overlapping portion corresponding image 311 included in the 1-th radiation image 310 may be referred to as an overlapping portion 311. Further, a 1-th IP overlapping portion corresponding image 321 included in the 2-th radiation image 320 may be referred to as an overlapping portion 321.

In FIG. 18, the 1-th radiation image 310 is shown such that a reading start side of the 1-th radiation image 310 in the reading device 100 is on an upper side of the figure. The same applies to the 2-th radiation image 320.

As described above, the reading device 100 reads a radiation image from a third side of the imaging plate 2. Therefore, in the 1-th radiation image 310, a side where an image read from an end portion on the third side of the 1-th imaging plate 2a is located (an image on a posterior side in the example of FIG. 17) becomes a reading start side for the 1-th radiation image 310 in the reading device 100. On the other hand, in the 1-th radiation image 310, a side where an image read from an end portion on a fourth side of the 1-th imaging plate 2a is located (an image on an anterior side in the example of FIG. 17) becomes a reading end side for the 1-th radiation image 310 in the reading device 100. The same applies to the 2-th radiation image 320.

In the example of FIG. 18, the 1-th radiation image 310 includes mark images 350a and 350b, and a dental arch image 360a that is a part of a dental arch image 360 shown in FIG. 17. The dental arch image 360a is an image representing a dentition recorded on the imaging plate 2a. The 2-th radiation image 320 includes mark images 350c and 350d, and a dental arch image 360b that is a part of the dental arch image 360. The dental arch image 360b is an image representing a dentition recorded on the imaging plate 2b. The overlapping portion 311 included in the 1-th radiation image 310 and the overlapping portion 321 included in the 2-th radiation image 320 show the same part of a dental arch of a subject.

The image processing unit 281 registers the 1-th radiation image 310 and the 2-th radiation image 320 based on the mark images 350a, 350b, 350c, and 350d, such that the overlapping portion 311 and the overlapping portion 321 overlap each other. An example of a method for registering the 1-th radiation image 310 and the 2-th radiation image 320 will be described below.

A first layout diagram 460 indicating relative positions of the plurality of marks 10 on the holding member 5 is stored in advance in a storage unit 280b of the control unit 280. The image processing unit 281 aligns the first radiographic image 310 and the second radiographic image 320 based on the first layout diagram 460 in the storage unit 280b and the mark images 350a, 350b, 350c, and 350d.

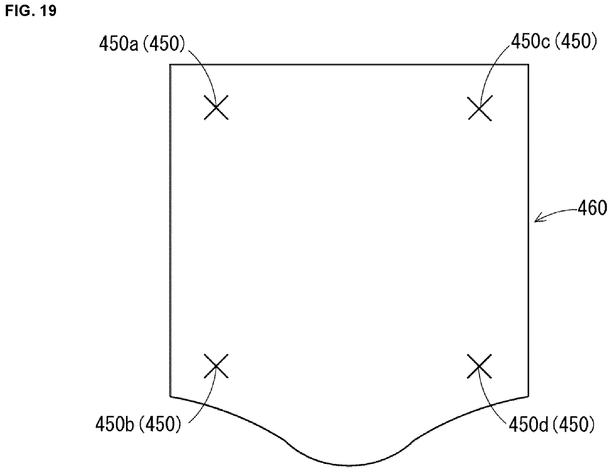

FIG. 19 is a schematic diagram showing an example of a first layout diagram 460. Relative positions of the plurality of marks 10 on the holding member 5 are constant regardless of a relative positional relationship of the plurality of imaging plates 2 held by the holding member 5. Therefore, even if a degree of overlap of the plurality of imaging plates 2 on the holding member 5 changes, the relative positions of the plurality of marks 10 on the holding member 5 do not change.

The first layout diagram 460 shows relative positions 450 of the plurality of marks 10 when the holding member 5A is viewed from a side from which radiation is irradiated, along a direction perpendicular to a reading surface 21 of the imaging plate 2. It can also be said that the first layout diagram 460 shows the relative positions 450 of the plurality of marks 10 when the holding member 5A is plan-viewed from a side of an outer surface of the cover portion 52, along a direction perpendicular to the reading surface 21 of the imaging plate 2. The first layout diagram 460 can also be referred to as layout information.

The first layout diagram 460 shows a position 450a of the mark 10a, a position 450b of the mark 10b, a position 450c of the mark 10c, and a position 450d of the mark 10d. The position 450 of the mark 10 shown in the first layout diagram 460 may be, for example, a position of a center of gravity of the mark 10. An outer shape of the first layout diagram 460 matches, for example, an outer shape of the holding member 5A when the holding member 5A is viewed from the side from which radiation is irradiated, along a direction perpendicular to the reading surface 21 of the imaging plate 2. Note that the outer shape of the first layout diagram 460 is not limited to this. The outer shape of the first layout diagram 460 may not match the outer shape of the holding member 5A. The first layout diagram 460 may have a size that can include a region to be diagnosed (in other words, to be observed) of a subject.

When the IP assembly 1 is viewed from a side from which radiation is irradiated, along a direction perpendicular to the reading surface 21 of the imaging plate 2, positions of a plurality of marks 10 and recording positions of a plurality of mark images 350 on a combined imaging plate 420 (in other words, on one large imaging plate) held by the holding member 5 match. Therefore, it can also be said that the first layout diagram 460 indicates relative recording positions of the plurality of mark images 350 on the combined imaging plate 420 when the combined imaging plate 420 held by the holding member 5 is viewed from the side from which radiation is irradiated, along a direction perpendicular to the reading surface 21 of the imaging plate 2. Further, it can also be said that the first layout diagram 460 indicates a relative positional relationship of the plurality of marks 10, and it can also be said that it indicates a relative positional relationship of recording positions of the plurality of mark images 350 on the combined imaging plate 420 held by the holding member 5A.

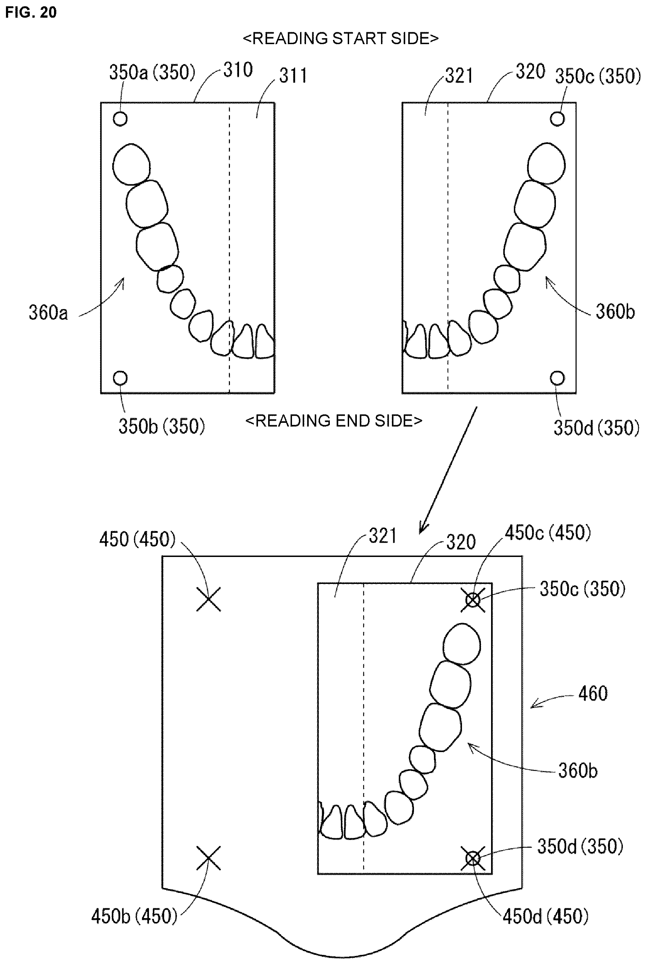

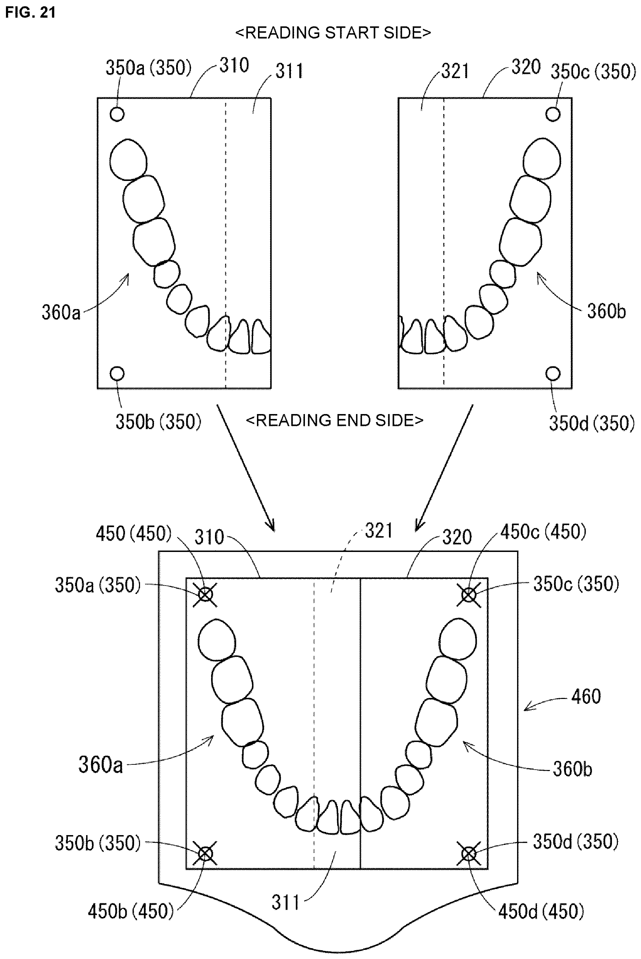

FIGS. 20 and 21 are schematic diagrams for explaining an example of a method for registering a 1-th radiation image 310 and a 2-th radiation image 320. As shown in FIG. 20, the image processing unit 281 places, for example, the 2-th radiation image 320 on the first layout diagram 460 such that a position (for example, a position of a center of gravity) of a mark image 350c corresponding to a mark 10c, included in the 2-th radiation image 320, matches a position 450c of the mark 10c in the first layout diagram 460, and a position (for example, a position of a center of gravity) of a mark image 350d corresponding to a mark 10d, included in the 2-th radiation image 320, matches a position 450d of the mark 10d in the first layout diagram 460.

Next, as shown in FIG. 21, the image processing unit 281 places, for example, the 1-th radiation image 310 on the first layout diagram 460 such that a position (for example, a position of a center of gravity) of a mark image 350a corresponding to a mark 10a, included in the 1-th radiation image 310, matches a position 450a of the mark 10a in the first layout diagram 460, and a position (for example, a position of a center of gravity) of a mark image 350b corresponding to a mark 10b, included in the 1-th radiation image 310, matches a position 450b of the mark 10b in the first layout diagram 460. Thereby, basically, the 1-th radiation image 310 and the 2-th radiation image 320 are registered such that an overlapping portion 311 of the 1-th radiation image 310 exactly overlaps an overlapping portion 321 of the 2-th radiation image 320.

Here, the image processing unit 281 does not know in advance which mark 10 of the holding member 5 corresponds to a mark image 350 included in a read radiation image. That is, the image processing unit 281 does not know in advance which mark image 350 included in the read radiation image corresponds to a position 450 of a certain mark 10 in the first layout diagram 460. Therefore, in order to register the 1-th radiation image 310 and the 2-th radiation image 320 as shown in FIGS. 20 and 21, the image processing unit 281 needs to identify which mark 10 corresponds to a mark image 350 included in a read radiation image.

In this example, the image processing unit 281 identifies two mark images 350 included in a first read radiation image read by the optical measuring instrument 290. Then, in the identified two mark images 350, the image processing unit 281 defines a mark image 350 on a reading start side as a mark image 350a corresponding to the mark 10a, and a mark image 350 on a reading end side as a mark image 350b corresponding to the mark 10b.

Further, the image processing unit 281 identifies two mark images 350 included in a second read radiation image read by the optical measuring instrument 290. Then, in the identified two mark images 350, the image processing unit 281 defines a mark image 350 on a reading start side as a mark image 350c corresponding to the mark 10c, and a mark image 350 on a reading end side as a mark image 350d corresponding to the mark 10d.

As described above, the first read radiation image read by the optical measuring instrument 290 is the 1-th radiation image 310. Further, as shown in FIG. 17 described above, a mark image 350a corresponding to the mark 10a is recorded on an end portion on a third side of the imaging plate 2a, and a mark image 350b corresponding to the mark 10b is recorded on an end portion on a fourth side of the imaging plate 2a. Then, as described above, the third side of the imaging plate 2 becomes the reading start side in the reading device 100, and the fourth side of the imaging plate 2 becomes the reading end side in the reading device 100. Therefore, of the two mark images 350 included in the read radiation image first read by the optical measuring instrument 290, the mark image 350 on the reading start side becomes the mark image 350a corresponding to the mark 10a, and the mark image 350 on the reading end side becomes the mark image 350b corresponding to the mark 10b. For the same reason, of the two mark images 350 included in the read radiation image second read by the optical measuring instrument 290, the mark image 350 on the reading start side becomes the mark image 350c corresponding to the mark 10c, and the mark image 350 on the reading end side becomes the mark image 350d corresponding to the mark 10d.

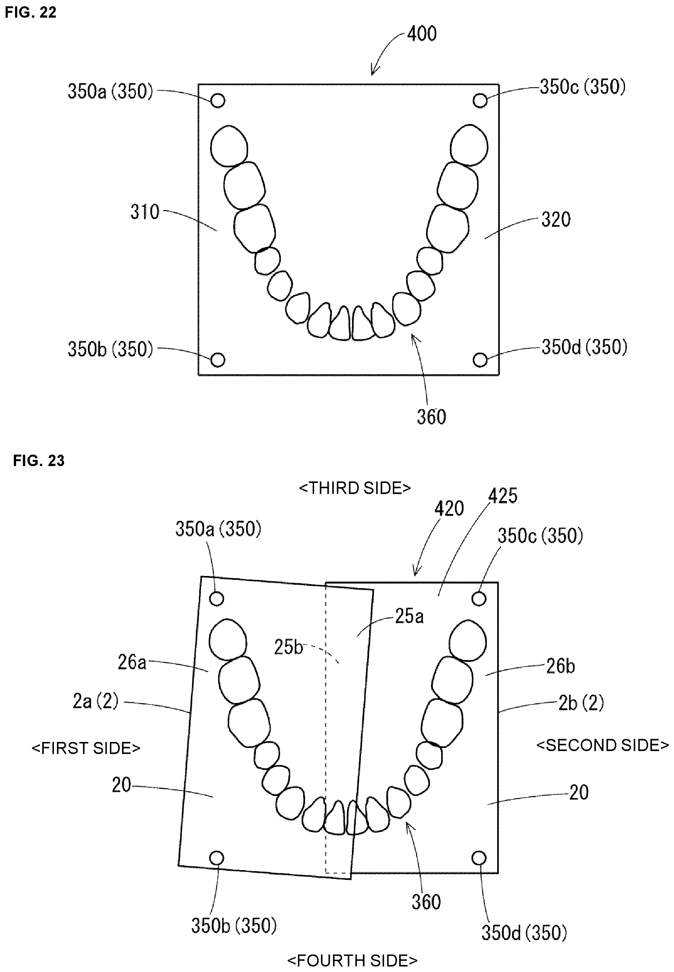

When the image processing unit 281 registers the 1-th radiation image 310 and the 2-th radiation image 320 as shown in FIG. 21, it deletes a lower overlapping portion 321 from the 2-th radiation image 320. Then, the image processing unit 281 connects the 2-th radiation image 320, from which the overlapping portion 321 has been deleted, and the 1-th radiation image 310. Thereby, the 1-th radiation image 310 and the 2-th radiation image 320 are combined, and as shown in FIG. 22, a composite image 400 including a dental arch image 360 of an object and mark images 350a, 350b, 350c, and 350d is generated. Since an overlapping portion 311 taken from an upper imaging plate 2a has a better image quality than an overlapping portion 321 read from a lower imaging plate 2b, by leaving the overlapping portion 311 and deleting the overlapping portion 321, an image quality of the composite image 400 can be improved. The composite image 400 generated by the image processing unit 281 is displayed on the display unit 130.

Note that when combining the 1-th radiation image 310 and the 2-th radiation image 320, the image processing unit 281 may delete the upper overlapping portion 311 from the 1-th radiation image 310 and connect the 1-th radiation image 310, from which the overlapping portion 311 has been deleted, and the 2-th radiation image 320.

As described above, in the holding member 5A, the imaging plates 2a and 2b are basically stacked such that their long sides are parallel and their short sides are aligned in a straight line. However, in the holding member 5A, at least one of the imaging plates 2a and 2b may be placed slightly displaced, and the long sides of the imaging plates 2a and 2b held by the holding member 5A may not be exactly parallel, or the short sides of the imaging plates 2a and 2b held by the holding member 5A may not be exactly aligned in a straight line. In this way, even when the overlap of the imaging plates 2a and 2b is slightly displaced, the image processing unit 281 can register the 1-th radiation image 310 and the 2-th radiation image 320 by operating in the same manner as described above, such that the overlapping portion 311 of the 1-th radiation image 310 and the overlapping portion 321 of the 2-th radiation image 320 overlap each other.

Note that in this disclosure, it is assumed that a displacement of the overlap of the imaging plates 2a and 2b is small. Even if the overlap of the imaging plates 2a and 2b is displaced, it is assumed that the long sides of the imaging plates 2a and 2b are substantially parallel, and an angle formed by the long side of the imaging plate 2a and the long side of the imaging plate 2b is within a few degrees or less. Further, even if the overlap of the imaging plates 2a and 2b is displaced, it is assumed that the short sides of the imaging plates 2a and 2b are substantially aligned in a straight line, and a positional displacement in a longitudinal direction of the imaging plate 2 between a short side on a third side of the imaging plate 2a and a short side on the third side of the imaging plate 2b (in other words, a positional displacement between a short side on a fourth side of the imaging plate 2a and a short side on the fourth side of the imaging plate 2b) is within a few mm or less. That is, it is assumed that the mark image 350 has a positional displacement to the extent that it is located approximately around a position 450 when compared with the first layout diagram 460.

FIG. 23 is a diagram schematically showing an example of a radiation image recorded on the combined imaging plate 420 when an overlap of the imaging plates 2a and 2b in the holding member 5A is slightly displaced.

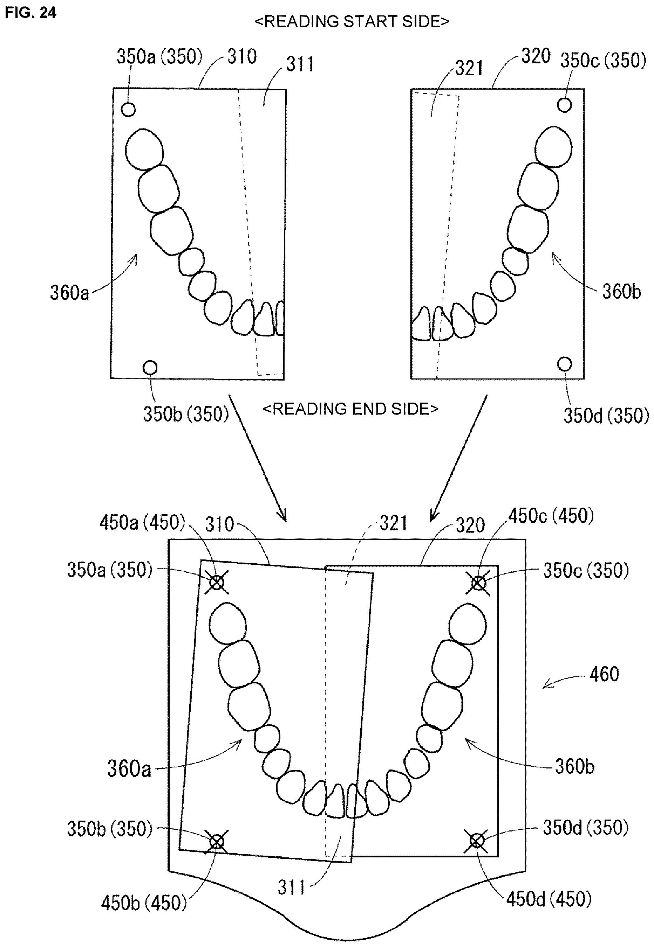

FIG. 24 is a schematic diagram for explaining an example of a method for registering a 1-th radiation image 310 and a 2-th radiation image 320 when a radiation image as shown in FIG. 23 is recorded on the combined imaging plate 420.

As illustrated in FIG. 24, the image processing unit 281 arranges a 2-th radiation image 320 on a first layout diagram 460 such that a position of a mark image 350c corresponding to a mark 10c included in the 2-th radiation image 320 matches a position 450c of the mark 10c in the first layout diagram 460, and a position of a mark image 350d corresponding to a mark 10d included in the 2-th radiation image 320 matches a position 450d of the mark 10d in the first layout diagram 460. The image processing unit 281 can identify which mark 10 corresponds to the mark image 350 included in the 2-th radiation image 320 in the same manner as described above.

Next, as illustrated in FIG. 24, the image processing unit 281 arranges a 1-th radiation image 310 on the first layout diagram 460 such that a position of a mark image 350a corresponding to a mark 10a included in the 1-th radiation image 310 matches a position 450a of the mark 10a in the first layout diagram 460, and a position of a mark image 350b corresponding to a mark 10b included in the 1-th radiation image 310 matches a position 450b of the mark 10b in the first layout diagram 460. Thereby, basically, the 1-th radiation image 310 and the 2-th radiation image 320 are registered such that an overlapping portion 311 of the 1-th radiation image 310 precisely overlaps over an overlapping portion 321 of the 2-th radiation image 320. The image processing unit 281 can identify which mark 10 corresponds to the mark image 350 included in the 1-th radiation image 310 in the same manner as described above.

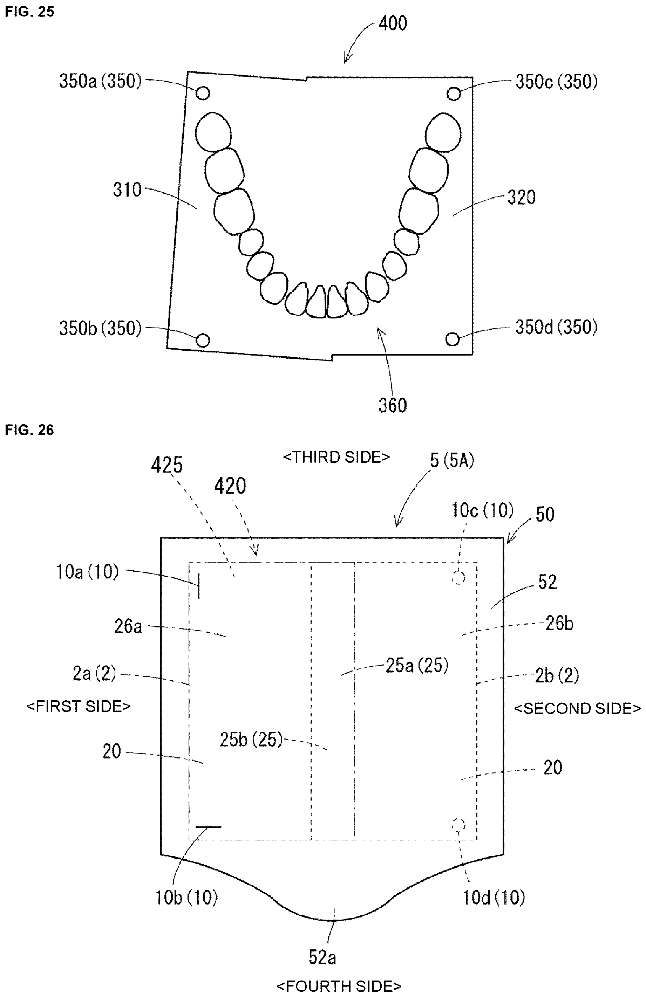

When the image processing unit 281 performs registration of the 1-th radiation image 310 and the 2-th radiation image 320, it combines the 1-th radiation image 310 and the 2-th radiation image 320 as described above. Thereby, as illustrated in FIG. 25, a composite image 400 is generated, which includes a dental arch image 360 of a subject of radiography and the mark images 350a, 350b, 350c, and 350d.

The location where the mark image 350 is recorded on the imaging plate 2 is not limited to the above-described example. For example, the plurality of mark images 350 recorded on the first imaging plate 2a may include a mark image 350 recorded at a location other than a corner portion 28a of the imaging plate 2a, or may not include a mark image 350 recorded at the corner portion 28a of the imaging plate 2a. The same applies to the imaging plate 2b.

Further, the plurality of mark images 350 recorded on a combined imaging plate 420 may include a mark image 350 recorded at a location other than an end portion 425 of the combined imaging plate 420, or may not include a mark image 350 recorded at the end portion 425.

In the above-described example, the mark 10 is circular (specifically, a perfect circle), but is not limited thereto. For example, the mark 10 may be a line segment, a curved line, a polygon, a numeral, a character such as an alphabet letter, a symbol, or a pattern.

Further, the plurality of marks 10 provided on the holding member 5 may include a plurality of marks 10 with mutually different shapes. For example, the plurality of marks 10 provided on the holding member 5 may include a line-segment-shaped (in other words, bar-shaped) mark 10, a mark 10 indicating an alphabet letter, and a mark 10 indicating a numeral. Alternatively, the plurality of marks 10 may include a plurality of marks 10 having the same shape but different sizes (for example, a short line segment and a long line segment).

Further, the plurality of marks 10 provided on the holding member 5 may include both a mark 10 provided on an inner surface of a protection cover 52 and a mark 10 provided on an outer surface of the protection cover 52, or may not include the mark 10 provided on the inner surface of the protection cover 52.

FIG. 26 is a schematic diagram illustrating another example of a holding member 5A. In the example of FIG. 26, marks 10a and 10b included in the holding member 5A are line segments instead of being circular. Further, the marks 10a and 10b are provided on the outer surface of the protection cover 52, not on the inner surface.

In the example of FIG. 26, line-segment-shaped mark images 350a and 350b corresponding to the line-segment-shaped marks 10a and 10b, respectively, are recorded along an edge of the imaging plate 2a. Specifically, the mark image 350a is recorded along a long side on a first side of the imaging plate 2a, and the mark image 350b is recorded along a short side on a fourth side of the imaging plate 2a. In this way, when the mark image 350 is recorded along the edge of the imaging plate 2, the mark image 350 is less likely to overlap with a subject image.

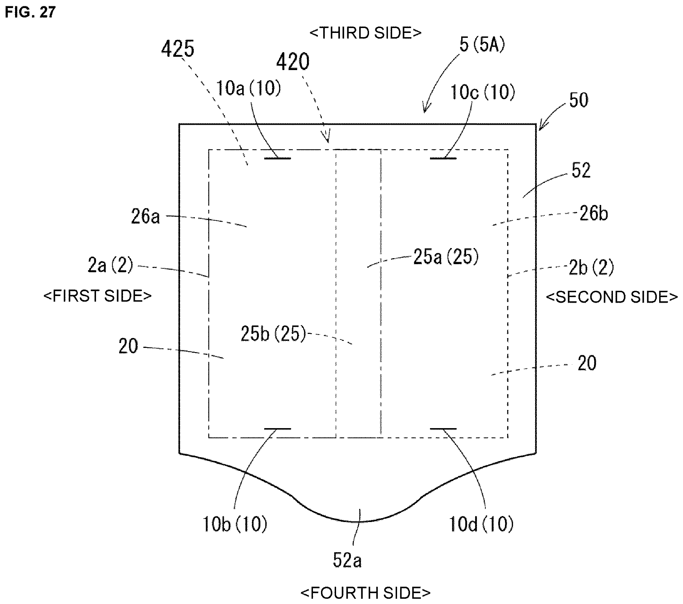

FIG. 27 is a schematic diagram illustrating another example of the holding member 5A. In the example of FIG. 27, marks 10a, 10b, 10c, and 10d included in the holding member 5A are line segments instead of being circular. Further, the marks 10a, 10b, 10c, and 10d are provided on the outer surface of the protection cover 52, not on the inner surface.

Since the line-segment-shaped marks illustrated in FIG. 27 have a shape with no thickness, imaging toward a center of the imaging plate 2a is reduced, so that they are less likely to interfere with a radiation image of a subject to be observed, and can be said to be a clinically desirable shape.

In the example of FIG. 27, line-segment-shaped mark images 350a and 350b corresponding to the line-segment-shaped marks 10a and 10b, respectively, are recorded along the edge of the imaging plate 2a. Specifically, the line-segment-shaped mark image 350a is recorded at a central portion in a short-side direction of the imaging plate 2a at an end portion on a third side of the imaging plate 2a, along an edge on the third side of the imaging plate 2a. Further, the line-segment-shaped mark image 350b is recorded at a central portion in the short-side direction of the imaging plate 2a at an end portion on a fourth side of the imaging plate 2a, along an edge on the fourth side of the imaging plate 2a.

Line-segment-shaped mark images 350c and 350d corresponding to the line-segment-shaped marks 10c and 10d, respectively, are recorded along an edge of the imaging plate 2b. Specifically, the line-segment-shaped mark image 350c is recorded at a central portion in a short-side direction of the imaging plate 2b at an end portion on a third side of the imaging plate 2b, along an edge on the third side of the imaging plate 2b. Further, the line-segment-shaped mark image 350d is recorded at a central portion in the short-side direction of the imaging plate 2b at an end portion on a fourth side of the imaging plate 2b, along an edge on the fourth side of the imaging plate 2b.

The above-described example was a case where N=2, but even in a case where N≥3 and the holding member 5A holds three or more imaging plates 2, similarly to the above-described registration of the 1-th radiation image and the 2-th radiation image, the image processing unit 281 can, for each of two or more pairs of radiation images to be combined, perform registration of the two read radiation images constituting the pair of radiation images to be combined, based on a plurality of mark images 350 included in the two read radiation images.

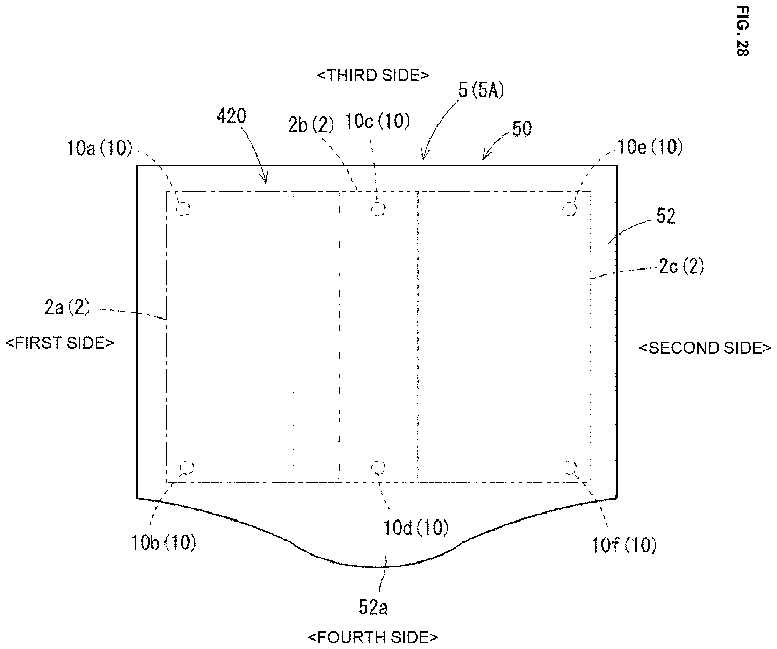

FIG. 28 is a schematic diagram illustrating an example of the holding member 5A and a 1-th imaging plate 2a, a 2-th imaging plate 2b, and a 3-th imaging plate 2 held thereby, in a case where N=3. Hereinafter, the 3-th imaging plate 2 may be referred to as a 3-th imaging plate 2c or the imaging plate 2c. In FIG. 28, the imaging plate 2c is indicated by a two-dot chain line.

In the example of FIG. 28, the plurality of marks 10 provided on the holding member 5A includes, in addition to the marks 10a, 10b, 10c, and 10d, two marks 10e and 10f for recording a mark image 350 on the imaging plate 2c. Two mark images 350 corresponding to the marks 10e and 10f are recorded on a portion of the imaging plate 2c that does not overlap with the imaging plate 2b. Further, two mark images 350 corresponding to the marks 10c and 10d are recorded on a portion of the imaging plate 2b that does not overlap with the imaging plate 2a and the imaging plate 2c.

The first layout diagram 460 in a storage unit 280b indicates relative positions 450 of the marks 10a, 10b, 10c, 10d, 10e, and 10f on the holding member 5A. The image processing unit 281, for example, arranges a 3-th radiation image 330, which is read from the 3-th imaging plate 2c, on the first layout diagram 460 such that positions of two mark images 350 included in the 3-th radiation image 330 respectively match positions 450 of two marks 10, which are indicated in the first layout diagram 460 and correspond to the two mark images 350. Next, the image processing unit 281 arranges the 2-th radiation image 320 on the first layout diagram 460 in the same manner as in FIG. 20. Thereby, registration of the 2-th radiation image 320 and the 3-th radiation image 330, which constitute one pair of radiation images to be combined, is completed. Then, the image processing unit 281 arranges the 1-th radiation image 310 on the first layout diagram 460 in the same manner as in FIG. 21. Thereby, registration of the 1-th radiation image 310 and the 2-th radiation image 320, which constitute one pair of radiation images to be combined, is completed. As a result, basically, the 1-th radiation image 310, the 2-th radiation image 320, and the 3-th radiation image 330 are registered such that an 3-th IP overlapping portion corresponding image 322 of the 2-th radiation image 320 precisely overlaps with a 2-th IP overlapping portion corresponding image 331 of the 3-th radiation image 330, and a 2-th IP overlapping portion corresponding image 311 of the 1-th radiation image 310 precisely overlaps with a 1-th IP overlapping portion corresponding image 321 of the 2-th radiation image 320. Thereafter, the image processing unit 281 combines the 1-th radiation image 310, the 2-th radiation image 320, and the 3-th radiation image 330 to generate the composite image 400.

Note that a method for identifying which mark 10 corresponds to the mark image 350 included in a 3-th radiation image 330 is the same as the method for identifying which mark 10 correspond to the mark image 350 included in the 1-th radiation image 310 and the mark image 350 included in the 2-th radiation image 320.

In this example, the image processing unit 281 registers an n-th radiation image and an (n+1)-th radiation image based on a position of a mark image 350 corresponding to a n-th IP non-overlapping portion mark 10 and a position of a mark image 350 corresponding to an (n+1)-th IP non-overlapping portion mark 10. In such a case, the image processing unit 281 can perform registration of the n-th radiation image and the (n+1)-th radiation image in the same manner as described above, by using mark images corresponding to at least two n-th IP non-overlapping portion mark 10 and mark images corresponding to at least two (n+1)-th IP non-overlapping portion mark 10. Therefore, for example, in a case where N=2, the holding member 5 may be provided with three or more 1-th IP non-overlapping portion mark 10, or may be provided with three or more 2-th IP non-overlapping portion mark 10. Further, the number of the 1-th IP non-overlapping portion mark 10 and the number of the 2-th IP non-overlapping portion mark 10 may be different from each other.

### <Second Example (Use of Overlapping Portion Mark)>

In this example, the image processing unit 281 registers an n-th radiation image and an (n+1)-th radiation image by using a mark image 350 corresponding to an overlapping portion mark 10 (also referred to as an overlapping portion mark 10 of an n-th IP and an (n+1)-th IP) for recording a mark image on an overlapping portion between an n-th imaging plate 2 and an (n+1)-th imaging plate 2. For example, the image processing unit 281 registers the n-th radiation image and the (n+1)-th radiation image based on a position of the mark image 350 corresponding to the overlapping portion mark 10 of the n-th IP and the (n+1)-th IP.

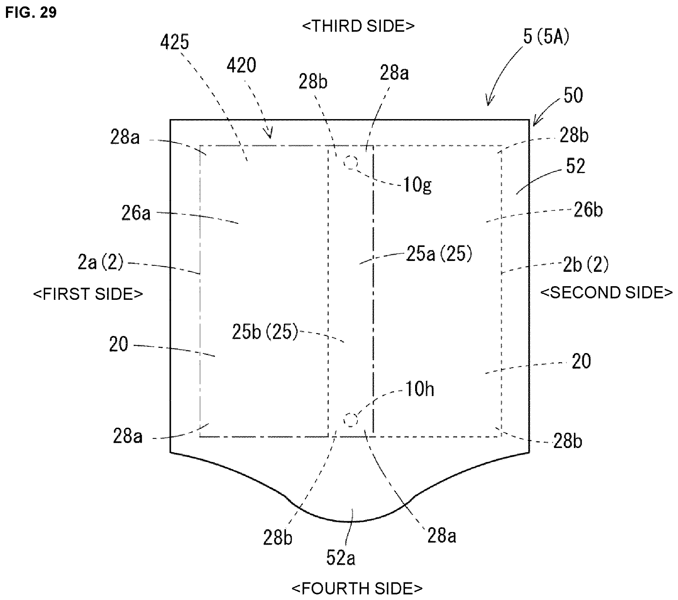

FIG. 29 is a schematic diagram illustrating an example of the holding member 5A. In the example of FIG. 29, the plurality of marks 10 provided on the holding member 5A includes marks 10g and 10h for recording a mark image on an overlapping portion 25a of the imaging plate 2a and an overlapping portion 25b of the imaging plate 2b. The marks 10g and 10h can also be referred to as overlapping portion marks 10 of a 1-th IP and a 2-th IP. The marks 10g and 10h are provided, for example, on an inner surface of a portion of the protection cover 52 that faces the overlapping portions 25a and 25b.

A corner portion 28a on a second side and a third side of the imaging plate 2a (also referred to as a first corner portion 28a) and a corner portion 28b on a first side and a third side of the imaging plate 2b (also referred to as a first corner portion 28b) overlap with each other. The mark 10g is provided on an inner surface of a portion of the protection cover 52 that faces the first corner portion 28a and the first corner portion 28b.

A corner portion 28a on a second side and a fourth side of the imaging plate 2a (also referred to as a second corner portion 28a) and a corner portion 28b on a first side and a fourth side of the imaging plate 2b (also referred to as a second corner portion 28b) overlap with each other. The mark 10h is provided on an inner surface of a portion of the protection cover 52 that faces the second corner portion 28a and the second corner portion 28b.

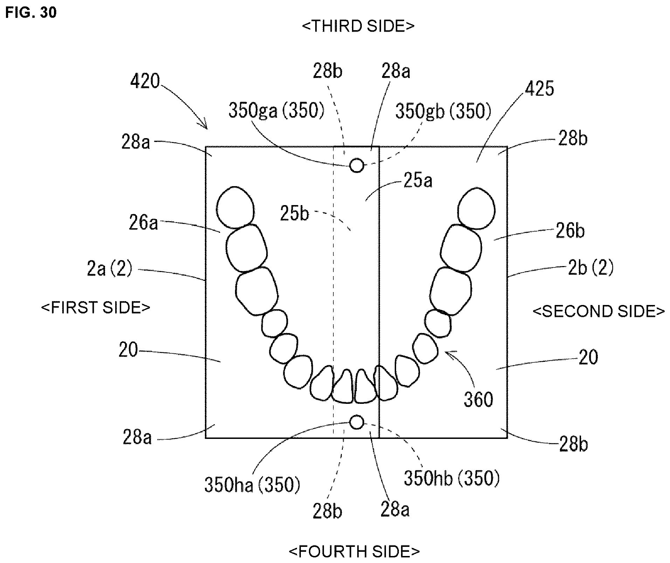

FIG. 30 is a diagram schematically illustrating an example of a radiation image recorded on the combined imaging plate 420 illustrated in FIG. 29.

On each of the imaging plate 2a and the imaging plate 2b, a mark image 350 corresponding to the mark 10g is recorded. Specifically, a mark image 350ga corresponding to the mark 10g is recorded on the overlapping portion 25a of the imaging plate 2a, and a mark image 350gb corresponding to the mark 10g is recorded on the overlapping portion 25b of the imaging plate 2b.

The mark images 350ga and 350gb are recorded on the end portion 425 of the combined imaging plate 420. The mark image 350ga is recorded on the first corner portion 28a of the imaging plate 2a, and the mark image 350gb is recorded on the first corner portion 28b of the imaging plate 2b.

On each of the imaging plate 2a and the imaging plate 2b, a mark image 350 corresponding to the mark 10h is recorded. Specifically, a mark image 350ha corresponding to the mark 10h is recorded on the overlapping portion 25a of the imaging plate 2a, and a mark image 350hb corresponding to the mark 10h is recorded on the overlapping portion 25b of the imaging plate 2b.

The mark images 350ha and 350hb are recorded on the end portion 425 of the combined imaging plate 420. The mark image 350ha is recorded on the second corner portion 28a of the imaging plate 2a, and the mark image 350hb is recorded on the second corner portion 28b of the imaging plate 2b.

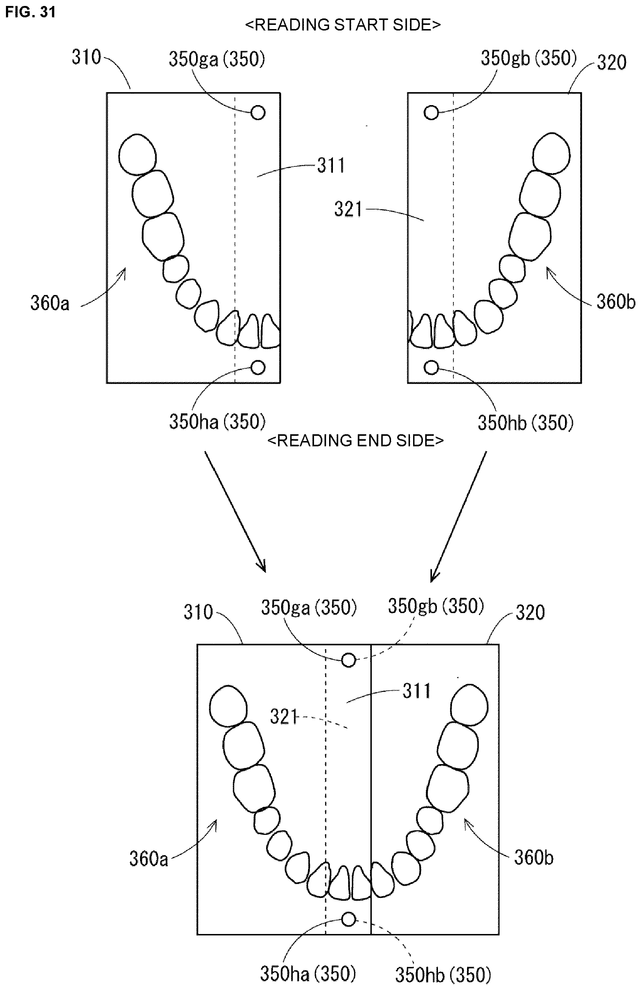

FIG. 31 is a schematic diagram for explaining an example of a method for registering the 1-th radiation image 310 and the 2-th radiation image 320, which are read from the imaging plate 2a and the imaging plate 2b held by the holding member 5A illustrated in FIG. 29. The upper side of FIG. 31 illustrates the 1-th radiation image 310 and the 2-th radiation image 320 before registration, and the lower side of FIG. 31 illustrates the 1-th radiation image 310 and the 2-th radiation image 320 after the registration.

In this example, the image processing unit 281 registers a plurality of read radiation images without using the first layout diagram 460. As illustrated in FIG. 31, the image processing unit 281 partially overlaps the 1-th radiation image 310 and the 2-th radiation image 320 such that positions of a mark image 350ga and a mark image 350gb corresponding to the same mark 10g match each other, and positions of a mark image 350ha and a mark image 350hb corresponding to the same mark 10h match each other. At this time, the image processing unit 281, for example, positions the 1-th radiation image 310 above the 2-th radiation image 320. Thereby, basically, the 1-th radiation image 310 and the 2-th radiation image 320 are registered such that the overlapping portion 311 of the 1-th radiation image 310 precisely overlaps over the overlapping portion 321 of the 2-th radiation image 320.

Here, as described above, the image processing unit 281 does not know in advance which mark 10 of the holding member 5 corresponds to the mark image 350 included in each read radiation image. In this example, the image processing unit 281 defines, among two mark images 350 included in a read radiation image first read by an optical measuring instrument 290, the mark image 350 on a reading start side as a mark image 350ga corresponding to the mark 10g, and defines the mark image 350 on a reading end side as a mark image 350ha corresponding to the mark 10h. Further, the image processing unit 281 defines, among two mark images 350 included in a read radiation image second read by the optical measuring instrument 290, the mark image 350 on the reading start side as a mark image 350gb corresponding to the mark 10g, and defines the mark image 350 on the reading end side as a mark image 350hb corresponding to the mark 10h.

The read radiation image first read by the optical measuring instrument 290 is the 1-th radiation image 310. Further, as illustrated in the above-described FIG. 30, the mark image 350ga is recorded on an end portion on the third side of the imaging plate 2a, and the mark image 350ha is recorded on an end portion on the fourth side of the imaging plate 2a. Then, the third side of the imaging plate 2 becomes the reading start side in a reading device 100, and the fourth side of the imaging plate 2 becomes the reading end side in the reading device 100. Therefore, among the two mark images 350 included in the read radiation image first read by the optical measuring instrument 290, the mark image 350 on the reading start side becomes the mark image 350ga corresponding to the mark 10g, and the mark image 350 on the reading end side becomes the mark image 350ha corresponding to the mark 10h. For the same reason, among the two mark images 350 included in the read radiation image second read by the optical measuring instrument 290, the mark image 350 on the reading start side becomes the mark image 350gb corresponding to the mark 10g, and the mark image 350 on the reading end side becomes the mark image 350hb corresponding to the mark 10h.

When the image processing unit 281 performs registration of the 1-th radiation image 310 and the 2-th radiation image 320, as illustrated in the lower side of FIG. 31, it combines the 1-th radiation image 310 and the 2-th radiation image 320 to generate the composite image 400 in the same manner as described above. This composite image 400 includes the mark images 350ga and 350ha included in the 1-th radiation image 310, but does not include the mark images 350gb and 350hb included in the 2-th radiation image 320.

The above-described example was a case where N=2, but even in a case where N≥3 and the holding member 5A holds three or more imaging plates 2, similarly to the above-described registration of the 1-th radiation image and the 2-th radiation image, the image processing unit 281 can, for each of two or more pairs of radiation images to be combined, perform registration of the two read radiation images constituting one pair of radiation images to be combined, based on a plurality of mark images 350 included in the two read radiation images.

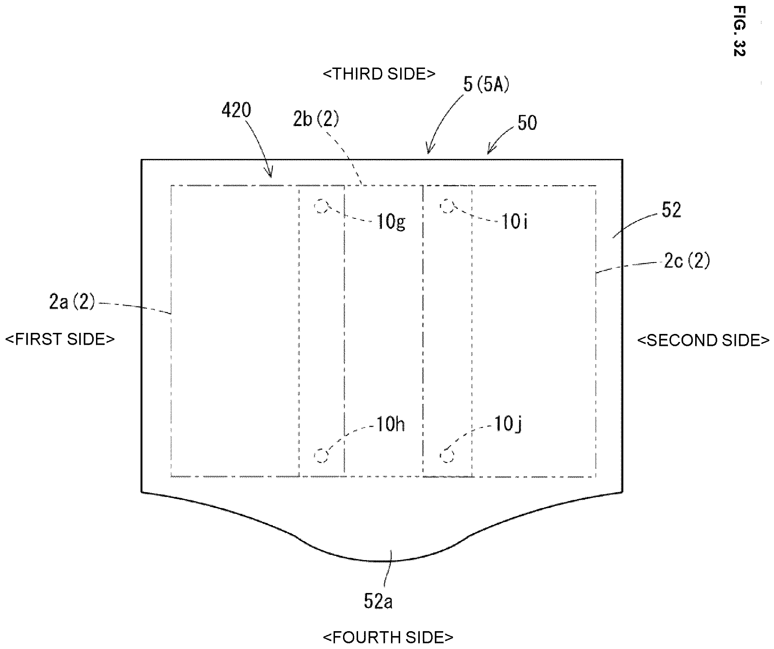

FIG. 32 is a schematic diagram illustrating an example of the holding member 5A and a 1-th imaging plate 2a, a 2-th imaging plate 2b, and a 3-th imaging plate 2c held thereby, in a case where N=3.

In the example of FIG. 32, the plurality of marks 10 provided on the holding member 5A includes, in addition to the marks 10g and 10h, two marks 10i and 10j for recording a mark image 350 on a portion of the third imaging plate 2c that overlaps with the imaging plate 2b. Two mark images 350 corresponding to the marks 10i and 10j are recorded on a portion of the imaging plate 2c that overlaps with the imaging plate 2b.

Since the marks 10g and 10h are the marks 10 for recording a mark image on an overlapping portion between the imaging plate 2a and the imaging plate 2b, they are the overlapping portion marks 10 in a relationship between the imaging plate 2a and the imaging plate 2b. On the other hand, since the marks 10g and 10h can also be said to be the marks 10 for recording a mark image on a portion of the imaging plate 2b that does not overlap with the imaging plate 2c, they serve as non-overlapping portion marks 10 in a relationship between the imaging plate 2b and the imaging plate 2c.

The image processing unit 281, for example, partially overlaps the 2-th radiation image 320 and the 3-th radiation image 330 such that positions of two mark images 350 included respectively in the 2-th radiation image 320 and the 3-th radiation image 330, which correspond to the same mark 10i, match each other, and positions of two mark images 350 included respectively in the 2-th radiation image 320 and the 3-th radiation image 330, which correspond to the same mark 10j, match each other. At this time, the image processing unit 281, for example, positions the 2-th radiation image 320 above the 3-th radiation image 330. Thereby, registration of the 2-th radiation image 320 and the 3-th radiation image 330, which constitute one pair of radiation images to be combined, is completed. Next, the image processing unit 281 registers the 2-th radiation image 320 on the 3-th radiation image 330 and the 1-th radiation image 310, based on the mark images 350 corresponding to the marks 10g and 10h, in the same manner as in FIG. 31. Thereby, registration of the 1-th radiation image 310 and the 2-th radiation image 320, which constitute one pair of radiation images to be combined, is completed. As a result, basically, the 1-th radiation image 310, the 2-th radiation image 320, and the 3-th radiation image 330 are registered such that an 3-th IP overlapping portion corresponding image 322 of the 2-th radiation image 320 precisely overlaps over a 2-th IP overlapping portion corresponding image 321 of the 3-th radiation image 330, and a 2-th IP overlapping portion corresponding image 311 of the 1-th radiation image 310 precisely overlaps over a 1-th IP overlapping portion corresponding image 321 of the 2-th radiation image 320. Thereafter, the image processing unit 281 combines the 1-th radiation image 310, the 2-th radiation image 320, and the 3-th radiation image 330 to generate the composite image 400.

As described above, in this example, the image processing unit 281 registers the n-th radiation image and the (n+1)-th radiation image based on the position of the mark image 350 corresponding to the overlapping portion mark 10 of the n-th IP and the (n+1)-th IP. In such a case, the image processing unit 281 can perform registration of the n-th radiation image and the (n+1)-th radiation image in the same manner as described above by using at least two or more overlapping portion marks 10 of the n-th IP and the (n+1)-th IP. Therefore, for example, in a case where N=2, the holding member 5 may be provided with three or more overlapping portion marks 10 of the first IP and the second IP.

Further, the position of the overlapping portion mark 10 of the n-th IP and the (n+1)-th IP used in the registration of the n-th radiation image and the (n+1)-th radiation image is not limited to the above-described example. The position of each mark 10 in the present disclosure may be any position that is unlikely to interfere with a radiation image to be observed during diagnosis. The same applies to marks described later.

### <Third Example (Use of Non-Overlapping Portion Mark and Overlapping Portion Marks)>

In this example, the image processing unit 281 registers an n-th radiation image and an (n+1)-th radiation image by using a mark image 350 corresponding to a non-overlapping portion mark 10 of an n-th IP, a mark image 350 corresponding to a non-overlapping portion mark 10 of an (n+1)-th IP, and a mark image 350 corresponding to an overlapping portion mark 10 of the n-th IP and the (n+1)-th IP. For example, the image processing unit 281 registers the n-th radiation image and the (n+1)-th radiation image based on a position of the mark image 350 corresponding to the non-overlapping portion mark 10 of the n-th IP, a position of the mark image 350 corresponding to the non-overlapping portion mark 10 of the (n+1)-th IP, and a position of the mark image 350 corresponding to the overlapping portion mark 10 of the n-th IP and the (n+1)-th IP.

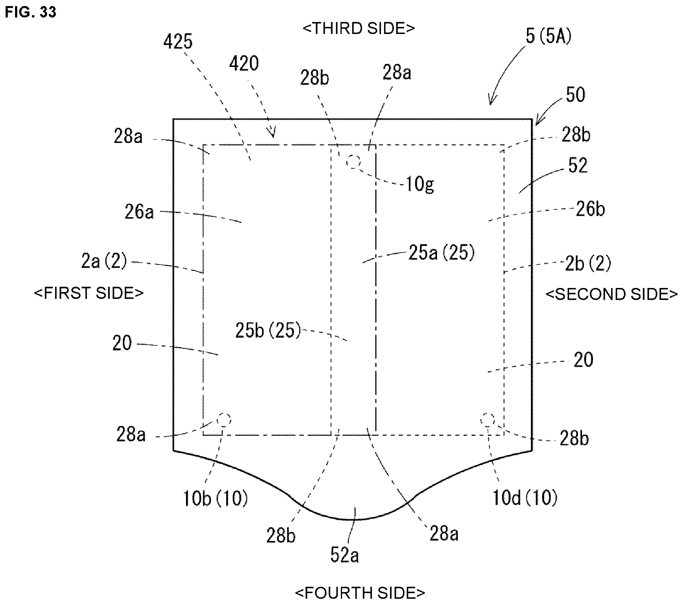

FIG. 33 is a schematic diagram illustrating an example of the holding member 5A. In the example of FIG. 33, the plurality of marks 10 provided on the holding member 5A includes the above-described marks 10b, 10d, and 10g.

The first layout diagram 460 in the storage unit 280b indicates relative positions 450 of the marks 10b and 10d on the holding member 5A. The first layout diagram 460 indicates a position 450b of the mark 10b and a position 450d of the mark 10d (see FIG. 19). The mark 10b is a non-overlapping portion mark 10 of a 1-th IP, and the mark 10d is a non-overlapping portion mark 10of a 2-th IP. The mark 10g is the overlapping portion mark 10 of the 1-th IP and the 2-th IP.

The image processing unit 281, for example, arranges the 1-th radiation image 310 and the 2-th radiation image 320 on the first layout diagram 460 such that a position of a mark image 350ga (see FIG. 31) corresponding to the mark 10g included in the 1-th radiation image 310 and a position of a mark image 350gb (see FIG. 31) corresponding to the mark 10g included in the 2-th radiation image 320 match each other, while a mark image 350b (see FIGS. 21 and 24) corresponding to the mark 10b included in the 1-th radiation image 310 and a mark image 350d (see FIGS. 21 and 24) corresponding to the mark 10d included in the 2-th radiation image 320 respectively match positions 450b and 450d indicated in the first layout diagram 460. At this time, the 1-th radiation image 310 is positioned above the 2-th radiation image 320. Thereby, similarly to the lower side of FIGS. 21 and 24, basically, the 1-th radiation image 310 and the 2-th radiation image 320 are registered such that the overlapping portion 311 of the 1-th radiation image 310 precisely overlaps over the overlapping portion 321 of the 2-th radiation image 320.

The image processing unit 281 defines, among two mark images 350 included in the 1-th radiation image 310, the mark image 350 on the reading start side as a mark image 350ga corresponding to the mark 10g, and defines the mark image 350 on the reading end side as a mark image 350b corresponding to the mark 10b. Similarly, the image processing unit 281 defines, among two mark images 350 included in a 2-th radiation image 320, the mark image 350 on the reading start side as a mark image 350gb corresponding to the mark 10g, and defines the mark image 350 on the reading end side as a mark image 350d corresponding to the mark 10d.

Note that the first layout diagram 460 may also indicate a position 450 of the mark 10g. In this case, the image processing unit 281, for example, arranges the 2-th radiation image 320 on the first layout diagram 460 such that positions of two mark images 350 respectively corresponding to the marks 10g and 10d included in the 2-th radiation image 320 match the positions 450 of the marks 10g and 10d indicated in the first layout diagram 460. Thereafter, the image processing unit 281 arranges the 1-th radiation image 310 on the first layout diagram 460 such that positions of two mark images 350 respectively corresponding to the marks 10g and 10b included in the 1-th radiation image 310 match the positions 450 of the marks 10g and 10b indicated in the first layout diagram 460.

The above-described example was a case where N=2, but even in a case where N≥3 and the holding member 5A holds three or more imaging plates 2, similarly to the above-described registration of the 1-th radiation image and the 2-th radiation image, the image processing unit 281 can, for each of two or more pairs of radiation images to be combined, perform registration of the two read radiation images constituting one pair of radiation images to be combined, based on a plurality of mark images 350 included in the two read radiation images.

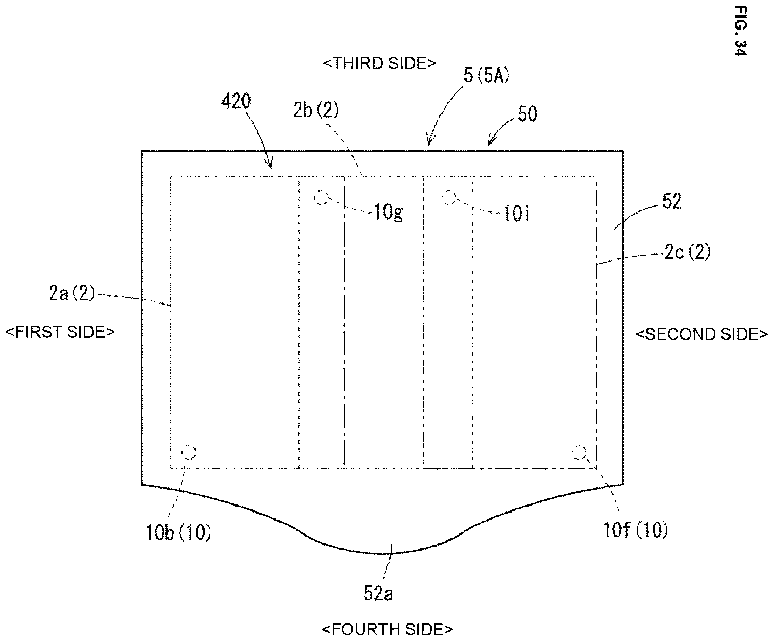

FIG. 34 is a schematic diagram illustrating an example of the holding member 5A and imaging plates 2a, 2b, and 2c held thereby, in a case where N=3. In the example of FIG. 34, the plurality of marks 10 provided on the holding member 5A includes marks 10b, 10f, 10g, and 10i. The first layout diagram 460 in the storage unit 280b, for example, indicates relative positions 450 of the marks 10b, 10f, and 10g on the holding member 5A. In a relationship between the imaging plate 2a and the imaging plate 2b, the mark 10i serves as the non-overlapping portion mark 10 of a 2-th IP, and in a relationship between the 2-th imaging plate 2b and the 3-th imaging plate 2c, the mark 10g serves as the non-overlapping portion mark 10 of the 2-th IP.

The image processing unit 281, for example, arranges the 2-th radiation image 320 and the 3-th radiation image 330 on the first layout diagram 460 such that positions of two mark images 350 respectively included in the 2-th radiation image 320 and the 3-th radiation image 330, which correspond to the same mark 10i, match each other, a position of a mark image 350 corresponding to the mark 10g included in the 2-th radiation image 320 matches the position 450 of the mark 10g indicated in the first layout diagram 460, and a position of a mark image 350 corresponding to the mark 10f included in the 3-th radiation image 330 matches the position 450 of the mark 10f indicated in the first layout diagram 460. At this time, the 2-th radiation image 320 is positioned above the 3-th radiation image 330.

Next, the image processing unit 281 arranges the 1-th radiation image 310 on the first layout diagram 460 such that a position of a mark image 350 corresponding to the mark 10g included in the 1-th radiation image 310 matches a position of a mark image 350 corresponding to the mark 10g included in the 2-th radiation image 320 on the first layout diagram 460, and a position of a mark image 350 corresponding to the mark 10b included in the 1-th radiation image 310 matches the position 450 of the mark 10b indicated in the first layout diagram 460. In other words, the image processing unit 281 arranges the 1-th radiation image 310 on the first layout diagram 460 such that the position of the mark image 350 corresponding to the mark 10g included in the 1-th radiation image 310 matches the position 450 of the mark 10g indicated in the first layout diagram 460, and the position of the mark image 350 corresponding to the mark 10b included in the 1-th radiation image 310 matches the position 450 of the mark 10b indicated in the first layout diagram 460. Thereby, basically, the 1-th radiation image 310, the 2-th radiation image 320, and the 3-th radiation image 330 are registered such that a 3-th IP overlapping portion corresponding image 322 of the 2-th radiation image 320 precisely overlaps above a 2-th IP overlapping portion corresponding image 331 of the 3-th radiation image 330, and a 2-th IP overlapping portion corresponding image 311 of the 1-th radiation image 310 precisely overlaps above a 1-th IP overlapping portion corresponding image 321 of the 2-th radiation image 320. Thereafter, the image processing unit 281 combines the 1-th radiation image 310, the 2-th radiation image 320, and the 3-th radiation image 330 to generate a composite image.

Note that the first layout diagram 460 may also indicate a position 450 of the mark 10i. In this case, the image processing unit 281, for example, arranges the 3-th radiation image 330 on the first layout diagram 460 such that positions of two mark images 350 respectively corresponding to the marks 10i and 10f included in the 3-th radiation image 330 match the positions 450 of the marks 10i and 10f indicated in the first layout diagram 460. Next, the image processing unit 281 arranges the 2-th radiation image 320 on the first layout diagram 460 such that positions of two mark images 350 respectively corresponding to the marks 10g and 10i included in the 2-th radiation image 320 match the positions 450 of the marks 10g and 10i indicated in the first layout diagram 460. Then, the image processing unit 281 arranges the 1-th radiation image 310 on the first layout diagram 460 such that positions of two mark images 350 respectively corresponding to the marks 10g and 10b included in the 1-th radiation image 310 match the positions 450 of the marks 10g and 10b indicated in the first layout diagram 460.

As described above, in this example, the image processing unit 281 registers the n-th radiation image and the (n+1)-th radiation image based on the position of the mark image 350 corresponding to the non-overlapping portion mark 10 of the n-th IP, the position of the mark image 350 corresponding to the non-overlapping portion mark 10 of the (n+1)-th IP, and the position of the mark image 350 corresponding to the overlapping portion mark 10 of the n-th IP and the (n+1)-th IP. In such a case, the image processing unit 281 can perform registration of the n-th radiation image and the (n+1)-th radiation image in the same manner as described above, by using a mark image 350 corresponding to at least one or more non-overlapping portion marks 10 of the n-th IP, a mark image 350 corresponding to at least one or more non-overlapping portion marks 10 of the (n+1)-th IP, and a mark image 350 corresponding to at least one or more overlapping portion marks 10 of the n-th IP and the (n+1)-th IP. Therefore, for example, in a case where N=2, the holding member 5 may be provided with two or more non-overlapping portion marks 10 of the 1-th IP. Further, the holding member 5 may be provided with two or more non-overlapping portion marks 10 of the second IP. Further, the holding member 5 may be provided with two or more marks 10 for the overlapping portion of the first IP and the second IP. Further, the number of the non-overlapping portion marks 10 of the 1-th IP and the number of the non-overlapping portion marks 10 of the 2-th IP may be different from each other.

Further, the positions of the non-overlapping portion mark 10 of the n-th IP, the non-overlapping portion mark 10 of the (n+1)-th IP, and the overlapping portion mark 10 of the n-th IP and the (n+1)-th IP used in the registration of the n-th radiation image and the (n+1)-th radiation image in this example are not limited to the above-described example.

### <Fourth Example (Use of Non-Overlapping Portion Mark)>

In this example, the image processing unit 281 registers an n-th radiation image and an (n+1)-th radiation image based on a position and an orientation of a mark image 350 corresponding to a non-overlapping portion mark 10 of an n-th IP and a position and an orientation of a mark image 350 corresponding to a non-overlapping portion mark 10 of an (n+1)-th IP.

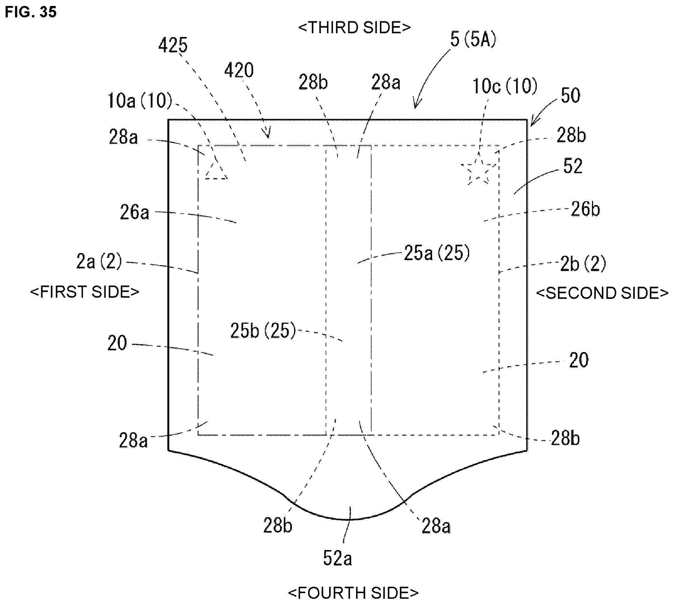

FIG. 35 is a schematic diagram illustrating an example of the holding member 5A. In the example of FIG. 35, the plurality of marks 10 provided on the holding member 5A includes marks 10a and 10c. In the example of FIG. 35, the mark 10a is an equilateral triangle, and the mark 10c is a star-shaped pentagon.

In the storage unit 280b of a control unit 280, a second layout diagram 490 is stored in advance, which indicates relative positions of a plurality of marks 10 on the holding member 5 and relative orientations of the plurality of marks 10 on the holding member 5. The image processing unit 281 registers the 1-th radiation image 310 and the 2-th radiation image 320 based on the second layout diagram 490 in the storage unit 280b and the mark images 350 corresponding to the marks 10a and 10c.

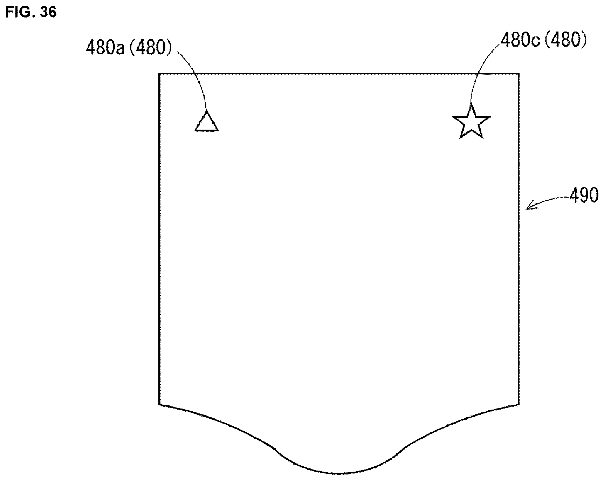

FIG. 36 is a schematic diagram illustrating an example of the second layout diagram 490. The second layout diagram 490 illustrates, for each mark 10, a mark correspondence diagram 480 that imitates the mark 10. A shape of the mark correspondence diagram 480 that imitates the mark 10 matches a shape of the mark 10 when the holding member 5A is viewed from a side on which radiation is irradiated, along a direction perpendicular to a reading surface 21 of the imaging plate 2. An outer shape of the second layout diagram 490, for example, matches an outer shape of the holding member 5A when the holding member 5A is viewed from the side on which radiation is irradiated, along the direction perpendicular to the reading surface 21 of the imaging plate 2. Note that the outer shape of the second layout diagram 490 is not limited to this.

Relative positions of a plurality of mark correspondence diagrams 480 in the second layout diagram 490 match relative positions of the plurality of marks 10 when the holding member 5A is viewed from the side on which radiation is irradiated, along the direction perpendicular to the reading surface 21 of the imaging plate 2. Further, relative orientations of the plurality of mark correspondence diagrams 480 in the second layout diagram 490 match relative orientations of the plurality of marks 10 when the holding member 5A is viewed from the side on which radiation is irradiated, along the direction perpendicular to the reading surface 21 of the imaging plate 2. The second layout diagram 490 illustrates a mark correspondence diagram 480a that imitates the mark 10a and a mark correspondence diagram 480c that imitates the mark 10c.

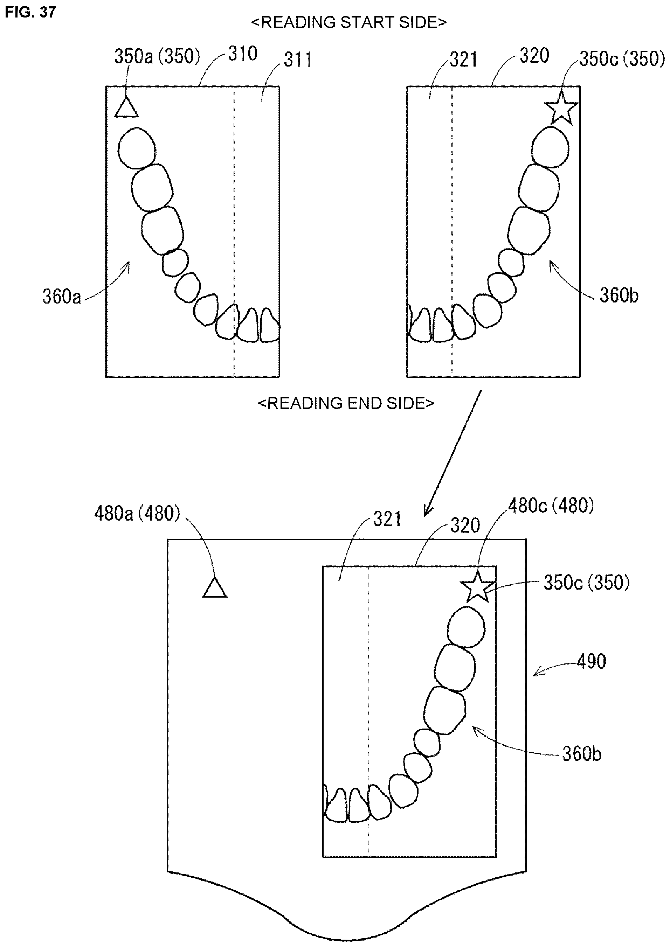

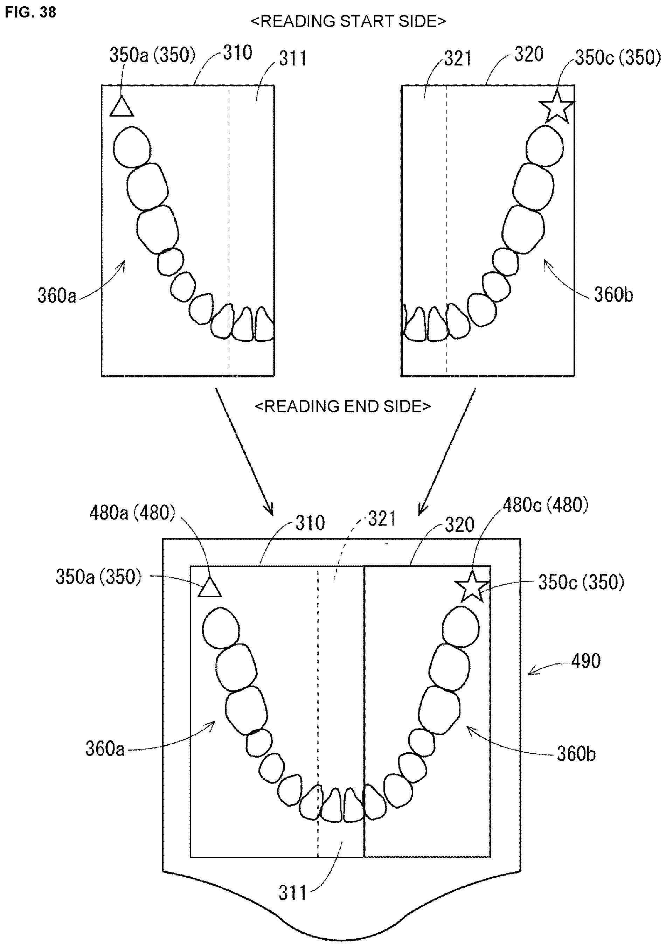

FIGS. 37 and 38 are schematic diagrams illustrating an example of a method for registering the 1-th radiation image 310 and the 2-th radiation image 320 in this example. The upper sides of FIGS. 37 and 38 illustrate an example of the 1-th radiation image 310 and the 2-th radiation image 320 read from the imaging plate 2a and the imaging plate 2b illustrated in FIG. 35. Hereinafter, a left side and a right side of a read radiation image such as the 1-th radiation image 310 respectively mean the left side and the right side of the read radiation image when the read radiation image is arranged such that a reading start side and a reading end side of the read radiation image are positioned on an upper side and a lower side, respectively, as illustrated in the upper sides of FIGS. 37 and 38.

The image processing unit 281, as illustrated in the lower side of FIG. 37, for example, arranges the 2-th radiation image 320 on the second layout diagram 490 such that a mark image 350c, which is included in the 2-th radiation image 320 and corresponds to the star-shaped pentagon mark 10c, precisely overlaps with a mark correspondence diagram 480c that imitates the mark 10c, which is indicated by the second layout diagram 490. In other words, the image processing unit 281 arranges the 2-th radiation image 320 on the second layout diagram 490 such that the position and the orientation of the mark image 350c respectively match the position and the orientation of the mark correspondence diagram 480c having the same shape as the mark image 350c.

Next, the image processing unit 281, as illustrated in the lower side of FIG. 38, arranges the 1-th radiation image 310 on the second layout diagram 490 such that a mark image 350a, which is included in the 1-th radiation image 310 and corresponds to the triangular mark 10a, precisely overlaps with a mark correspondence diagram 480a that imitates the mark 10a, which is indicated by the second layout diagram 490.

Here, a shape of the mark image 350a and the mark correspondence diagram 480a has three-fold rotational symmetry. Therefore, there are three possible orientations of the mark image 350a in which the mark image 350a precisely overlaps with the mark correspondence diagram 480a of the second layout diagram 490. Further, a shape of the mark image 350c and the mark correspondence diagram 480c has five-fold rotational symmetry. Therefore, there are five possible orientations of the mark image 350c in which the mark image 350c precisely overlaps with the mark correspondence diagram 480c of the second layout diagram 490. Thus, merely arranging the 1-th radiation image 310 and the 2-th radiation image 320 on the second layout diagram 490 such that the mark images 350a and 350c respectively precisely overlap with the mark correspondence diagrams 480a and 480c may result in the overlapping portion 311 of the 1-th radiation image 310 and the overlapping portion 321 of the 2-th radiation image 320 not precisely overlapping with each other.

On the other hand, this example assumes a case where a misalignment of the overlap between the imaging plate 2a and the imaging plate 2b on the holding member 5A is small. Therefore, even if the overlap of the imaging plate 2a and the imaging plate 2b is misaligned, in an IP assembly 1, an end portion 25a on the second side of the imaging plate 2a and an end portion 25b on the first side of the imaging plate 2b overlap, and long sides of the imaging plate 2a and the imaging plate 2b are substantially parallel.

Therefore, when arranging the 1-th radiation image 310 and the 2-th radiation image 320 on the second layout diagram 490, the image processing unit 281 precisely overlaps the mark images 350a and 350c with the mark correspondence diagrams 480a and 480c, respectively, such that the right side of the 1-th radiation image 310 is positioned toward the 2-th radiation image 320 side, the left side of the 2-th radiation image 320 is positioned toward the 1-th radiation image 310 side, and the long side of the 1-th radiation image 310 and the long side of the 2-th radiation image 320 are parallel or substantially parallel. At this time, for example, a pattern matching technique may be used to overlap the mark images 350a and 350c with the mark correspondence diagrams 480a and 480c, respectively. Thereby, basically, the 1-th radiation image 310 and the 2-th radiation image 320 are registered such that the overlapping portion 311 of the 1-th radiation image 310 precisely overlaps above the overlapping portion 321 of the 2-th radiation image 320.

Note that even if N≥3, the image processing unit 281 can, for each of two or more pairs of radiation images to be combined, perform registration of the two read radiation images constituting the pair of radiation images to be combined, in the same manner as the above-described registration of the 1-th radiation image and the 2-th radiation image.

As in this example, when the image processing unit 281 registers the n-th radiation image and the (n+1)-th radiation image based on the position and the orientation of the mark image 350 corresponding to the non-overlapping portion mark 10 of the n-th IP and the position and the orientation of the mark image 350 corresponding to the non-overlapping portion mark 10 of the (n+1)-th IP, the shape of the mark 10 may be any shape from which a rotation of the mark 10 can be identified. In other words, the shape of the mark 10 may be any shape from which the orientation of the mark 10 can be identified. In this example, a mark whose rotation cannot be identified, such as a perfect circle (in other words, a mark whose orientation cannot be identified), is not adopted as the registration mark 10.

The mark 10 may be rotationally symmetric, as in the example of FIG. 35 (excluding, however, n-fold rotational symmetry that holds for any value of n, such as a circle), or may be non-rotationally symmetric. The shape of the mark 10 as non-rotationally symmetric may be, for example, an alphabet letter "A" or "B", a numeral "2" or "3", or another shape. The shape of the mark 10 as non-rotationally symmetric can also be said to be a shape from which its rotation can be identified regardless of a rotation angle of the mark 10. For example, the mark 10 may be a partially cut-out circle, or a vertically and horizontally asymmetric figure (for example, a trapezoid or a smiley face mark).

Further, as in this example, when registration of the n-th radiation image and the (n+1)-th radiation image is performed based on the position and the orientation of the mark image 350 corresponding to the non-overlapping portion mark 10 of the n-th IP and the position and the orientation of the mark image 350 corresponding to the non-overlapping portion mark 10 of the (n+1)-th IP, the image processing unit 281 can perform registration of the n-th radiation image and the (n+1)-th radiation image in the same manner as described above, by using a mark image corresponding to at least one non-overlapping portion mark 10 of the n-th IP and a mark image corresponding to at least one non-overlapping portion mark 10 for the (n+1)-th IP. Therefore, for example, in a case where N=2, the holding member 5 may be provided with two or more non-overlapping portion marks 10 of the 1-th IP, or may be provided with two or more non-overlapping portion marks 10 of the 2-th IP. Further, the number of the non-overlapping portion marks 10 of the 1-th IP and the number of the non-overlapping portion marks 10 of the 2-th IP may be different from each other.

Further, the positions of the non-overlapping portion mark 10 of the n-th IP and the non-overlapping portion mark 10 of the (n+1)-th IP used in the registration of the n-th radiation image and the (n+1)-th radiation image are not limited to the above-described example.

### <Fifth Example (Use of Overlapping Portion Mark)>

In this example, the image processing unit 281 registers an n-th radiation image and an (n+1)-th radiation image based on a position and an orientation of a mark image 350 corresponding to an overlapping portion mark 10 of an n-th IP and an (n+1)-th IP.

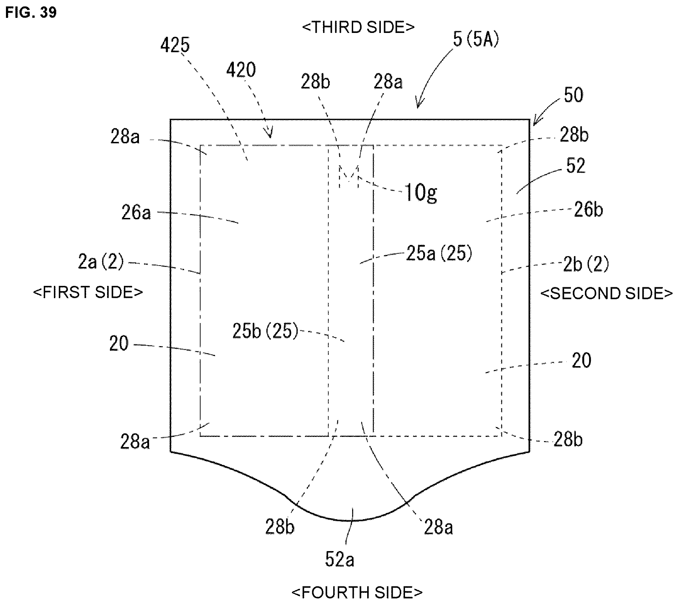

FIG. 39 is a schematic diagram illustrating an example of the holding member 5A. In the example of FIG. 39, the plurality of marks 10 provided on the holding member 5A includes a mark 10g as the overlapping portion mark 10 of a 1-th IP and a 2-th IP. In the example of FIG. 39, the mark 10g is an alphabet letter "M" and is non-rotationally symmetric.

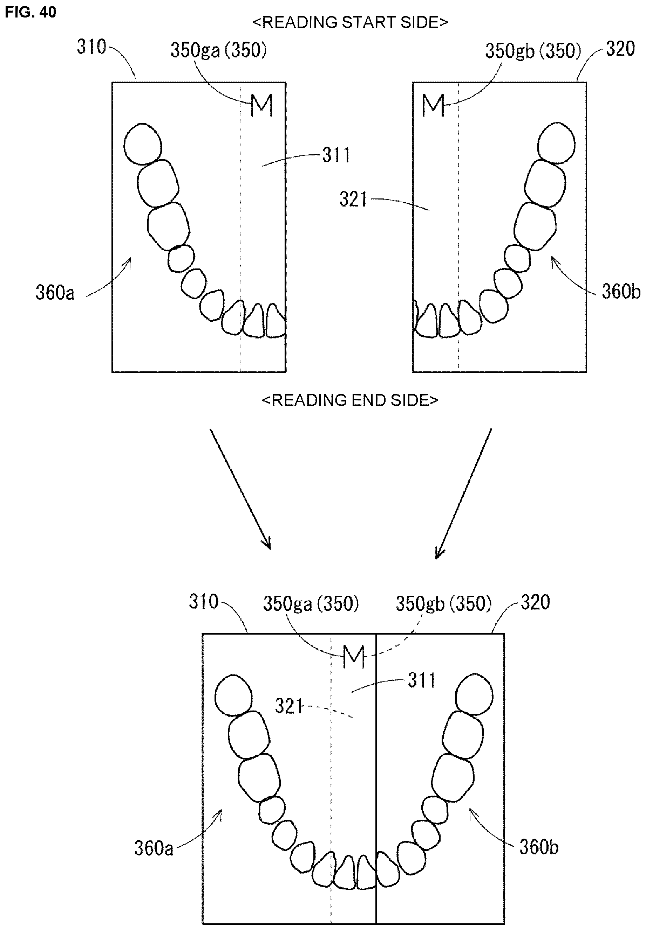

FIG. 40 is a schematic diagram for explaining an example of a method for registering the 1-th radiation image 310 and the 2-th radiation image 320 in this example. The upper side of FIG. 40 illustrates an example of the 1-th radiation image 310 and the 2-th radiation image 320 read from the imaging plate 2a and the imaging plate 2b illustrated in FIG. 39. The 1-th radiation image 310 includes a mark image 350ga corresponding to the mark 10g, and the 2-th radiation image 320 includes a mark image 350gb corresponding to the mark 10g.

In this example, as illustrated in the lower side of FIG. 40, the image processing unit 281 overlaps the 1-th radiation image 310 above the 2-th radiation image 320 such that a mark image 350ga and a mark image 350gb corresponding to the same mark 10g precisely overlap with each other. In other words, the image processing unit 281 overlaps the 1-th radiation image 310 above the 2-th radiation image 320 such that a position and an orientation of the mark image 350ga respectively match a position and an orientation of the mark image 350gb having the same shape as the mark image 350ga. Thereby, basically, the 1-th radiation image 310 and the 2-th radiation image 320 are registered such that the overlapping portion 311 of the 1-th radiation image 310 precisely overlaps above the overlapping portion 321 of the 2-th radiation image 320.

Note that in a case where the mark 10g is rotationally symmetric, merely overlapping the mark image 350ga and the mark image 350gb precisely may result in the overlapping portion 311 of the 1-th radiation image 310 and the overlapping portion 321 of the 2-th radiation image 320 not precisely overlapping with each other. In a case where the mark image 350g is rotationally symmetric, the image processing unit 281 precisely overlaps the mark image 350ga and the mark image 350gb with each other such that the right side of the 1-th radiation image 310 is positioned toward the 2-th radiation image 320 side, the left side of the 2-th radiation image 320 is positioned toward the 1-th radiation image 310 side, and the long side of the 1-th radiation image 310 and the long side of the 2-th radiation image 320 are parallel or substantially parallel. Thereby, basically, the overlapping portion 311 of the 1-th radiation image 310 comes to precisely overlap above the overlapping portion 321 of the 2-th radiation image 320.

Note that even if N≥3, the image processing unit 281 can, for each of two or more pairs of radiation images to be combined, perform registration of the two read radiation images constituting the pair of radiation images to be combined, in the same manner as the above-described registration of the 1-th radiation image and the 2-th radiation image.

As in this example, when the image processing unit 281 registers the n-th radiation image and the (n+1)-th radiation image based on the position and the orientation of the mark image 350 corresponding to the overlapping portion mark 10 of the n-th IP and the (n+1)-th IP, the shape of the mark 10, similarly to the fourth example, may be any shape from which a rotation of the mark 10 can be identified. In this example, a mark whose rotation cannot be identified, such as a perfect circle, is not adopted as the registration mark 10.

Further, as in this example, when registration of the n-th radiation image and the (n+1)-th radiation image is performed based on the position and the orientation of the mark image 350 corresponding to the overlapping portion mark 10 of the n-th IP and the (n+1)-th IP, the image processing unit 281 can perform registration of the n-th radiation image and the (n+1)-th radiation image in the same manner as described above by using a mark image corresponding to at least one overlapping portion mark 10 of the n-th IP and the (n+1)-th IP. Therefore, for example, in a case where N=2, the holding member 5 may be provided with two or more overlapping portion marks 10 of the 1-th IP and the 2-th IP.

Further, the position of the overlapping portion mark 10 of the n-th IP and the (n+1)-th IP used in the registration of the n-th radiation image and the (n+1)-th radiation image is not limited to the above-described example.

### <Sixth Example (Use of Boundary Image)>

As described above, an n-th imaging plate 2 and an (n+1)-th imaging plate 2 are held by the holding member 5A in a state of being partially overlapped with each other such that the (n+1)-th imaging plate 2 is positioned on a lower side. Therefore, an (n+1)-th radiation image includes a boundary image corresponding to a boundary between the n-th imaging plate 2 and the (n+1)-th imaging plate 2 on the (n+1)-th imaging plate 2. The boundary image included in the (n+1)-th radiation image can also be said to be an image of a portion of a peripheral edge (also referred to as an outer peripheral edge) of the n-th imaging plate 2 that is positioned on the (n+1)-th imaging plate 2.

In this example, the image processing unit 281 registers an n-th radiation image and an (n+1)-th radiation image based on a position of a mark image 350 corresponding to a non-overlapping portion mark 10 of an n-th IP, a position of a mark image 350 corresponding to a non-overlapping portion mark 10 of an (n+1)-th IP, and a boundary image included in the (n+1)-th radiation image. The image processing unit 281 can easily and appropriately perform registration of the n-th radiation image and the (n+1)-th radiation image, based on the mark images 350 included in the n-th radiation image and the (n+1)-th radiation image, and the boundary image included in the (n+1)-th radiation image.

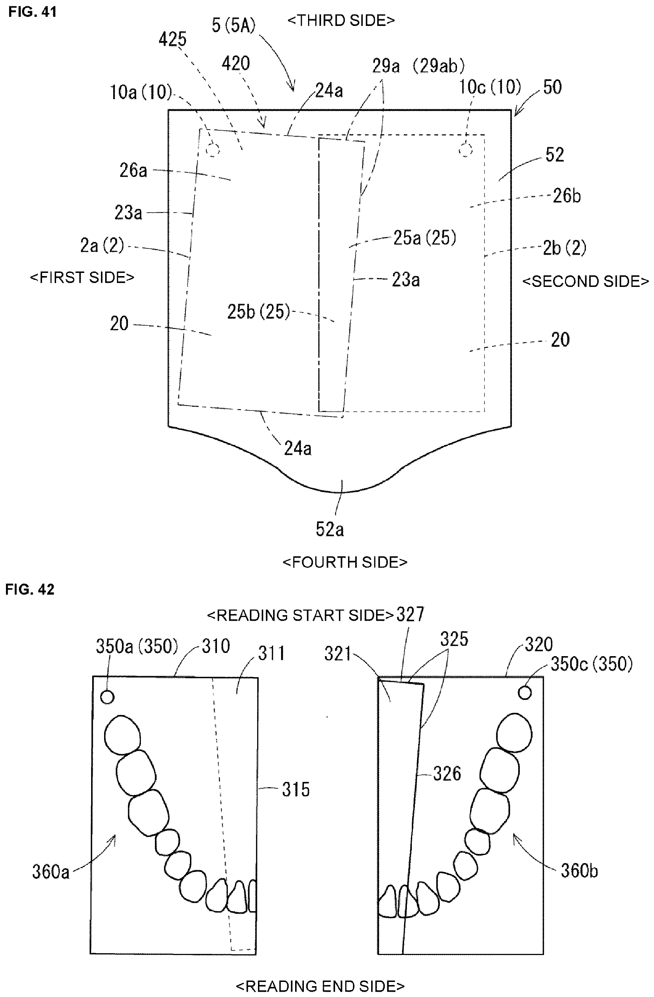

FIG. 41 is a schematic diagram illustrating an example of the holding member 5A and a 1-th imaging plate 2 and a 2-th imaging plate 2 held thereby. In the example of FIG. 41, the plurality of marks 10 provided on the holding member 5A includes a circular mark 10a as a non-overlapping portion mark 10 of a 1-th IP and a circular mark 10c as a non-overlapping portion mark 10 of a 2-th IP. In the storage unit 280b, the first layout diagram 460 indicating relative positions of the marks 10a and 10c on the holding member 5 is stored in advance.

Further, in the example of FIG. 41, as in the example of the above-described FIG. 23, the overlap of the imaging plate 2a and the imaging plate 2b is slightly misaligned in the holding member 5A. The peripheral edge of the imaging plate 2a includes a portion 29a positioned on the imaging plate 2b, in other words, a portion that overlaps with the imaging plate 2b. Hereinafter, the portion 29a may be referred to as an overlapping edge 29a. The overlapping edge 29a indicates a boundary 29ab between the 1-th imaging plate 2a and the 2-th imaging plate 2b on the 2-th imaging plate 2b.

As illustrated in FIG. 16 and the like, in a case where the overlap of the imaging plate 2a and the imaging plate 2b on the holding member 5A is not misaligned, only one long side 23a on the imaging plate 2b side of the imaging plate 2a (see FIG. 41) constitutes the overlapping edge 29a. Therefore, in a case where the overlap of the imaging plate 2a and the imaging plate 2b on the holding member 5A is not misaligned, the overlapping edge 29a does not include the other long side 23a of the imaging plate 2a and a short side 24a of the imaging plate 2a.

On the other hand, as in the example of FIG. 41, in a case where the overlap of the imaging plate 2a and the imaging plate 2b on the holding member 5A is misaligned, the overlapping edge 29a may include a portion of the peripheral edge of the imaging plate 2a other than the long side 23a on the imaging plate 2b side. As described above, in this example, even if the overlap of the imaging plate 2a and the imaging plate 2b is misaligned, the misalignment is small. Therefore, even in a case where the overlap of the imaging plate 2a and the imaging plate 2b is misaligned, a major part of the overlapping edge 29a is constituted by the one long side 23a on the imaging plate 2b side of the first imaging plate 2a, and the other long side 23a of the imaging plate 2a is not included in the overlapping edge 29a. Further, even if a short side 24a of the imaging plate 2a is included in the overlapping edge 29a, a proportion of the short side 24a included in the overlapping edge 29a is much smaller than a proportion of the long side 23a included in the overlapping edge 29a. Hereinafter, the long side 23a on the imaging plate 2b side of the imaging plate 2a may be referred to as a 2-th IP-side long side 23a. All or a major part of the overlapping edge 29a is constituted by the 2-th IP-side long side 23a. The 2-th IP-side long side 23a constitutes all or a major part of the boundary 29ab.

FIG. 42 is a schematic diagram illustrating an example of the 1-th radiation image 310 and the 2-th radiation image 320 read from the 1-th imaging plate 2a and the 2-th imaging plate 2b illustrated in FIG. 41.

As illustrated in FIG. 42, the 1-th radiation image 310 includes a mark image 350a corresponding to the mark 10a. The 2-th radiation image 320 includes a mark image 350c corresponding to the mark 10c. Further, the 2-th radiation image 320 includes a boundary image 325 corresponding to the boundary 29ab between the 1-th imaging plate 2a and the 2-th imaging plate 2b on the 2-th imaging plate 2b. The boundary image 325 can also be said to be an image of the overlapping edge 29a of the 1-th imaging plate 2. The boundary image 325 indicates a contour of an overlapping portion 321 (in other words, a 1-th IP overlapping portion corresponding image 321). In the 2-th radiation image 320, basically, a dental arch image 360b and the mark image 350c appear more clearly than the boundary image 325. Note that in figures where an explanation of the boundary image 325 is unnecessary, such as the above-described figures illustrating an example of the 2-th radiation image 320, illustration of the boundary image 325 is omitted.

The boundary image 325 includes an image 326 (also referred to as a long-side corresponding image 326) corresponding to the 2-th IP-side long side 23a of the imaging plate 2a. The long-side corresponding image 326 is an image of at least a part of the 2-th IP-side long side 23a. In a case where the overlap of the imaging plate 2a and the imaging plate 2b on the holding member 5A is not misaligned, the long-side corresponding image 326 becomes an image of the entire 2-th IP-side long side 23a, and the boundary image 325 is constituted only by the long-side corresponding image 326. On the other hand, in a case where the overlap of the imaging plate 2a and the imaging plate 2b on the holding member 5A is slightly misaligned as in the example of FIG. 41, the long-side corresponding image 326 becomes an image of a part of the 2-th IP-side long side 23a, and a major part of the boundary image 325 is constituted by the long-side corresponding image 326. In the example of FIG. 41, the boundary image 325 also includes an image 327 (also referred to as a short-side corresponding image 327) corresponding to the short side 24a of the imaging plate 2a. A proportion of the short-side corresponding image 327 included in the boundary image 325 is much smaller than a proportion of the long-side corresponding image 326 included in the boundary image 325.

The image processing unit 281 registers the 1-th radiation image 310 and the 2-th radiation image 320 based on the first layout diagram 460, the mark image 350a included in the 1-th radiation image 310, and the mark image 350c and the boundary image 325 included in the 2-th radiation image 320.

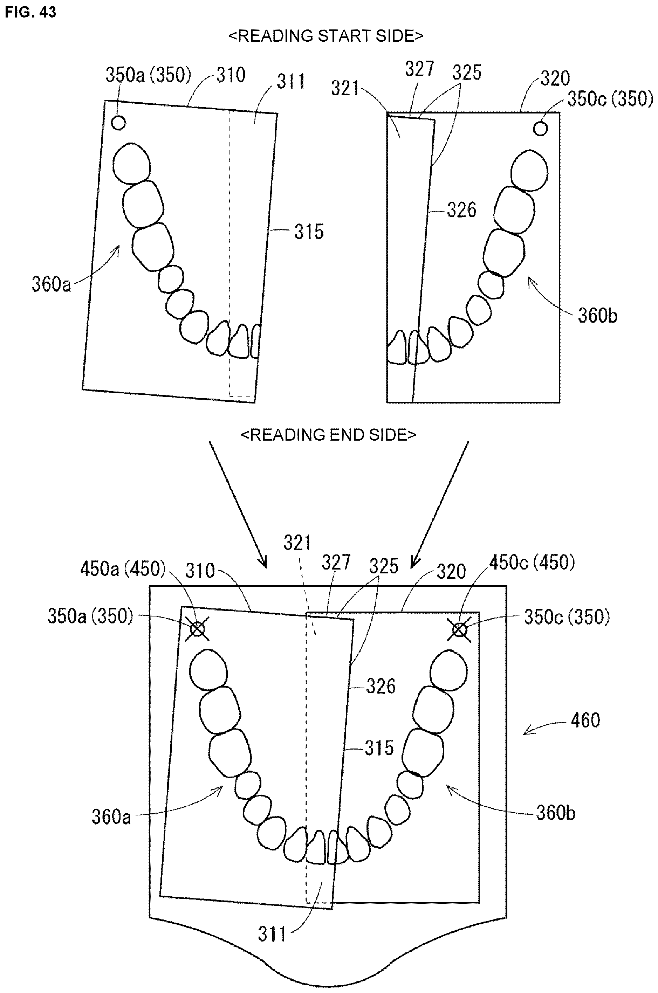

FIG. 43 is a schematic diagram for explaining an example of a method for registering the 1-th radiation image 310 and the 2-th radiation image 320 in this example. First, the image processing unit 281, for example, places a reading start side of the 1-th radiation image 310 and the 2-th radiation image 320 on an upper side, places the 1-th radiation image 310 and the 2-th radiation image 320 on a left side and a right side, respectively, and arranges the 1-th radiation image 310 and the 2-th radiation image 320 side by side. Next, the image processing unit 281, as illustrated in the upper side of FIG. 43, makes a long side 315 on a 2-th radiation image 320 side in the 1-th radiation image 310 (also referred to as a 2-th radiation image-side long side 315) and the long-side corresponding image 326 included in the boundary image 325 of the 2-th radiation image 320 parallel. Each of the 2-th radiation image-side long side 315 of the 1-th radiation image 310 and the long-side corresponding image 326 included in the boundary image 325 corresponds to the 2-th IP-side long side 23a of the imaging plate 2a.

In a case where there is no misalignment in the overlap of the imaging plate 2a and the imaging plate 2b on the holding member 5A, the boundary image 325 is constituted only by the long-side corresponding image 326. Then, on a left end portion of the 2-th radiation image 320, a straight line image appears as the long-side corresponding image 326 (in other words, the boundary image 325), extending from one end on the reading start side to one end on the reading end side of the 2-th radiation image 320. In a case where the left end portion of the 2-th radiation image 320 includes a straight line image extending from one end on the reading start side to one end on the reading end side of the 2-th radiation image 320, the image processing unit 281 defines the straight line image as the long-side corresponding image 326 (that is, the boundary image 325) and arranges the 1-th radiation image 310 and the 2-th radiation image 320 side by side such that the long-side corresponding image 326 and the 2-th radiation image-side long side 315 are parallel.

As described above, in this example, even if a misalignment occurs in the overlap of the imaging plate 2a and the imaging plate 2b on the holding member 5A, the misalignment is small. Therefore, even in a case where a misalignment occurs in the overlap of the imaging plate 2a and the imaging plate 2b on the holding member 5A, a long straight line image appears on the left end portion of the 2-th radiation image 320 as the long-side corresponding image 326 of the boundary image 325. This long straight line image substantially extends from one end on the reading start side to one end on the reading end side of the 2-th radiation image 320. In a case where the left end portion of the 2-th radiation image 320 includes a straight line image substantially extending from one end on the reading start side to one end on the reading end side of the 2-th radiation image 320, the image processing unit 281 defines the straight line image as the long-side corresponding image 326 and arranges the 1-th radiation image 310 and the 2-th radiation image 320 side by side such that the long-side corresponding image 326 and the second radiation image-side long side 315 are parallel.

Next, the image processing unit 281, as illustrated in the lower side of FIG. 43, while maintaining a parallel state between the 2-th radiation image-side long side 315 and the long-side corresponding image 326, arranges the 2-th radiation image 320 and the 1-th radiation image 310 on the first layout diagram 460 such that a position of the mark image 350a corresponding to the mark 10a, which is included in the 1-th radiation image 310, matches the position 450a of the mark 10a in the first layout diagram 460, and a position of the mark image 350c corresponding to the mark 10c, which is included in the 2-th radiation image 320, matches the position 450c of the mark 10c in the first layout diagram 460. At this time, the image processing unit 281 positions the 2-th radiation image 320 below the 1-th radiation image 310. As a result, basically, the 1-th radiation image 310 and the 2-th radiation image 320 are registered such that the overlapping portion 311 of the 1-th radiation image 310 precisely overlaps with the overlapping portion 321 of the 2-th radiation image 320.

The registration processing of the 1-th radiation image 310 and the 2-th radiation image 320 as described above can also be said to be a process of making an edge of the 1-th radiation image 310 and the edge imaged in the 2-th radiation image 320 parallel and overlapping them.

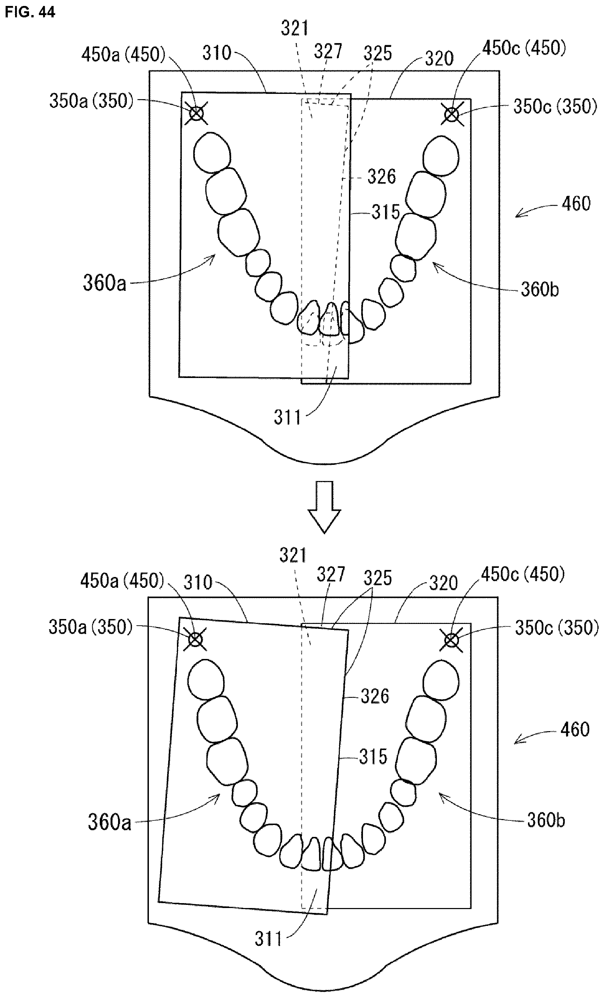

Note that the image processing unit 281 may change an orientation of at least one of the 1-th radiation image 310 and the 2-th radiation image 320 such that the 2-th radiation image-side long side 315 of the 1-th radiation image 310 overlaps with the long-side corresponding image 326 of the 2-th radiation image 320, after arranging the 1-th radiation image 310 and the 2-th radiation image 320 on the first layout diagram 460. FIG. 44 is a schematic diagram for explaining an example of a method for registering the 1-th radiation image 310 and the 2-th radiation image 320 in this case.

In the example of FIG. 44, the image processing unit 281 first arranges the 2-th radiation image 320 on the first layout diagram 460 such that a position of a mark image 350c, which is included in the 2-th radiation image 320 and corresponds to the mark 10c, matches the position 450c of the mark 10c in the first layout diagram 460. Next, the image processing unit 281 arranges the 1-th radiation image 310 on the first layout diagram 460 such that a position of a mark image 350a, which is included in the 1-th radiation image 310 and corresponds to the mark 10a, matches the position 450a of the mark 10a in the first layout diagram 460. Thereby, as illustrated in the upper side of FIG. 44, the 1-th radiation image 310 and the 2-th radiation image 320 are arranged on the first layout diagram 460 such that the position of the mark image 350a of the 1-th radiation image 310 matches the position 450a in the first layout diagram 460, and the position of the mark image 350c of the 2-th radiation image 320 matches the position 450c in the first layout diagram 460.

Next, the image processing unit 281, as illustrated in the lower side of FIG. 44, changes an orientation of at least one of the 1-th radiation image 310 and the 2-th radiation image 320 such that the 2-th radiation image-side long side 315 of the 1-th radiation image 310 and the long-side corresponding image 326 of the 2-th radiation image 320 overlap with each other, while maintaining a state where the positions of the mark images 350a and 350c respectively match the positions 450a and 450c in the first layout diagram 460. Thereby, similarly to the example of FIG. 43, basically, the 1-th radiation image 310 and the 2-th radiation image 320 are registered such that the overlapping portion 311 of the 1-th radiation image 310 precisely overlaps above the overlapping portion 321 of the 2-th radiation image 320.

As can be understood from the above description, in both the example of FIG. 43 and the example of FIG. 44, the image processing unit 281 registers the 1-th radiation image 310 and the 2-th radiation image 320 by arranging the 1-th radiation image 310 and the 2-th radiation image 320 on the first layout diagram 460 such that a position of the mark image 350a included in the 1-th radiation image 310 matches a position 450a, a position of the mark image 350c included in the 2-th radiation image 320 matches a position 450c, and one long side of the 1-th radiation image 310 and the long-side corresponding image 326 of the 2-th radiation image 320 overlap with each other.

The above-described example was a case where N=2, but even in a case where N≥3 and the holding member 5A holds three or more imaging plates 2, the image processing unit 281 can, for each of two or more pairs of radiation images to be combined, perform registration of the two read radiation images constituting the pair of radiation images to be combined, in the same manner as the above-described registration of the 1-th radiation image and the 2-th radiation image.

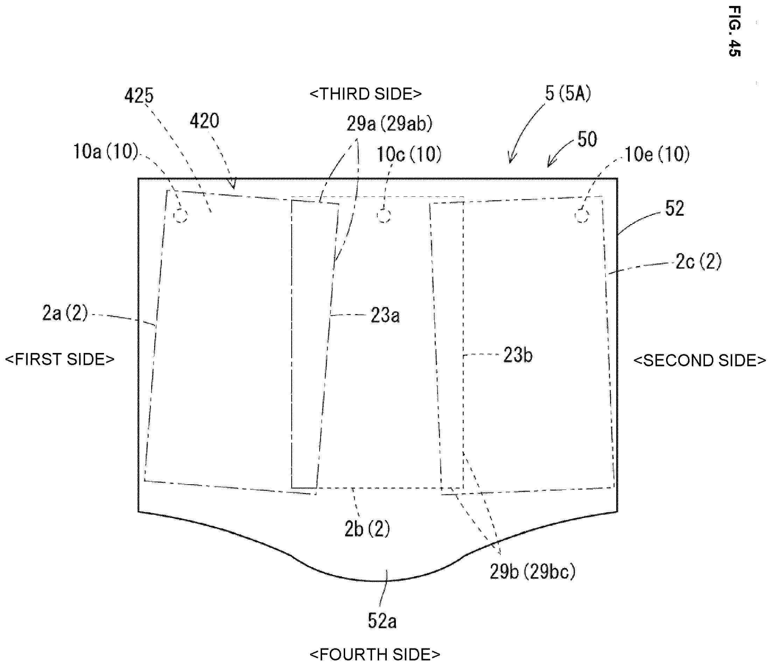

FIG. 45 is a schematic diagram illustrating an example of the holding member 5A and a 1-th imaging plate 2a, a 2-th imaging plate 2b, and a 3-th imaging plate 2c held thereby, in a case where N=3.

In the example of FIG. 45, the holding member 5A is provided not only with the marks 10a and 10c, but also with a mark 10e as a non-overlapping portion mark 10 of a 3-th IP. The first layout diagram 460 in the storage unit 280b indicates relative positions 450 of the marks 10a, 10c, and 10e on the holding member 5A.

In the example of FIG. 45, an overlap of the imaging plates 2b and 2c in a holding member 5A is slightly shifted. The periphery of the imaging plate 2b includes a portion 29b located on the imaging plate 2c, in other words, a portion 29b that overlaps with the imaging plate 2c. Hereinafter, the portion 29b may be referred to as an overlapping edge 29b. The overlapping edge 29b indicates a boundary 29bc between a 2-th imaging plate 2b and a 3-th imaging plate 2c on the 3-th imaging plate 2c. The overlapping edge 29b includes at least a part of a long side 23b of the imaging plate 2b on the imaging plate 2c side (also referred to as a 3-th IP-side long side 23b).

A 3-th radiation image 330 read from the 3-th imaging plate 2c includes a mark image 350 corresponding to a mark 10e. Furthermore, the 3-th radiation image 330 includes a boundary image (also referred to as a 2-th boundary image) corresponding to the boundary 29bc between the 2-th imaging plate 2b and the 3-th imaging plate 2c on the 3-th imaging plate 2c. The second boundary image includes an image corresponding to the 3-th IP-side long side 23b of the imaging plate 2b (also referred to as a second long side corresponding image). The second long side corresponding image is an image of at least a part of the 3-th IP-side long side 23b.

An image processing unit 281, for example, arranges a 2-th radiation image 320 and a 3-th radiation image 330 on a first layout diagram 460 such that a position of a mark image 350c corresponding to a mark 10c included in the 2-th radiation image 320 matches a position 450c of the mark 10c in the first layout diagram 460, a position of a mark image 350 corresponding to a mark 10e included in the 3-th radiation image 330 matches a position 450 of the mark 10e in the first layout diagram 460, and one long side of the 2-th radiation image 320 and the second long side corresponding image included in the second boundary image of the 3-th radiation image 330 overlap with each other. At this time, the image processing unit 281 positions the 3-th radiation image 330 below the 2-th radiation image 320. Thereby, the registration of the 2-th radiation image 320 and the 3-th radiation image 330, which constitute one pair of radiation images to be combined, is completed. The image processing unit 281 can identify the second long side corresponding image in the same manner as the method for identifying the long side corresponding image 326.

Next, the image processing unit 281 arranges a 1-th radiation image 310 over the 2-th radiation image 320 on the first layout diagram 460 such that a position of a mark image 350a included in the 1-th radiation image 310 matches a position 450a in the first layout diagram 460, a position of a mark image 350c included in the 2-th radiation image 320 matches the position 450c in the first layout diagram 460, and one long side of the 1-th radiation image 310 and a long side corresponding image 326 of the 2-th radiation image 320 overlap with each other. Thereby, the registration of the 1-th radiation image 310 and the 2-th radiation image 320, which constitute one pair of radiation images to be combined, is completed. As a result, basically, the 1-th radiation image 310, the 2-th radiation image 320, and the 3-th radiation image 330 are registered such that a 3-th IP overlapping portion corresponding image 322 of the 2-th radiation image 320 exactly overlaps a 2-th IP overlapping portion corresponding image 331 of the 3-th radiation image 330, and a 2-th IP overlapping portion corresponding image 311 of the 1-th radiation image 310 exactly overlaps a 1-th IP overlapping portion corresponding image 321 of the 2-th radiation image 320.

As in this example, when registration of an n-th radiation image and an (n+1)-th radiation image is performed based on a boundary image corresponding to a boundary between an n-th imaging plate 2 and an (n+1)-th imaging plate 2 on the (n+1)-th imaging plate 2 included in the (n+1)-th radiation image, a position of a mark image corresponding to a non-overlapping portion mark 10 of the n-th IP, and a position of a mark image corresponding to a non-overlapping portion mark 10 of the (n+1)-th IP, the image processing unit 281 can perform the registration of the n-th radiation image and the (n+1)-th radiation image in the same manner as described above, using a mark image corresponding to at least one non-overlapping portion mark 10 of the n-th IP and a mark image corresponding to at least one non-overlapping portion mark 10 of the (n+1)-th IP. Therefore, for example, when N=2, the holding member 5 may include two or more non-overlapping portion marks 10 of a 1-th IP, or may include two or more non-overlapping portion marks 10 of a 2-th IP. Further, the number of non-overlapping portion marks 10 of the 1-th IP and the number of non-overlapping portion marks 10 of the 2-th IP may be different from each other.

Further, the positions of the non-overlapping portion mark 10 of the n-th IP and the non-overlapping portion mark 10 of the (n+1)-th IP used in the registration of the n-th radiation image and the (n+1)-th radiation image are not limited to the above example.

### <Seventh Example (Use of Boundary Image)>

In this example, the image processing unit 281 performs registration of an n-th radiation image and an (n+1)-th radiation image based on a boundary image included in the (n+1)-th radiation image, which corresponds to a boundary between an n-th imaging plate 2 and an (n+1)-th imaging plate 2 on the (n+1)-th imaging plate 2, and a mark image 350 corresponding to an overlapping portion mark 10 for an n-th IP and an (n+1)-th IP.

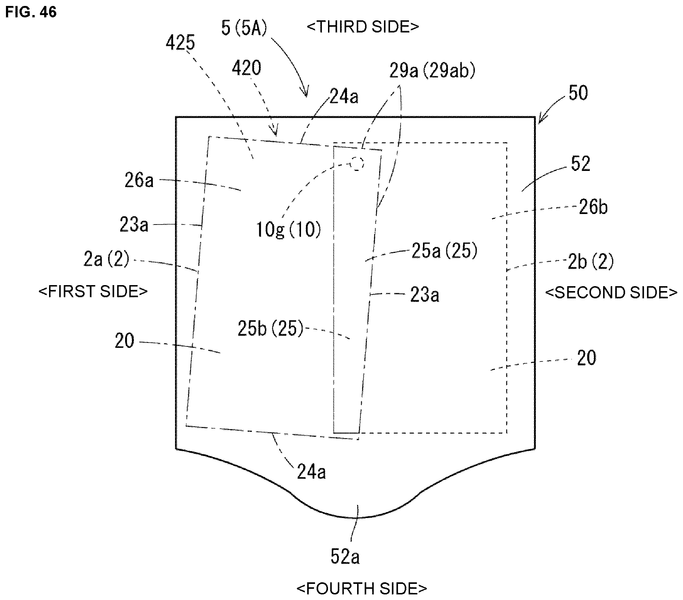

FIG. 46 is a schematic diagram illustrating an example of a holding member 5A. In the example of FIG. 46, the holding member 5A is provided with a mark 10g as an overlapping portion mark 10 for a 1-th IP and a 2-th IP. A 1-th radiation image 310 read from an imaging plate 2a includes a mark image 350ga corresponding to the mark 10g. A 2-th radiation image 320 read from an imaging plate 2b includes a mark image 350gb corresponding to the mark 10g and the aforementioned boundary image 325.

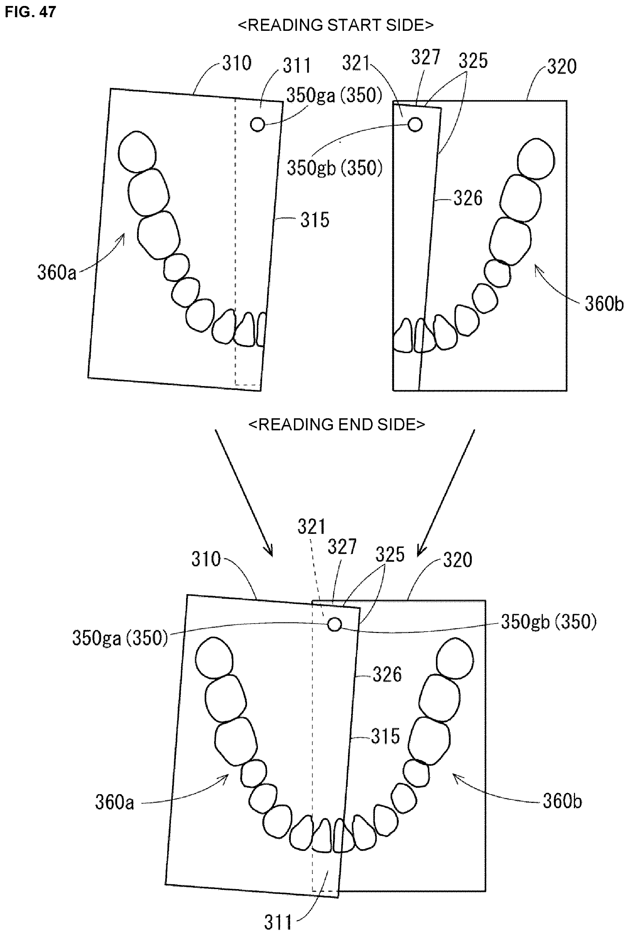

FIG. 47 is a schematic diagram for explaining an example of a method for registration of the 1-th radiation image 310 and the 2-th radiation image 320 in this example. First, the image processing unit 281, for example, similarly to the sixth example described above, places a reading start side of the 1-th radiation image 310 and the 2-th radiation image 320 on an upper side, and arranges them side by side such that the 1-th radiation image 310 and the 2-th radiation image 320 are positioned on a left side and a right side, respectively. Next, the image processing unit 281, as shown in the upper part of FIG. 47, makes a 2-th radiation image-side long side 315 of the 1-th radiation image 310 and a long side corresponding image 326 included in the boundary image 325 of the 2-th radiation image 320 parallel.

Next, as shown in the lower part of FIG. 47, the image processing unit 281 partially overlaps the 1-th radiation image 310 and the 2-th radiation image 320 such that the positions of a mark image 350ga and a mark image 350gb corresponding to the same mark 10g match each other, while maintaining a parallel state between the 2-th radiation image-side long side 315 and the long side corresponding image 326. At this time, the image processing unit 281, for example, positions the 1-th radiation image 310 above the 2-th radiation image 320. Thereby, basically, the 1-th radiation image 310 and the 2-th radiation image 320 are registered such that an overlapping portion 311 of the 1-th radiation image 310 exactly overlaps an overlapping portion 321 of the 2-th radiation image 320.

Note that after partially overlapping the 1-th radiation image 310 and the 2-th radiation image 320 such that the positions of the mark image 350ga and the mark image 350gb match each other and the 2-th radiation image 320 is positioned below the 1-th radiation image 310, the image processing unit 281 may change the orientation of at least one of the 1-th radiation image 310 and the 2-th radiation image 320 so that the 2-th radiation image-side long side 315 of the 1-th radiation image 310 overlaps the long side corresponding image 326 of the 2-th radiation image 320.

As can be understood from the above description, in this example, the image processing unit 281 performs registration of the 1-th radiation image 310 and the 2-th radiation image 320 such that the positions of the mark image 350ga and the mark image 350gb match each other, and one long side of the 1-th radiation image 310 overlaps the long side corresponding image 326 of the 2-th radiation image 320.

Even when N ≥ 3, the image processing unit 281 can perform registration of two read radiation images constituting each of two or more pairs of radiation images to be combined, in the same manner as the above-described registration of the 1-th radiation image and the 2-th radiation image.

As in this example, when registration of an n-th radiation image and an (n+1)-th radiation image is performed based on a boundary image included in the (n+1)-th radiation image corresponding to a boundary between an n-th imaging plate 2 and an (n+1)-th imaging plate 2 on the (n+1)-th imaging plate 2, and a mark image 350 corresponding to an overlapping portion mark 10 of an n-th IP and an (n+1)-th IP, the image processing unit 281 can perform the registration of the n-th radiation image and the (n+1)-th radiation image in the same manner as described above, using at least one overlapping portion mark 10 of the n-th IP and the (n+1)-th IP. Therefore, for example, when N=2, the holding member 5 may include two or more overlapping portion marks 10 of a first IP and a second IP.

Further, the positions of the non-overlapping portion marks 10 of the n-th IP and the (n+1)-th IP used in the registration of the n-th radiation image and the (n+1)-th radiation image in this example are not limited to the above example.

### <Eighth Example (Identification of Reading Direction)>

When the radiography is finished, as described above, an imaging plate 2 is basically inserted into a reading device 100 from a posterior short side in the oral cavity, and an optical measuring instrument 290 reads a radiation image from the posterior short side in the oral cavity of the imaging plate 2 (in other words, a third side).

However, the imaging plate 2 may be accidentally inserted into the reading device 100 from an anterior short side in the oral cavity by a user. In this case, the optical measuring instrument 290 will read the radiation image from the anterior short side in the oral cavity of the imaging plate 2 (in other words, a fourth side).

Thus, the reading direction of the radiation image from the imaging plate 2 is opposite between a case where the imaging plate 2 is inserted into the reading device 100 from the posterior short side in the oral cavity and a case where the imaging plate 2 is inserted into the reading device 100 from the anterior short side in the oral cavity. This is because the reading device 100 always reads the radiation image from a constant direction without switching a predetermined reading direction when reading the radiation image.

Hereinafter, for convenience of explanation, a reading direction when a radiation image is read from the posterior short side in the oral cavity of the imaging plate 2 may be referred to as a correct reading direction. Further, a reading direction when a radiation image is read from the anterior short side in the oral cavity of the imaging plate 2 may be referred to as an incorrect reading direction. Further, hereinafter, an image reading direction means a reading direction when a radiation image is read from the imaging plate 2.

In the first example described above, an n-th radiation image and an (n+1)-th radiation image are registered on the premise that the image reading direction of an n-th imaging plate 2 and an (n+1)-th imaging plate 2 is the correct reading direction. Therefore, when the image reading direction of at least one of the n-th imaging plate 2 and the (n+1)-th imaging plate 2 is the incorrect reading direction, the image processing unit 281 cannot appropriately combine the n-th radiation image and the (n+1)-th radiation image, and there is a possibility that a composite image 400 in which a subject is appropriately captured cannot be generated.

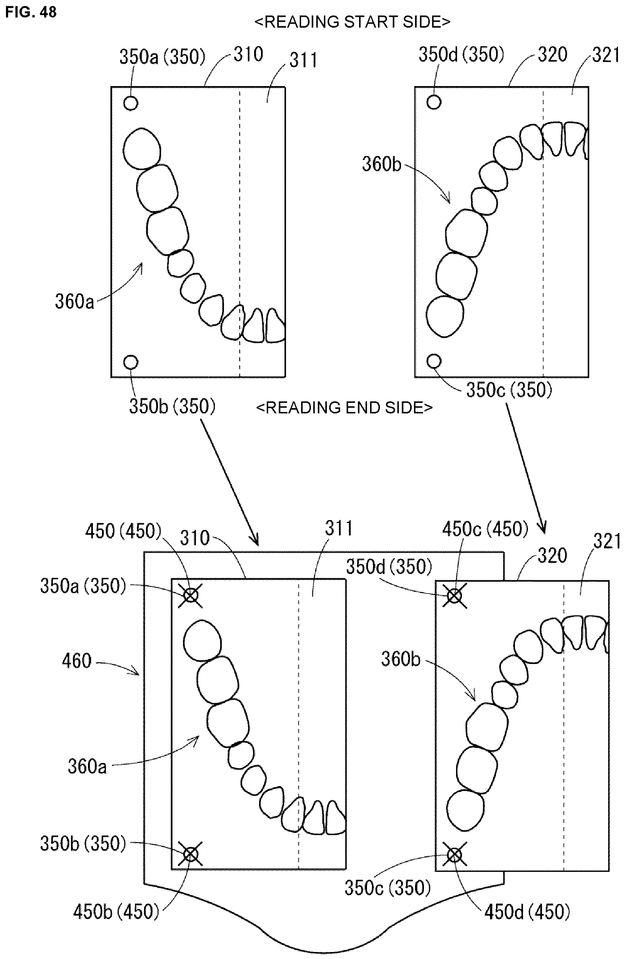

For example, a case where N=2, the image reading direction of a 1-th imaging plate 2 is the correct reading direction, and the image reading direction of a 2-th imaging plate 2 is the incorrect reading direction is considered. In this case, as shown in the upper part of FIG. 48, in a 1-th radiation image 310, a posterior side becomes a reading start side, and in a 2-th radiation image 320, an anterior side becomes a reading start side. Therefore, when the 1-th radiation image 310 and the 2-th radiation image 320 are arranged on the first layout diagram 460 as in the first example, as shown in the lower part of FIG. 48, an overlapping portion 311 of the 1-th radiation image 310 and an overlapping portion 321 of the 2-th radiation image 320 do not overlap. Therefore, an image processing unit 281 cannot appropriately combine the 1-th radiation image 310 and the 2-th radiation image 320. As a result, the image processing unit 281 cannot generate a composite image 400 in which a subject such as a dental arch is appropriately captured. Even when the image reading direction of the 1-th imaging plate 2 is the incorrect reading direction and the image reading direction of the 2-th imaging plate 2 is the correct reading direction, the overlapping portion 311 of the 1-th radiation image 310 and the overlapping portion 321 of the 2-th radiation image 320 do not overlap on the first layout diagram 460. Further, even when the image reading directions of the 1-th imaging plate 2 and the 2-th imaging plate 2 are both the incorrect reading direction, the overlapping portion 311 of the 1-th radiation image 310 and the overlapping portion 321 of the 2-th radiation image 320 do not overlap on the first layout diagram 460.

Therefore, in this example, a plurality of marks 10 provided on the holding member 5 include at least one reading direction identification mark (also referred to as a first identification mark) for identifying, based on the mark image, the image reading direction of each of N imaging plates 2 held by the holding member 5. The image processing unit 281 performs a direction identification process of identifying the image reading direction of the N imaging plates 2 based on a mark image 350 corresponding to the at least one reading direction identification mark included in N read radiation images. Thereby, the image processing unit 281 can easily and accurately identify the reading direction of the radiation image from the imaging plate 2. Then, the image processing unit 281 can perform registration of the N radiation images easily and appropriately by performing registration of the N read radiation images based on a result of the direction identification process. As a result, a possibility of obtaining a composite image in which a subject is appropriately captured increases.

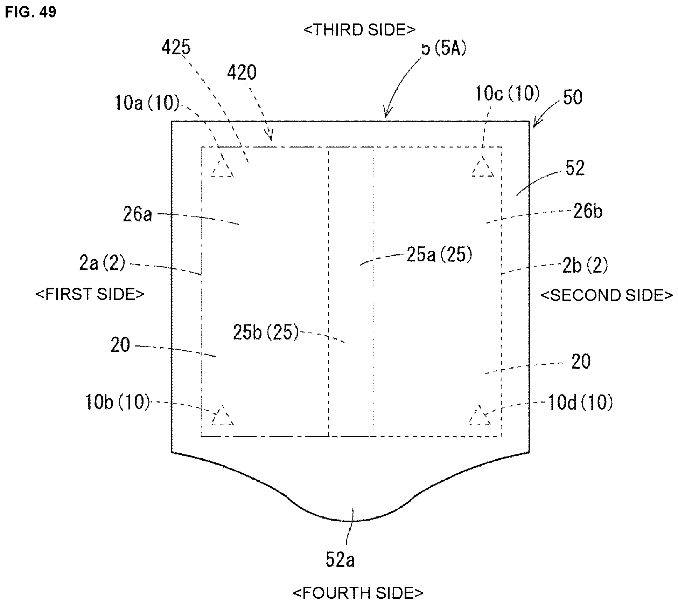

FIG. 49 is a schematic diagram illustrating an example of a holding member 5A. The example of FIG. 49 is a modification of the example of FIG. 16 described above, in which the shapes of marks 10a, 10b, 10c, and 10d are changed from circles to equilateral triangles.

In the holding member 5A, the marks 10a, 10b, 10c, and 10d are arranged in the same orientation (in other words, in the same posture). For example, the marks 10a, 10b, 10c, and 10d are arranged such that one vertex faces a third side. Therefore, when the holding member 5A is inserted into the oral cavity from the third side, one vertex of each of the marks 10a, 10b, 10c, and 10d faces a posterior side of the oral cavity. The marks 10a, 10b, 10c, and 10d are used as reading direction identification marks for identifying the image reading direction of imaging plates 2a and 2b based on a mark image 350.

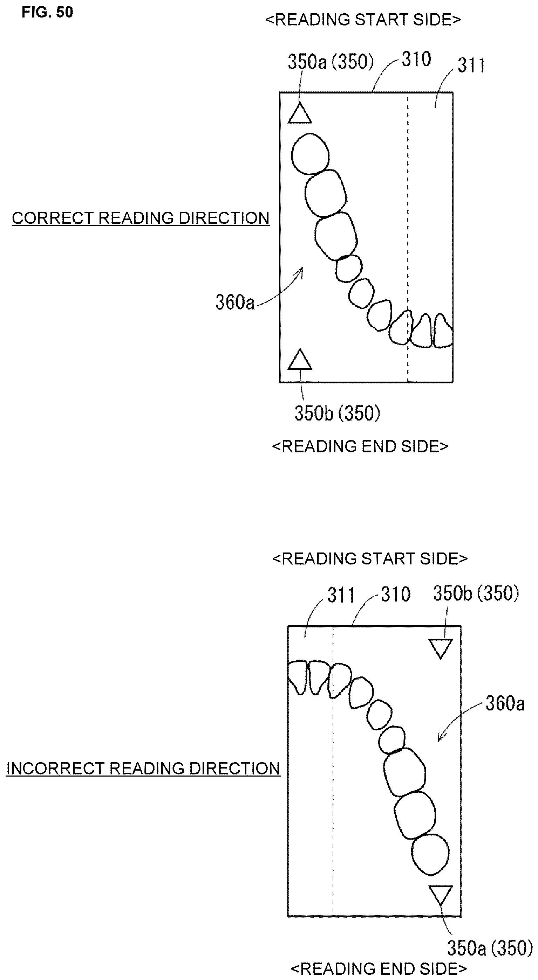

FIG. 50 is a schematic diagram illustrating an example of a 1-th radiation image 310 read from the imaging plate 2a shown in FIG. 49. The upper part of FIG. 50 shows an example of the 1-th radiation image 310 when the image reading direction of the imaging plate 2a is the correct reading direction. The lower part of FIG. 50 shows an example of the 1-th radiation image 310 when the image reading direction of the imaging plate 2a is the incorrect reading direction.

The marks 10a and 10b are configured such that the image reading direction of the imaging plate 2a is identified from the orientation of mark images 350a and 350b corresponding thereto. When the image reading direction of the imaging plate 2a is the correct reading direction, as shown in the upper part of FIG. 50, one vertex of each of equilateral triangular mark images 350a and 350b corresponding to the marks 10a and 10b faces a reading start side. On the other hand, when the image reading direction of the imaging plate 2a is the incorrect reading direction, as shown in the lower part of FIG. 50, one vertex of the mark images 350a and 350b faces a reading end side. As in the example of FIG. 50, when arranging the 1-th radiation image 310 with the reading start side facing upward, the orientation of the mark images 350a and 350b included in the 1-th radiation image 310 when the image reading direction of the imaging plate 2a is the incorrect reading direction is obtained by rotating the orientation of the mark images 350a and 350b included in the 1-th radiation image 310 by 180 degrees when the image reading direction of the imaging plate 2a is the correct reading direction. Note that in this example, since it is assumed that a shift in the overlap of the imaging plates 2a and 2b in the holding member 5A is small, the same can be said even if the overlap of the imaging plates 2a and 2b is shifted.

The marks 10c and 10d are configured such that the image reading direction of an imaging plate 2b is identified from the orientation of mark images 350c and 350d. When the image reading direction of the imaging plate 2b is the correct reading direction, one vertex of each of equilateral triangular mark images 350c and 350d corresponding to the marks 10c and 10d faces the reading start side. On the other hand, when the image reading direction of the imaging plate 2b is the incorrect reading direction, one vertex of the mark images 350c and 350d faces the reading end side.

The image processing unit 281, in a direction identification process, identifies the image reading direction of the imaging plate 2a based on the orientation of the mark images 350a and 350b included in the 1-th radiation image 310. Specifically, when one vertex of each of the mark images 350a and 350b included in the 1-th radiation image 310 faces the reading start side, the image processing unit 281 determines that the image reading direction of the imaging plate 2a is the correct reading direction. On the other hand, the image processing unit 281, in the direction identification process, determines that the image reading direction of the imaging plate 2a is the incorrect reading direction when one vertex of each of the mark images 350a and 350b faces the reading end side.

Similarly, the image processing unit 281, in the direction identification process, determines that the image reading direction of the imaging plate 2b is the correct reading direction when one vertex of each of mark images 350c and 350d included in a 2-th radiation image 320 faces the reading start side. On the other hand, the image processing unit 281, in the direction identification process, determines that the image reading direction of the imaging plate 2b is the incorrect reading direction when one vertex of each of the mark images 350c and 350d faces the reading end side.

When the image processing unit 281 determines in the direction identification process that the image reading direction of the 1-th imaging plate 2a is the correct reading direction, similarly to the first example, among two mark images 350 included in the 1-th radiation image 310, it sets the mark image 350 on the reading start side as a mark image 350a corresponding to the mark 10a, and the mark image 350 on the reading end side as a mark image 350b corresponding to the mark 10b. On the other hand, when the image processing unit 281 determines in the direction identification process that the image reading direction of the 1-th imaging plate 2a is the incorrect reading direction, among the two mark images 350 included in the 1-th radiation image 310, it sets the mark image 350 on the reading end side as a mark image 350a corresponding to the mark 10a, and the mark image 350 on the reading start side as a mark image 350b corresponding to the mark 10b.

Similarly, when the image processing unit 281 determines that the image reading direction of the 2-th imaging plate 2b is the correct reading direction, similarly to the first example, among two mark images 350 included in the 2-th radiation image 320, it sets the mark image 350 on the reading start side as a mark image 350c corresponding to the mark 10c, and the mark image 350 on the reading end side as a mark image 350d corresponding to the mark 10d. On the other hand, when the image processing unit 281 determines that the image reading direction of the 2-th imaging plate 2b is the incorrect reading direction, among the two mark images 350 included in the 2-th radiation image 320, it sets the mark image 350 on the reading end side as a mark image 350c corresponding to the mark 10c, and the mark image 350 on the reading start side as a mark image 350d corresponding to the mark 10d.

When the image processing unit 281 identifies a correspondence relationship between the mark images 350a, 350b, 350c, and 350d and positions 450a, 450b, 450c, and 450d in the first layout diagram 460, it arranges the 1-th radiation image 310 and the 2-th radiation image 320 on the first layout diagram 460, similarly to the first example. Specifically, the image processing unit 281 arranges the 2-th radiation image 320 on the first layout diagram 460 such that a position of the mark image 350c (for example, a position of its center of gravity) included in the 2-th radiation image 320 matches the position 450c of the mark 10c in the first layout diagram 460, and a position of a mark image 350d (for example, a position of its center of gravity) included in the 2-th radiation image 320 matches the position 450d of the mark 10d in the first layout diagram 460. Then, the image processing unit 281 arranges the 1-th radiation image 310 on the first layout diagram 460 such that a position of the mark image 350a included in the 1-th radiation image 310 matches the position 450a of the mark 10a in the first layout diagram 460, and a position of a mark image 350b included in the 1-th radiation image 310 matches the position 450b of the mark 10b in the first layout diagram 460. Thereby, as shown in the lower parts of FIGS. 21 and 24 described above, basically, the 1-th radiation image 310 and the 2-th radiation image 320 are registered such that the overlapping portion 311 of the 1-th radiation image 310 exactly overlaps the overlapping portion 321 of the 2-th radiation image 320. Therefore, the image processing unit 281 can generate the composite image 400 in which the subject is appropriately captured, regardless of whether the image reading direction of the imaging plate 2 is the correct reading direction or the incorrect reading direction. That is, regardless of whether a user of the reading device 100 inserts the imaging plate 2 into the reading device 100 from the posterior short side in the oral cavity or from the anterior short side in the oral cavity, the image processing unit 281 can generate the composite image 400 in which the subject is appropriately captured. As a result, the user does not need to worry about the direction in which the imaging plate 2 is inserted into the reading device 100, and usability of the reading device 100 is improved.

Note that the image processing unit 281 may use only one of the marks 10a and 10b as the reading direction identification mark and identify the image reading direction of the imaging plate 2a based on the orientation of a mark image 350 corresponding to the one mark 10. Similarly, the image processing unit 281 may use only one of the marks 10c and 10d as the reading direction identification mark and identify the image reading direction of the imaging plate 2b based on the orientation of a mark image 350 corresponding to the one mark 10.

The shape of the reading direction identification mark is not limited to the above example. As in this example, when the image reading direction of the imaging plate 2 is identified based on the orientation of the mark image 350 corresponding to the reading direction identification mark, the reading direction identification mark may have any shape as long as a 180-degree rotation of the reading direction identification mark is discernible. In other words, the reading direction identification mark should have such a shape that when the reading direction identification mark is rotated by 180 degrees, its orientation (in other words, posture) before rotation and its orientation (in other words, posture) after rotation can be distinguished. Therefore, when the image reading direction of the imaging plate 2 is identified based on the orientation of the mark image 350 corresponding to the reading direction identification mark, a mark such as a perfect circle or a rectangle, whose 180-degree rotation is not discernible, is not adopted as the reading direction identification mark. It can also be said that the reading direction identification mark may have any shape as long as the orientation of the mark image 350 corresponding to the reading direction identification mark can be distinguished between when the image reading direction of the imaging plate 2 is the correct reading direction and when it is the incorrect reading direction. The reading direction identification mark may be a rotationally symmetric figure other than an equilateral triangle, or it may be non-rotationally symmetric. The shape of the marks 10 other than the reading direction identification mark included in the plurality of marks 10 of the holding member 5 may be any shape.

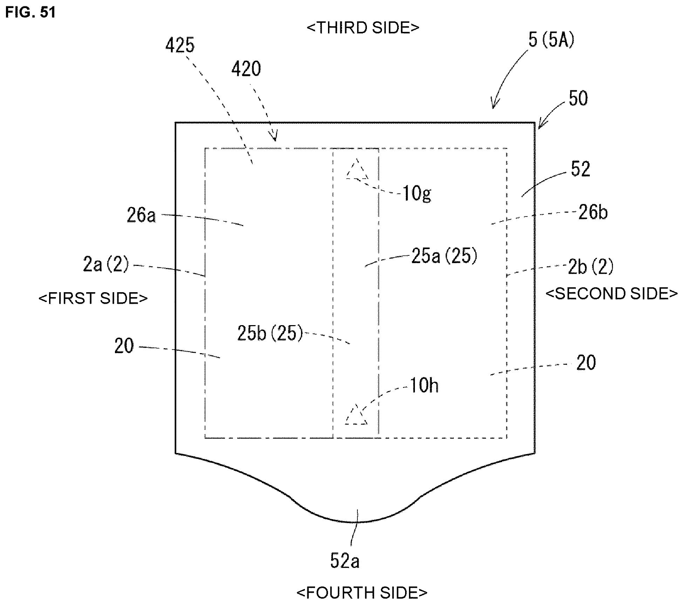

In the above example, the reading direction identification mark is used in the first example, but the reading direction identification mark may be used in a second example. FIG. 51 is a schematic diagram illustrating an example of the holding member 5A in this case. The example of FIG. 51 is a modification of the example of FIG. 29 described above, in which the shapes of marks 10g and 10h are changed from circles to equilateral triangles. In the holding member 5A, the marks 10g and 10h are arranged in the same orientation. For example, the marks 10g and 10h are arranged such that one vertex faces a third side. The marks 10g and 10h are used as reading direction identification marks.

The marks 10g and 10h are configured such that the image reading direction of the imaging plate 2a is identified from the orientation of mark images 350ga and 350ha corresponding thereto. In the case of the example of FIG. 51, when the image reading direction of the imaging plate 2a is the correct reading direction, one vertex of each of equilateral triangular mark images 350ga and 350ha corresponding to the marks 10g and 10h faces a reading start side. On the other hand, when the image reading direction of the imaging plate 2a is the incorrect reading direction, one vertex of the mark images 350ga and 350ha faces a reading end side.

Further, the marks 10g and 10h are configured such that the image reading direction of an imaging plate 2b is identified from the orientation of mark images 350gb and 350hb corresponding thereto. When the image reading direction of the imaging plate 2b is the correct reading direction, one vertex of each of equilateral triangular mark images 350gb and 350hb corresponding to the marks 10g and 10h faces the reading start side. On the other hand, when the image reading direction of the imaging plate 2b is the incorrect reading direction, one vertex of the mark images 350gb and 350hb faces the reading end side.

The image processing unit 281, in the direction identification process, determines that the image reading direction of the imaging plate 2a is the correct reading direction when one vertex of each of the mark images 350ga and 350ha included in the 1-th radiation image 310 faces the reading start side. On the other hand, the image processing unit 281, in the direction identification process, determines that the image reading direction of the imaging plate 2a is the incorrect reading direction when one vertex of each of the mark images 350ga and 350ha faces the reading end side. The image processing unit 281 can similarly identify the image reading direction of the 2-th imaging plate 2b based on the orientation of the mark images 350gb and 350hb.

When the image processing unit 281 determines that the image reading direction of the 1-th imaging plate 2a is the correct reading direction, similarly to the second example, among two mark images 350 included in the 1-th radiation image 310, it sets the mark image 350 on the reading start side as a mark image 350ga corresponding to the mark 10g, and the mark image 350 on the reading end side as a mark image 350ha corresponding to the mark 10h. On the other hand, when the image processing unit 281 determines that the image reading direction of the 1-th imaging plate 2a is the incorrect reading direction, among the two mark images 350 included in the 1-th radiation image 310, it sets the mark image 350 on the reading start side as a mark image 350ha corresponding to the mark 10h, and the mark image 350 on the reading end side as a mark image 350ga corresponding to the mark 10g.

Similarly, when the image processing unit 281 determines that the image reading direction of the 2-th imaging plate 2b is the correct reading direction, among two mark images 350 included in the 2-th radiation image 320, it sets the mark image 350 on the reading start side as a mark image 350gb corresponding to the mark 10g, and the mark image 350 on the reading end side as a mark image 350hb corresponding to the mark 10h. On the other hand, when the image processing unit 281 determines that the image reading direction of the 2-th imaging plate 2b is the incorrect reading direction, among the two mark images 350 included in the 2-th radiation image 320, it sets the mark image 350 on the reading start side as a mark image 350hb corresponding to the mark 10h, and the mark image 350 on the reading end side as a mark image 350gb corresponding to the mark 10g.

Thereafter, the image processing unit 281, similarly to the second example, partially overlaps the 1-th radiation image 310 and the 2-th radiation image 320 such that a position of a mark image 350ga and a position of a mark image 350gb corresponding to the same mark 10g match each other, and a position of a mark image 350ha and a position of a mark image 350hb corresponding to the same mark 10h match each other.

Note that the image processing unit 281 may use only one of the marks 10g and 10h as the reading direction identification mark and identify the image reading direction of the imaging plates 2a and 2b based on the orientation of a mark image 350 corresponding to the one mark 10.

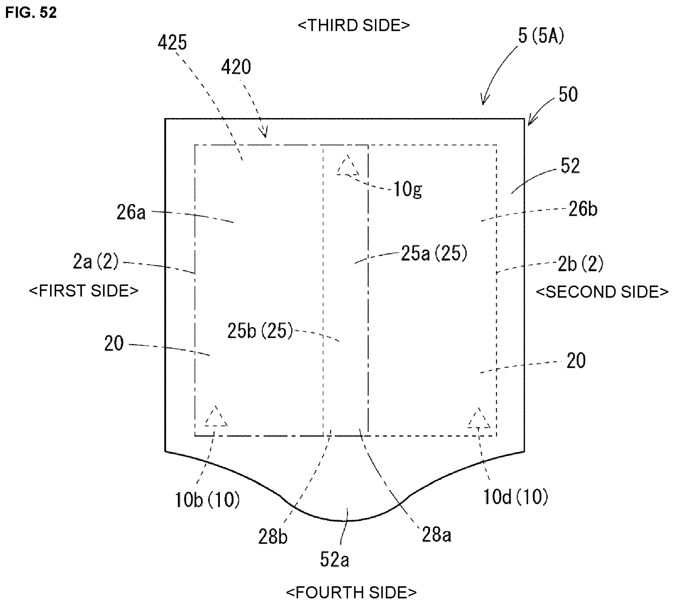

In the third example described above, a reading direction identification mark may be used. FIG. 52 is a schematic diagram illustrating an example of the holding member 5A in this case. The example of FIG. 52 is a modification of the example of FIG. 33 described above, in which the shapes of marks 10b, 10d, and 10g are changed from circles to equilateral triangles. In the holding member 5A, the marks 10b, 10d, and 10g are arranged, for example, such that one vertex faces a third side. The marks 10b, 10d, and 10g are used as reading direction identification marks.

The image processing unit 281, in the direction identification process, determines that the image reading direction of the imaging plate 2a is the correct reading direction when one vertex of each of mark images 350b and 10ga included in the 1-th radiation image 310 faces the reading start side. On the other hand, the image processing unit 281, in the direction identification process, determines that the image reading direction of the imaging plate 2a is the incorrect reading direction when one vertex of each of the mark images 350b and 350ga faces the reading end side. The image processing unit 281 can similarly identify the image reading direction of the 2-th imaging plate 2b based on the orientation of mark images 350d and 350gb.

When the image processing unit 281 determines that the image reading direction of the 1-th imaging plate 2a is the correct reading direction, among two mark images 350 included in the 1-th radiation image 310, it sets the mark image 350 on the reading start side as a mark image 350ga corresponding to a mark 10g, and the mark image 350 on the reading end side as a mark image 350b corresponding to a mark 10b. On the other hand, when the image processing unit 281 determines that the image reading direction of the first imaging plate 2a is the incorrect reading direction, among the two mark images 350 included in the 1-th radiation image 310, it sets the mark image 350 on the reading end side as a mark image 350ga corresponding to the mark 10g, and the mark image 350 on the reading start side as a mark image 350b corresponding to the mark 10b.

Similarly, when the image processing unit 281 determines that the image reading direction of the 2-th imaging plate 2b is the correct reading direction, among two mark images 350 included in the 2-th radiation image 320, it sets the mark image 350 on the reading start side as a mark image 350gb corresponding to the mark 10g, and the mark image 350 on the reading end side as a mark image 350d corresponding to a mark 10d. On the other hand, when the image processing unit 281 determines that the image reading direction of the second imaging plate 2b is the incorrect reading direction, among the two mark images 350 included in the 2-th radiation image 320, it sets the mark image 350 on the reading start side as a mark image 350d corresponding to the mark 10d, and the mark image 350 on the reading end side as a mark image 350gb corresponding to the mark 10g.

Thereafter, the image processing unit 281, similarly to the third example, arranges the 1-th radiation image 310 and the 2-th radiation image 320 on the first layout diagram 460 such that a position of a mark image 350ga corresponding to the mark 10g included in the 1-th radiation image 310 and a position of a mark image 350gb corresponding to the mark 10g included in the 2-th radiation image 320 match each other, while a mark image 350b corresponding to a mark 10b included in the 1-th radiation image 310 and a mark image 350d corresponding to a mark 10d included in the 2-th radiation image 320 respectively match positions 450b and 450d shown in the first layout diagram 460.

Note that the image processing unit 281 may use only one of the marks 10b and 10g as the reading direction identification mark and identify the image reading direction of the imaging plate 2a based on the orientation of a mark image 350 corresponding to the one mark 10. Similarly, the image processing unit 281 may use only one of the marks 10d and 10g as the reading direction identification mark and identify the image reading direction of the imaging plate 2b based on the orientation of a mark image 350 corresponding to the one mark 10.

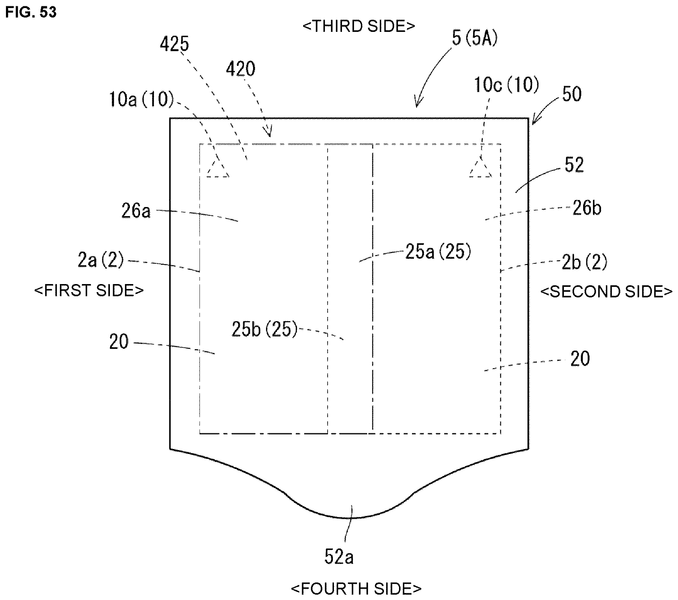

In the fourth example described above, a reading direction identification mark may be used. FIG. 53 is a schematic diagram illustrating an example of the holding member 5A in this case. The example of FIG. 53 is a modification of the example of FIG. 35 described above, in which the shape of a mark 10c is changed to an equilateral triangle. In the holding member 5A, marks 10b and 10c are arranged, for example, such that one vertex faces a third side. The marks 10b and 10c are used as reading direction identification marks.

The image processing unit 281, in the direction identification process, determines that the image reading direction of a 1-th imaging plate 2a is the correct reading direction when one vertex of a mark image 350a included in a 1-th radiation image 310 faces a reading start side. On the other hand, the image processing unit 281, in the direction identification process, determines that the image reading direction of the 1-th imaging plate 2a is the incorrect reading direction when one vertex of the mark image 350a included in the 1-th radiation image 310 faces a reading end side. In the direction identification process, the image processing unit 281 can similarly identify the image reading direction of a 2-th imaging plate 2b based on the orientation of a mark image 350b.

Next, a registration process in the image processing unit 281 will be described. First, a case where it is determined in the direction identification process that the image reading directions of the imaging plates 2a and 2b are both the correct reading direction is considered. In this case, the image processing unit 281 performs registration of the 1-th radiation image 310 and the 2-th radiation image 320 in the same manner as in the fourth example.

Next, a case where it is determined in the direction identification process that the image reading direction of the imaging plate 2a is the correct reading direction and the image reading direction of the imaging plate 2b is the incorrect reading direction is considered. In this case, when arranging the 1-th radiation image 310 and the 2-th radiation image 320 on a second layout diagram 490, the image processing unit 281 rotates the 2-th radiation image 320 by 180 degrees as compared to the fourth example. That is, the image processing unit 281 exactly superimposes the mark images 350a and 350c on mark corresponding diagrams 480a and 480c of the second layout diagram 490, respectively, such that a right side of the 1-th radiation image 310 is positioned on a 2-th radiation image 320 side, a right side of the 2-th radiation image 320 is positioned on a 1-th radiation image 310 side, and a long side of the 1-th radiation image 310 and a long side of the 2-th radiation image 320 are parallel or substantially parallel. Thereby, basically, the 1-th radiation image 310 and the 2-th radiation image 320 are registered such that the overlapping portion 311 of the 1-th radiation image 310 exactly overlaps an overlapping portion 321 of the 2-th radiation image 320. Note that the right side and the left side of the 1-th radiation image 310 respectively mean the right side and the left side when the 1-th radiation image 310 is arranged with the reading start side facing upward and the reading end side facing downward as described above (see the upper part of FIG. 37). The same applies to the right side and the left side of the 2-th radiation image 320.

Next, a case where it is determined in the direction identification process that the image reading direction of the imaging plate 2a is the incorrect reading direction and the image reading direction of the imaging plate 2b is the correct reading direction is considered. In this case, when arranging the 1-th radiation image 310 and the 2-th radiation image 320 on the second layout diagram 490, the image processing unit 281 rotates the 1-th radiation image 310 by 180 degrees as compared to the fourth example. That is, the image processing unit 281 exactly superimposes the mark images 350a and 350c on the mark corresponding diagrams 480a and 480c, respectively, such that a left side of the 1-th radiation image 310 is positioned on the 2-th radiation image 320 side, a left side of the 2-th radiation image 320 is positioned on the 1-th radiation image 310 side, and a long side of the 1-th radiation image 310 and a long side of the 2-th radiation image 320 are parallel or substantially parallel.

Finally, a case where it is determined in the direction identification process that the image reading directions of the imaging plates 2a and 2b are both the incorrect reading direction is considered. In this case, when arranging the 1-th radiation image 310 and the 2-th radiation image 320 on the second layout diagram 490, the image processing unit 281 rotates the 1-th radiation image 310 and the 2-th radiation image 320 by 180 degrees as compared to the fourth example. That is, the image processing unit 281 exactly superimposes the mark images 350a and 350c on the mark corresponding diagrams 480a and 480c, respectively, such that a left side of the 1-th radiation image 310 is positioned on the 2-th radiation image 320 side, a right side of the 2-th radiation image 320 is positioned on the 1-th radiation image 310 side, and a long side of the 1-th radiation image 310 and a long side of the 2-th radiation image 320 are parallel or substantially parallel.

Note that when the mark 10a is non-rotationally symmetric, the 1-th radiation image 310 is appropriately arranged on the second layout diagram 490 by exactly superimposing a mark image 350a corresponding to the mark 10a on a mark corresponding diagram 480a of the second layout diagram 490, regardless of whether the image reading direction of the imaging plate 2a is the correct reading direction or the incorrect reading direction. Therefore, when the mark 10a is non-rotationally symmetric, the identification of the image reading direction of the imaging plate 2a may not be executed. Similarly, when a mark 10b is non-rotationally symmetric, the identification of the image reading direction of an imaging plate 2b may not be executed.

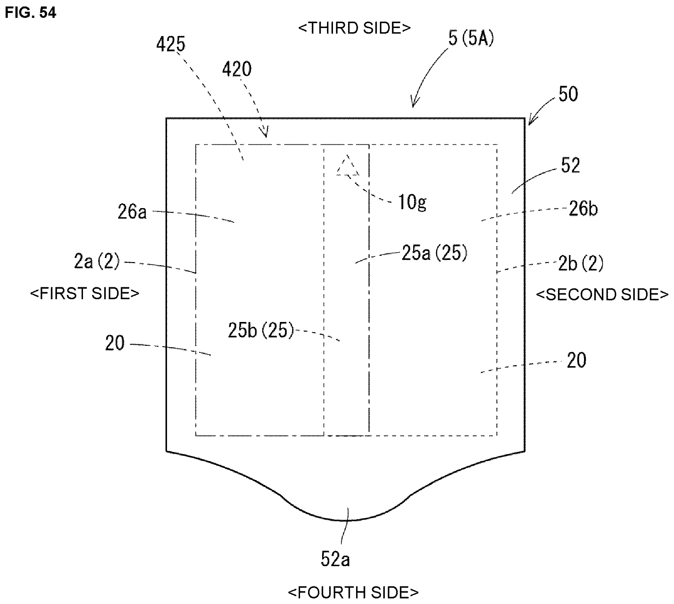

In the fifth example described above, a reading direction identification mark may be used. FIG. 54 is a schematic diagram illustrating an example of the holding member 5A in this case. The example of FIG. 54 is a modification of the example of FIG. 39 described above, in which the shape of a mark 10g is changed to an equilateral triangle. The mark 10g is used as a reading direction identification mark.

The image processing unit 281, in the direction identification process, determines that the image reading direction of a 1-th imaging plate 2a is the correct reading direction when one vertex of a mark image 350ga included in a 1-th radiation image 310 faces a reading start side. On the other hand, the image processing unit 281, in the direction identification process, determines that the image reading direction of the 1-th imaging plate 2a is the incorrect reading direction when one vertex of the mark image 350ga included in the 1-th radiation image 310 faces a reading end side. The image processing unit 281 can similarly identify the image reading direction of a 2-th imaging plate 2b based on the orientation of a mark image 350gb.

Next, registration of the imaging plate 2 by the image processing unit 281 will be described. First, a case where it is determined in the direction identification process that the image reading directions of imaging plates 2a and 2b are both the correct reading direction is considered. In this case, the image processing unit 281 performs registration of the 1-th radiation image 310 and the 2-th radiation image 320 in the same manner as in the fifth example.

Next, a case where it is determined in the direction identification process that the image reading direction of the imaging plate 2a is the correct reading direction and the image reading direction of the imaging plate 2b is the incorrect reading direction is considered. In this case, the image processing unit 281 exactly superimposes a mark image 350ga and a mark image 350gb on each other such that a right side of a 1-th radiation image 310 is positioned on a 2-th radiation image 320 side, a right side of the 2-th radiation image 320 is positioned on a 1-th radiation image 310 side, and a long side of the 1-th radiation image 310 and a long side of the 2-th radiation image 320 are parallel or substantially parallel.

Next, a case where it is determined in the direction identification process that the image reading direction of the imaging plate 2a is the incorrect reading direction and the image reading direction of the imaging plate 2b is the correct reading direction is considered. In this case, the image processing unit 281 exactly superimposes the mark image 350ga and the mark image 350gb on each other such that a left side of the 1-th radiation image 310 is positioned on the 2-th radiation image 320 side, a left side of the 2-th radiation image 320 is positioned on the 1-th radiation image 310 side, and a long side of the 1-th radiation image 310 and a long side of the 2-th radiation image 320 are parallel or substantially parallel.

Finally, a case where it is determined in the direction identification process that the image reading directions of the imaging plates 2a and 2b are both the incorrect reading direction is considered. In this case, the image processing unit 281 exactly superimposes the mark image 350ga and the mark image 350gb on each other such that a left side of the 1-th radiation image 310 is positioned on the 2-th radiation image 320 side, a right side of the 2-th radiation image 320 is positioned on the 1-th radiation image 310 side, and a long side of the 1-th radiation image 310 and a long side of the 2-th radiation image 320 are parallel or substantially parallel.

Note that when the mark 10g is non-rotationally symmetric, the 1-th radiation image 310 and the 2-th radiation image 320 are appropriately registered such that an overlapping portion 311 of the 1-th radiation image 310 and an overlapping portion 321 of the 2-th radiation image 320 exactly overlap by superimposing a mark image 350ga and a mark image 350gb exactly on each other, regardless of whether the image reading direction of the imaging plates 2a and 2b is the correct reading direction or the incorrect reading direction. Therefore, when the mark 10g is non-rotationally symmetric, the direction identification process may not be executed.

In the sixth example described above, the direction identification process may not be executed. In the sixth example, a 1-th radiation image 310 and a 2-th radiation image 320 are arranged on a first layout diagram 460 such that a position of a mark image 350a included in the 1-th radiation image 310 matches a position 450a on the first layout diagram 460, a position of a mark image 350c included in the 2-th radiation image 320 matches a position 450c on the first layout diagram 460, and one long side of the 1-th radiation image 310 and a long side corresponding image 326 of the 2-th radiation image 320 overlap each other. Thereby, basically, the 1-th radiation image 310 and the 2-th radiation image 320 are appropriately registered such that an overlapping portion 311 of the 1-th radiation image 310 and an overlapping portion 321 of the 2-th radiation image 320 exactly overlap, regardless of whether the image reading direction of the imaging plates 2a and 2b is the correct reading direction or the incorrect reading direction. Therefore, in the sixth example, the direction identification process may not be executed.

In the seventh example described above, the direction identification process may not be executed. In the seventh example, the 1-th radiation image 310 and the 2-th radiation image 320 are registered such that a position of a mark image 350ga included in the 1-th radiation image 310 and a position of a mark image 350gb included in the 2-th radiation image 320 match each other, and one long side of the 1-th radiation image 310 overlaps a long side corresponding image 326 of the 2-th radiation image 320. Thereby, basically, the 1-th radiation image 310 and the 2-th radiation image 320 are appropriately registered such that an overlapping portion 311 of the 1-th radiation image 310 and an overlapping portion 321 of the 2-th radiation image 320 exactly overlap, regardless of whether the image reading direction of the imaging plates 2a and 2b is the correct reading direction or the incorrect reading direction. Therefore, in the seventh example, the direction identification process may not be executed.

The image processing unit 281 may identify the image reading direction of the imaging plate 2 based on a position of a mark image 350 corresponding to a reading direction identification mark, instead of an orientation of the mark image 350 corresponding to the reading direction identification mark.

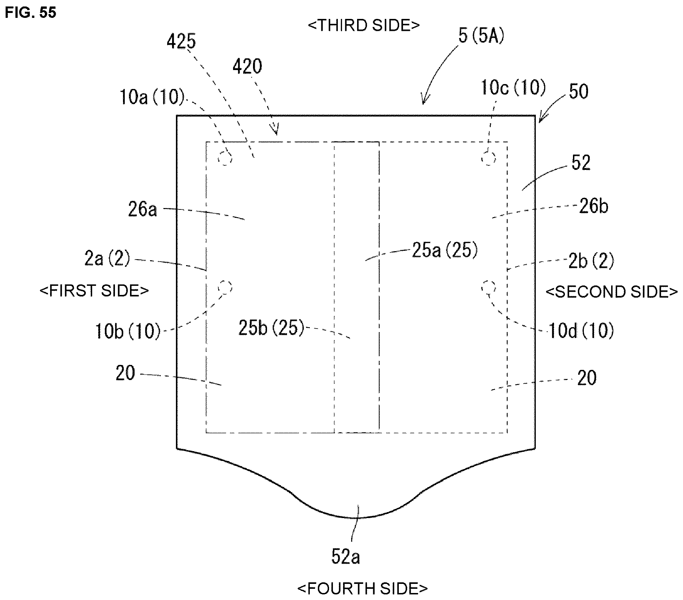

FIG. 55 is a schematic diagram illustrating an example of the holding member 5A in a case where the image reading direction of the imaging plate 2 is identified based on the position of the mark image 350 corresponding to the reading direction identification mark in the first example.

The example of FIG. 55 is a modification of the example of FIG. 16 described above, in which positions of marks 10b and 10d are changed. In the example of FIG. 55, a mark image 350b corresponding to the mark 10b is recorded at a central portion in a longitudinal direction of an imaging plate 2a at an end portion on a first side of the imaging plate 2a, unlike the example of FIG. 17 described above. Further, a mark image 350d corresponding to a mark 10d is recorded at a central portion in a longitudinal direction of an imaging plate 2b at an end portion on a second side of the imaging plate 2b, unlike the example of FIG. 17. The marks 10a, 10b, 10c, and 10d are used as reading direction identification marks.

The marks 10a and 10b are configured such that the image reading direction of the imaging plate 2a is identified from the positions of mark images 350a and 350b corresponding thereto. In this example, a layout of the mark images 350a and 350b in a 1-th radiation image 310 differs between a case where the image reading direction of the imaging plate 2a is the correct reading direction and a case where it is the incorrect reading direction. When the image reading direction of the imaging plate 2a is the correct reading direction, in the 1-th radiation image 310, a mark image 350a corresponding to the mark 10a is located on a reading start side, and a mark image 350b corresponding to the mark 10b is located at a central portion in a longitudinal direction of the 1-th radiation image 310. On the other hand, when the image reading direction of the imaging plate 2a is the incorrect reading direction, in the 1-th radiation image 310, the mark image 350a is located on a reading end side, and the mark image 350b is located at the central portion in the longitudinal direction of the 1-th radiation image 310.

The marks 10c and 10d are configured such that the image reading direction of an imaging plate 2b is identified from the positions of mark images 350c and 350d corresponding thereto. When the image reading direction of the imaging plate 2b is the correct reading direction, in a 2-th radiation image 320, a mark image 350c corresponding to the mark 10c is located on a reading start side, and a mark image 350d corresponding to a mark 10d is located at a central portion in a longitudinal direction of the 2-th radiation image 320. On the other hand, when the image reading direction of the imaging plate 2b is the incorrect reading direction, in the 2-th radiation image 320, the mark image 350c is located on a reading end side, and the mark image 350d is located at the central portion in the longitudinal direction of the 2-th radiation image 320.

The image processing unit 281 identifies the image reading direction of the imaging plate 2a based on the positions (in other words, layout) of the mark images 350a and 350b included in the 1-th radiation image 310. Specifically, when, of two mark images 350a and 350b included in the 1-th radiation image 310, a position of one mark image 350 is located on the reading start side and a position of the other mark image 350 is located at a central portion in a longitudinal direction of the 1-th radiation image 310, the image processing unit 281 determines that the image reading direction of the imaging plate 2a is the correct reading direction. On the other hand, when the position of one mark image 350 is located on the reading end side and the position of the other mark image 350 is located at the central portion in the longitudinal direction of the 1-th radiation image 310, the image processing unit 281 determines that the image reading direction of the imaging plate 2a is the incorrect reading direction.

Similarly, when, of two mark images 350c and 350d included in the 2-th radiation image 320, a position of one mark image 350 is located on the reading start side and a position of the other mark image 350 is located at a central portion in a longitudinal direction of the 2-th radiation image 320, the image processing unit 281 determines that the image reading direction of an imaging plate 2b is the correct reading direction. On the other hand, when the position of one mark image 350 is located on the reading end side and the position of the other mark image 350 is located at the central portion in the longitudinal direction of the 2-th radiation image 320, the image processing unit 281 determines that the image reading direction of the imaging plate 2b is the incorrect reading direction.

After the direction identification process, the image processing unit 281 performs registration of the 1-th radiation image 310 and the 2-th radiation image 320 in the same manner as in the eighth example described above.

Note that in the case of the example of FIG. 55, only a mark 10a of the marks 10a and 10b may be used as the reading direction identification mark. When the image reading direction of the imaging plate 2a is the correct reading direction, a mark image 350a corresponding to the mark 10a is located at an end portion on a reading start side of a 1-th radiation image 310. On the other hand, when the image reading direction of the imaging plate 2a is the incorrect reading direction, the mark image 350a corresponding to the mark 10a is located at an end portion on a reading end side of the 1-th radiation image 310. When a mark image 350 is located at the end portion on the reading start side of the 1-th radiation image 310, the image processing unit 281 determines that the image reading direction of the imaging plate 2a is the correct reading direction. Then, when the mark image 350 is located at the end portion on the reading end side of the 1-th radiation image 310, the image processing unit 281 determines that the image reading direction of the imaging plate 2a is the incorrect reading direction. Similarly, only a mark 10c of marks 10c and 10d may be used as the reading direction identification mark.

The image processing unit 281 can identify the image reading direction of the imaging plates 2a and 2b even if a layout of a plurality of marks 10a, 10b, 10c, and 10d is a layout like that of FIGS. 16 and 49 described above. In the case of the examples of FIGS. 16 and 49, when the image reading direction of the imaging plate 2a is the correct reading direction, two mark images 350a and 350b included in a 1-th radiation image 310 are located on a left side of the 1-th radiation image 310 (see the upper part of FIG. 50). On the other hand, when the image reading direction of the imaging plate 2a is the incorrect reading direction, the two mark images 350a and 350b included in the 1-th radiation image 310 are located on a right side of the 1-th radiation image 310 (see the lower part of FIG. 50). The same applies to two mark images 350c and 350d included in a 2-th radiation image 320.

When two mark images 350 (that is, mark images 350a and 350b) included in the 1-th radiation image 310 are located on the left side of the 1-th radiation image 310, the image processing unit 281 determines that the image reading direction of an imaging plate 2a is the correct reading direction. On the other hand, when the two mark images included in the 1-th radiation image 310 are located on the right side of the 1-th radiation image 310, the image processing unit 281 determines that the image reading direction of the imaging plate 2a is the incorrect reading direction. The image processing unit 281 can similarly identify the image reading direction of an imaging plate 2b based on the positions (in other words, layout) of mark images 350c and 350d included in a 2-th radiation image 320.

As in this example, when the image reading direction of the imaging plate 2 is identified based on the position of a mark image 350, if a layout of a plurality of mark images 350 included in a read radiation image read from the imaging plate 2 in the read radiation image can be distinguished between a case where the image reading direction of the imaging plate 2 is the correct reading direction and a case where it is the incorrect reading direction, the image processing unit 281 can identify the image reading direction of the imaging plate 2. That is, if a layout of the plurality of mark images 350 before rotation and a layout of the plurality of mark images 350 after rotation can be distinguished when the read radiation image is rotated by 180 degrees, the image processing unit 281 can identify the image reading direction of the imaging plate 2.

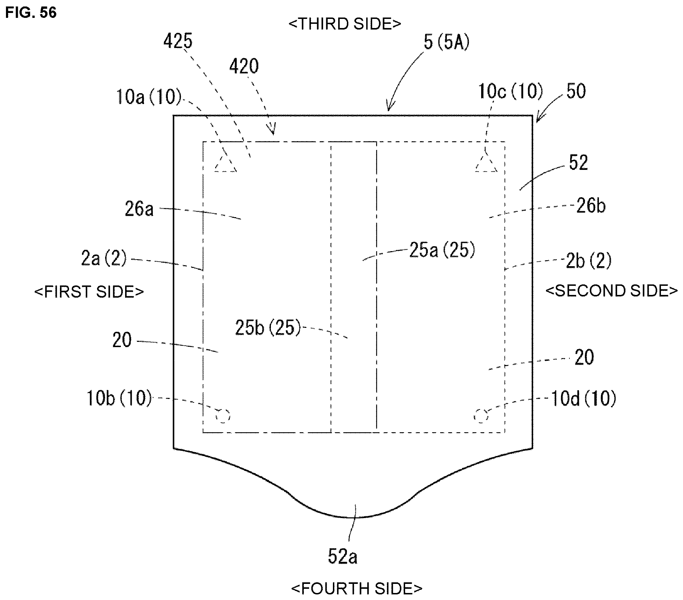

FIG. 56 is a schematic diagram illustrating another example of the holding member 5A. The example of FIG. 56 is a modification of the example of FIG. 16 described above, in which shapes of marks 10a and 10c are changed to equilateral triangles. In the example of FIG. 56, triangular marks 10a and 10c are used as reading direction identification marks.

In the case of the example of FIG. 56, the image processing unit 281 identifies the image reading direction of an imaging plate 2a based on a position of a mark image 350a corresponding to a mark 10a included in a 1-th radiation image 310. In the example of FIG. 56, when the image reading direction of the imaging plate 2a is the correct reading direction, a triangular mark image 350a is located on a reading start side in the 1-th radiation image 310. On the other hand, when the image reading direction of the imaging plate 2a is the incorrect reading direction, the triangular mark image 350a is located on a reading end side in the 1-th radiation image 310. When a triangular mark image 350 included in the first radiation image 310 is located on the reading start side, the image processing unit 281 determines that the image reading direction of the imaging plate 2a is the correct reading direction. Further, when the triangular mark image 350 included in the first radiation image 310 is located on the reading end side, the image processing unit 281 determines that the image reading direction of the imaging plate 2a is the incorrect reading direction. In the case of the example of FIG. 56, since the image processing unit 281 identifies the shape of the mark image 350, it can be said that the image processing unit 281 identifies the image reading direction of the imaging plate 2a based on the position and shape of the mark image 350a. The image processing unit 281 may similarly identify the image reading direction of an imaging plate 2b based on a position of a mark image 350c corresponding to a mark 10c included in a 2-th radiation image 320.

Note that in the example of FIG. 56, circular marks 10b and 10d may be used as reading direction identification marks. When the image reading direction of an imaging plate 2a is the correct reading direction, a circular mark image 350b is located on a reading end side in a 1-th radiation image 310. On the other hand, when the image reading direction of the imaging plate 2a is the incorrect reading direction, the circular mark image 350b is located on a reading start side in the 1-th radiation image 310. When a circular mark image 350 included in the 1-th radiation image 310 is located on the reading end side, the image processing unit 281 determines that the image reading direction of the imaging plate 2a is the correct reading direction. Further, when the circular mark image 350 included in the 1-th radiation image 310 is located on the reading start side, the image processing unit 81 determines that the image reading direction of the imaging plate 2a is the incorrect reading direction. The image processing unit 281 may similarly identify the image reading direction of an imaging plate 2b based on a position of a mark image 350d corresponding to a mark 10d included in a 2-th radiation image 320.

Further, in the example of FIG. 56, the image processing unit 281 may identify the image reading direction of an imaging plate 2a based on an orientation and a position of a mark image 350a corresponding to a mark 10a included in a 1-th radiation image 310. In this case, when a triangular mark image 350 included in the 1-th radiation image 310 is located on a reading start side and one vertex of the mark image 350 faces the reading start side, the image processing unit 281 determines that the image reading direction of the imaging plate 2a is the correct reading direction. On the other hand, when the triangular mark image 350 included in the 1-th radiation image 310 is located on a reading end side and one vertex of the mark image 350 faces the reading end side, the image processing unit 281 determines that the image reading direction of the imaging plate 2a is the incorrect reading direction. In the example of FIG. 56, it can be said that the mark 10a is configured such that the image reading direction of the imaging plate 2a is identified from the orientation and position of a mark image 350a. The image processing unit 281 may similarly identify the image reading direction of an imaging plate 2b based on an orientation and a position of a mark image 350c corresponding to a mark 10c included in a 2-th radiation image 320.

In the above example, the image reading direction of the imaging plate 2 is identified based on the position of the mark image 350 corresponding to the reading direction identification mark in the first example, but the image reading direction of the imaging plate 2 may be identified based on the position of the mark image 350 corresponding to the reading direction identification mark in a second example. For example, the example of FIG. 29 described above is considered. In this case, when two mark images 350 included in a 1-th radiation image 310 are located on a right side of the 1-th radiation image 310, the image processing unit 281 determines that the image reading direction of an imaging plate 2a is the correct reading direction. On the other hand, when the two mark images 350 included in the 1-th radiation image 310 are located on a left side of the 1-th radiation image 310, the image processing unit 281 determines that the image reading direction of the imaging plate 2a is the incorrect reading direction. Further, when two mark images 350 included in a 2-th radiation image 320 are located on a left side of the second radiation image 320, the image processing unit 281 determines that the image reading direction of an imaging plate 2b is the correct reading direction. On the other hand, when the two mark images 350 included in the 2-th radiation image 320 are located on a right side of the 2-th radiation image 320, the image processing unit 281 determines that the image reading direction of the imaging plate 2b is the incorrect reading direction. Note that also in the second example, the image reading direction of the imaging plate 2 may be identified based on the orientation and position of the mark image 350 corresponding to the reading direction identification mark.

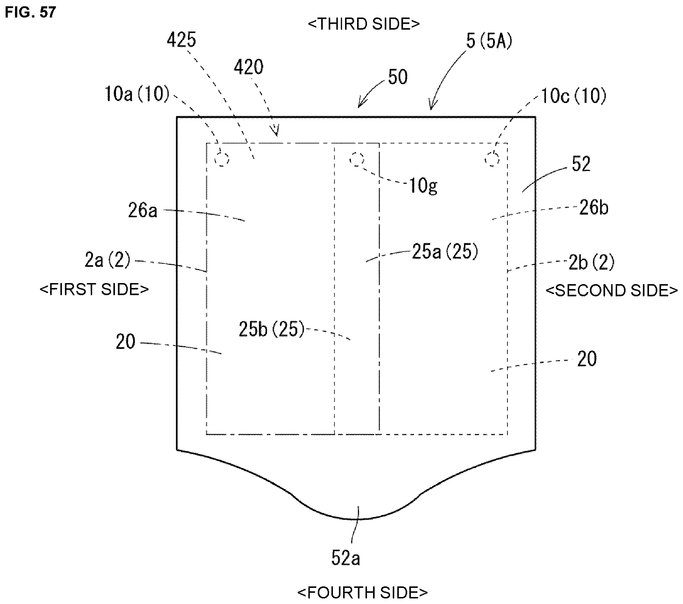

In a third example, the image reading direction of the imaging plate 2 may be identified based on the position of a mark image 350 corresponding to a reading direction identification mark. For example, the example of FIG. 57 is considered. In this case, when two mark images 350 included in a 1-th radiation image 310 are located on a reading start side of the 1-th radiation image 310, the image processing unit 281 determines that the image reading direction of an imaging plate 2a is the correct reading direction. On the other hand, when the two mark images 350 included in the 1-th radiation image 310 are located on a reading end side of the 1-th radiation image 310, the image processing unit 281 determines that the image reading direction of the imaging plate 2a is the correct reading direction. The image processing unit 281 can similarly identify the image reading direction of an imaging plate 2b based on the positions of two mark images 350 included in a 2-th radiation image 320. Note that also in the third example, the image reading direction of the imaging plate 2 may be identified based on the orientation and position of the mark image 350 corresponding to the reading direction identification mark.

In a fourth example, the image reading direction of the imaging plate 2 may be identified based on the position of a mark image 350 corresponding to a reading direction identification mark. For example, the example of FIG. 35 described above is considered. In this case, when a mark image 350 included in a 1-th radiation image 310 is located on a reading start side of the 1-th radiation image 310, the image processing unit 281 determines that the image reading direction of an imaging plate 2a is the correct reading direction. On the other hand, when the mark image 350 included in the 1-th radiation image 310 is located on a reading end side of the 1-th radiation image 310, the image processing unit 281 determines that the image reading direction of the imaging plate 2a is the correct reading direction. The image processing unit 281 can similarly identify the image reading direction of an imaging plate 2b based on the position of a mark image 350 included in a 2-th radiation image 320. Note that also in the fourth example, the image reading direction of the imaging plate 2 may be identified based on the orientation and position of the mark image 350 corresponding to the reading direction identification mark.

In a fifth example, the image reading direction of the imaging plate 2 may be identified based on the position of a mark image 350 corresponding to a reading direction identification mark. For example, the example of FIG. 39 described above is considered. In this case, when a mark image 350 included in a 1-th radiation image 310 is located on a reading start side of the 1-th radiation image 310, the image processing unit 281 determines that the image reading direction of an imaging plate 2a is the correct reading direction. On the other hand, when the mark image 350 included in the 1-th radiation image 310 is located on a reading end side of the 1-th radiation image 310, the image processing unit 281 determines that the image reading direction of the imaging plate 2a is the correct reading direction. The image processing unit 281 can similarly identify the image reading direction of an imaging plate 2b based on the position of a mark image 350 included in a 2-th radiation image 320. Note that also in the fifth example, the image reading direction of the imaging plate 2 may be identified based on the orientation and position of the mark image 350 corresponding to the reading direction identification mark.

Although the case of N=2 has been mainly described above, even when N≥3, the image processing unit 281 can similarly identify the image reading direction of N imaging plates 2 based on a mark image 350 corresponding to a reading direction identification mark included in N read radiation images.

As described above, in this example, since the image processing unit 281 performs alignment of N read radiographic images based on the reading direction of the radiographic image from each of the N imaging plates 2 and the mark image 350 included in the N read radiographic images read from the N imaging plates 2, alignment of the N read radiographic images can be performed easily and appropriately.

### <Ninth Example (Identification of Stacking Order)>

For example, it is assumed that a user inserts an imaging plate 2 on a first side of a holding member 5A into a reading device 100, and then inserts an imaging plate 2 on a second side of the holding member 5A into the reading device 100, among two imaging plates 2 held by the holding member 5A. Then, it is assumed that an image processing unit 281 performs registration of the read radiation images with a read radiation image read from the later inserted imaging plate 2 placed above a read radiation image read from the earlier inserted imaging plate 2. Further, the image processing unit 281, as in the example of FIG. 15, deletes an overlapping portion with a lower radiation image from an upper radiation image, leaves an overlapping portion with the upper radiation image in the lower radiation image, and combines the upper radiation image and a lower radiation latent image.

In such a case, as in the example above, when stacking order information indicated by the holding member 5A indicates that an imaging plate 2 on a first side is stacked so as to be positioned above an imaging plate 2 on a second side, the image processing unit 281 performs registration of a 1-th radiation image 310 and a 2-th radiation image 320 such that the 1-th radiation image 310 read from the imaging plate 2 on the first side positioned above is positioned above the 2-th radiation image 320 read from the imaging plate 2 on the second side positioned below. Then, the image processing unit 281 leaves an overlapping portion 311 included in the upper 1-th radiation image 310, deletes an overlapping portion 321 included in the lower 2-th radiation image 320, and combines the 1-th radiation image 310 and the 2-th radiation image 320.

Here, it is assumed that it is ideal for the image processing unit 281 to combine a 1-th radiation image 310 and a 2-th radiation image 320 by, for example, leaving a high-quality overlapping portion 311 included in the 1-th radiation image 310 read from an upper imaging plate 2 and deleting a low-quality overlapping portion 321 included in the 2-th radiation image 320 read from a lower imaging plate 2, in order to improve an image quality of a composite image 400. In the above example, since the low-quality overlapping portion 321 is deleted when combining the 1-th radiation image 310 and the 2-th radiation image 320, the high-quality overlapping portion 311 is used to generate a relatively high-quality composite image 400. Note that "high quality" of the overlapping portion 311 means that the image quality is better (superior) compared to an overlapping portion 321. Also, "low quality" of the overlapping portion 321 means that the image quality is worse (inferior) compared to the overlapping portion 311. The same applies hereinafter.

On the other hand, when the stacking order information indicated by the holding member 5A indicates that an imaging plate 2 on a second side is stacked so as to be positioned above an imaging plate 2 on a first side, the image processing unit 281 performs registration of a 1-th radiation image 310 and a 2-th radiation image 320 such that the 2-th radiation image 320 read from the imaging plate 2 on the first side positioned below is positioned above the 1-th radiation image 310 read from the imaging plate 2 on the second side positioned above. Then, the image processing unit 281 leaves an overlapping portion 321 included in the upper 2-th radiation image 320, deletes an overlapping portion 311 included in the lower 1-th radiation image 310, and combines the 1-th radiation image 310 and the 2-th radiation image 320. In this case, since the image processing unit 281 generates the composite image 400 using the low-quality overlapping portion 321 instead of the high-quality overlapping portion 311, the image quality of the composite image 400 is relatively degraded.

In this way, when the image processing unit 281 does not grasp a stacking order of two imaging plates 2 held by the holding member 5A, there is a possibility that the 1-th radiation image 310 and the 2-th radiation image 320 are combined without being able to delete the one of the overlapping portions 311 and 321 that should originally be deleted.

Therefore, in this example, the plurality of marks 10 of a holding member 5 include at least one stacking order identification mark (also referred to as a fourth identification mark) for identifying a stacking order of a plurality of imaging plates 2 on the holding member 5 based on a mark image. During registration, an image processing unit 281 performs a stacking order identification process to identify the stacking order of N imaging plates 2, based on a mark image 350 corresponding to the at least one stacking order identification mark. This allows the image processing unit 281 to easily and accurately identify the stacking order of the N imaging plates 2. Then, the image processing unit 281 performs registration of the N read radiation images based on the identified stacking order and the mark images 350 included in the N read radiation images. This allows the image processing unit 281 to easily and appropriately perform the registration of the N read radiation images. As a result, for example, a high-quality composite image 400 can be generated.

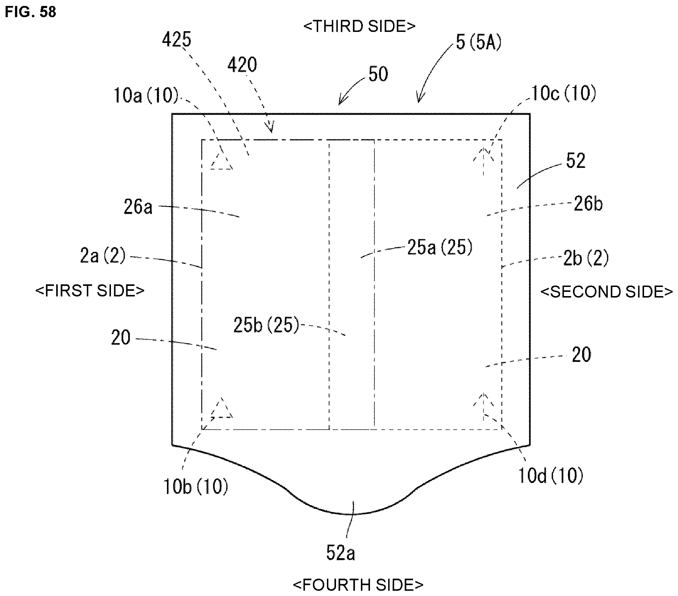

FIG. 58 is a schematic diagram illustrating an example of a holding member 5A. The example in FIG. 58 is a modification of the example in FIG. 16 described above, in which the shape of marks 10a and 10b is changed to an equilateral triangle, and the shape of marks 10c and 10d is changed to an arrow. The marks 10a, 10b, 10c, and 10d are used as the stacking order identification marks 10. In the example of FIG. 58, the stacking order of the two imaging plates 2 is identified from a difference in the shape of the mark images 350 corresponding to the stacking order identification marks 10 between two read radiation images read from the two imaging plates 2 held by the holding member 5A.

Further, in the example of FIG. 58, stacking order information indicated by the holding member 5A indicates that the imaging plates 2 are to be stacked such that an imaging plate 2 on a first side is positioned above an imaging plate 2 on a second side. Therefore, in the holding member 5A, the imaging plate 2 on the first side is positioned above the imaging plate 2 on the second side.

The marks 10a, 10b, 10c, and 10d are configured such that the stacking order of the two imaging plates 2 on the holding member 5A is identified from the shape of the corresponding mark images 350. Triangular marks 10a and 10b for recording a mark image 350 on the imaging plate 2 on the first side are for recording a mark image on the upper imaging plate 2 of the two imaging plates 2 held by the holding member 5A. Therefore, a read radiation image read from the upper imaging plate 2 includes triangular mark images 350a and 350b corresponding to the marks 10a and 10b. On the other hand, arrow-shaped marks 10c and 10d for recording a mark image 350 on the imaging plate 2 on the second side are for recording a mark image on the lower imaging plate 2 of the two imaging plates 2 held by the holding member 5A. Therefore, a read radiation image read from the lower imaging plate 2 includes arrow-shaped mark images 350c and 350d corresponding to the marks 10c and 10d.

Note that when the stacking order information indicated by the holding member 5A indicates that the imaging plates 2 are to be stacked such that the imaging plate 2 on the second side is positioned above the imaging plate 2 on the first side, the shape of the marks 10a and 10b is an arrow, and the shape of the marks 10c and 10d is an equilateral triangle.

The image processing unit 281 identifies the stacking order of the two imaging plates 2 held by the holding member 5A based on the shapes of the mark images 350a, 350b, 350c, and 350d corresponding to the marks 10a, 10b, 10c, and 10d. Specifically, when a read radiation image read from a certain imaging plate 2 includes a triangular mark image 350, the image processing unit 281 identifies that certain imaging plate 2 as the upper imaging plate 2 on the holding member 5A. Then, the image processing unit 281 defines the read radiation image read from that certain imaging plate 2 as a 1-th radiation image 310 read from the upper imaging plate 2.

On the other hand, when a read radiation image read from a certain imaging plate 2 includes an arrow-shaped mark image 350, the image processing unit 281 identifies that certain imaging plate 2 as the lower imaging plate 2 on the holding member 5A. Then, the image processing unit 281 defines the read radiation image read from that certain imaging plate 2 as a 2-th radiation image 320 read from the lower imaging plate 2.

After identifying the stacking order of the two imaging plates 2 on the holding member 5A, the image processing unit 281 performs registration of the 1-th radiation image 310 and the 2-th radiation image 320 based on a first layout diagram 460 and the mark images 350a, 350b, 350c, and 350d, as in the first example. At this time, the image processing unit 281 arranges the 1-th radiation image 310 above the 2-th radiation image 320. Thereafter, the image processing unit 281 leaves a high-quality overlapping portion 311 included in the upper 1-th radiation image 310, deletes a low-quality overlapping portion 321 included in the lower 2-th radiation image 320, and combines the 1-th radiation image 310 and the 2-th radiation image 320. Thereby, the overlapping portion 321 that is the original target for deletion is reliably deleted, and the high-quality composite image 400 is obtained.

Note that in the above example, the overlapping portion to be deleted out of the overlapping portions 311 and 321 was the overlapping portion 321, but it may be the overlapping portion 311. In other words, ideally, the image processing unit 281 may delete the overlapping portion 311 included in the 1-th radiation image 310 read from the upper imaging plate 2, and combine the 1-th radiation image 310 and the 2-th radiation image 320 while leaving the overlapping portion 321 included in the 2-th radiation image 320 read from the lower imaging plate 2. In this case, after identifying the stacking order of the two imaging plates 2 on the holding member 5A, the image processing unit 281 arranges the 1-th radiation image 310 above the 2-th radiation image 320, deletes the overlapping portion 311 of the upper 1-th radiation image 310, leaves the overlapping portion 321 of the lower 2-th radiation image 320, and combines the 1-th radiation image 310 and the 2-th radiation image 320.

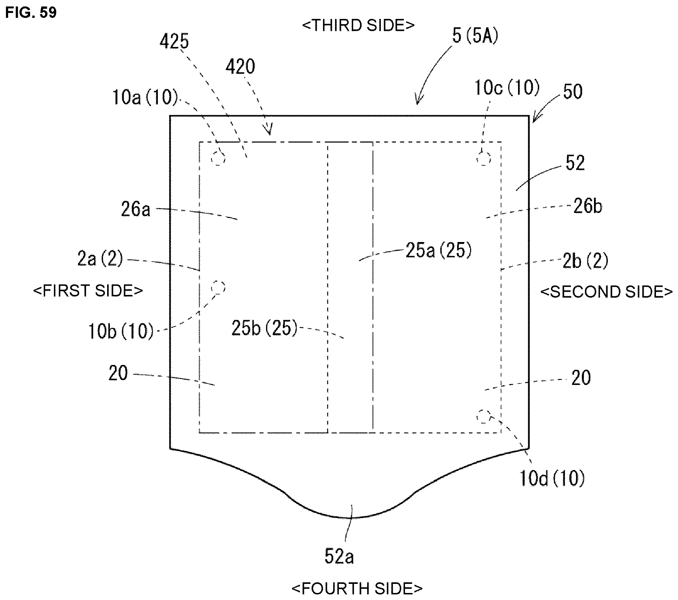

The image processing unit 281 may identify the stacking order of the N imaging plates 2 based on the position of the mark images 350 corresponding to the stacking order identification marks included in the N read radiation images. FIG. 59 is a schematic diagram illustrating an example of the holding member 5A in this case.

The example of FIG. 59 is a modification of the example of FIG. 16 described above, in which the position of the mark 10b has been changed. In the example of FIG. 59, unlike the example of FIG. 17 described above, a mark image 350b corresponding to the mark 10b is recorded at a central portion in a longitudinal direction of an imaging plate 2a, at an end portion on a first side of the imaging plate 2a. The marks 10a, 10b, 10c, and 10d are used as stacking order identification marks. In the example of FIG. 59, the stacking order of the two imaging plates 2 is identified based on a difference in the position of the mark images 350 corresponding to the stacking order identification marks between the two read radiation images read from the two imaging plates 2. The marks 10a, 10b, 10c, and 10d are configured such that the stacking order of the two imaging plates 2 on the holding member 5A is identified from the position of the corresponding mark images 350.

In the example of FIG. 59, when a read radiation image read from a certain imaging plate 2 includes a mark image 350 at its longitudinal end and includes a mark image 350 at its longitudinal center, the image processing unit 281 identifies that certain imaging plate 2 as the upper imaging plate 2 on the holding member 5A. Then, the image processing unit 281 defines the read radiation image read from that certain imaging plate 2 as the 1-th radiation image 310. On the other hand, when a read radiation image read from a certain imaging plate 2 includes a mark image 350 at one longitudinal end and includes a mark image 350 at the other longitudinal end, the image processing unit 281 identifies that certain imaging plate 2 as the lower imaging plate 2 on the holding member 5A. Then, the image processing unit 281 defines the read radiation image read from that certain imaging plate 2 as the 2-th radiation image 320.

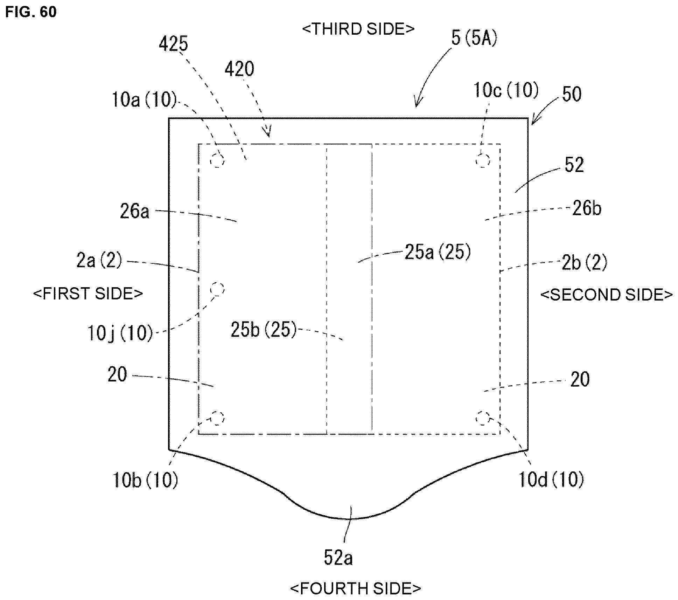

The image processing unit 281 may identify the stacking order of the N imaging plates 2 based on the number of mark images 350 corresponding to the stacking order identification marks included in the N read radiation images. FIG. 60 is a schematic diagram illustrating an example of the holding member 5A in this case.

The example of FIG. 60 is a modification of the example of FIG. 16 described above, in which a mark 10j is added to the holding member 5A. In the example of FIG. 60, a mark image 350 corresponding to the mark 10j is recorded at a central portion in a longitudinal direction of an imaging plate 2a, at an end portion on a first side of the imaging plate 2a. The marks 10a, 10b, 10c, 10d, and 10j are used as stacking order identification marks. In the example of FIG. 60, the stacking order of the two imaging plates 2 is identified based on a difference in the number of the mark images 350 corresponding to the stacking order identification marks between two read radiation images read from the two imaging plates 2 held by the holding member 5A. The marks 10a, 10b, 10c, 10d, and 10j are configured such that the stacking order of the two imaging plates 2 on the holding member 5A is identified from the number of the corresponding mark images 350.

In the example of FIG. 60, when a read radiation image read from a certain imaging plate 2 includes three mark images 350, the image processing unit 281 identifies that certain imaging plate 2 as the upper imaging plate 2 on the holding member 5A. Then, the image processing unit 281 defines the read radiation image read from that certain imaging plate 2 as the 1-th radiation image 310. On the other hand, when a read radiation image read from a certain imaging plate 2 includes two mark images 350, the image processing unit 281 identifies that certain imaging plate 2 as the lower imaging plate 2 on the holding member 5A. Then, the image processing unit 281 defines the read radiation image read from that certain imaging plate 2 as the 2-th radiation image 320.

The image processing unit 281 may identify the stacking order of the N imaging plates 2 based on the shape and position of the mark images 350 corresponding to the stacking order identification marks included in the N read radiation images. For example, a case in the example of FIG. 59 where the shape of the marks 10a and 10b is changed to a triangle, and the shape of the marks 10c and 10d is changed to an arrow is considered. In this case, when a read radiation image read from a certain imaging plate 2 includes a triangular mark image 350 at its longitudinal end and includes a triangular mark image 350 at its longitudinal center, the image processing unit 281 identifies that certain imaging plate 2 as the upper imaging plate 2 on the holding member 5A. On the other hand, when a read radiation image read from a certain imaging plate 2 includes an arrow-shaped mark image 350 at one longitudinal end and includes an arrow-shaped mark image 350 at the other longitudinal end, the image processing unit 281 identifies that certain imaging plate 2 as the lower imaging plate 2 on the holding member 5A.

The image processing unit 281 may identify the stacking order of the N imaging plates 2 based on the shape and number of the mark images 350 corresponding to the stacking order identification marks included in the N read radiation images. For example, a case in the example of FIG. 60 where the shape of the marks 10a, 10b, and 10j is changed to a triangle, and the shape of the marks 10c and 10d is changed to an arrow is considered. In this case, when a read radiation image read from a certain imaging plate 2 includes three triangular mark images 350, the image processing unit 281 identifies that certain imaging plate 2 as the upper imaging plate 2 on the holding member 5A. On the other hand, when a read radiation image read from a certain imaging plate 2 includes two arrow-shaped mark images 350, the image processing unit 281 identifies that certain imaging plate 2 as the lower imaging plate 2 on the holding member 5A.

The image processing unit 281 may identify the stacking order of the N imaging plates 2 based on the position and number of the mark images 350 corresponding to the stacking order identification marks included in the N read radiation images. For example, in the example of FIG. 60, when a read radiation image read from a certain imaging plate 2 includes three mark images 350, and the three mark images 350 consist of a mark image 350 located at one longitudinal end of the read radiation image, a mark image 350 located at the other longitudinal end of the read radiation image, and a mark image 350 located at the central end in the longitudinal direction of the read radiation image, that certain imaging plate 2 is identified as the upper imaging plate 2 on the holding member 5A. On the other hand, when a read radiation image read from a certain imaging plate 2 includes two mark images 350, and the two mark images 350 consist of a mark image 350 located at one longitudinal end of the read radiation image and a mark image 350 located at the other longitudinal end of the read radiation image, the image processing unit 281 identifies that certain imaging plate 2 as the lower imaging plate 2 on the holding member 5A.

The image processing unit 281 may identify the stacking order of the N imaging plates 2 based on the shape, position, and number of the mark images 350 corresponding to the stacking order identification marks included in the N read radiation images. For example, a case in the example of FIG. 60 where the shape of the marks 10a, 10b, and 10j is changed to a triangle, and the shape of the marks 10c and 10d is changed to an arrow is considered. In this case, when a read radiation image read from a certain imaging plate 2 includes three triangular mark images 350, and the three triangular mark images 350 consist of a triangular mark image 350 located at one longitudinal end of the read radiation image, a triangular mark image 350 located at the other longitudinal end of the read radiation image, and a triangular mark image 350 located at the central end in the longitudinal direction of the read radiation image, the image processing unit 281 identifies that certain imaging plate 2 as the upper imaging plate 2 on the holding member 5A. On the other hand, when a read radiation image read from a certain imaging plate 2 includes two arrow-shaped mark images 350, and the two arrow-shaped mark images 350 consist of an arrow-shaped mark image 350 located at one longitudinal end of the read radiation image and an arrow-shaped mark image 350 located at the other longitudinal end of the read radiation image, the image processing unit 281 identifies that certain imaging plate 2 as the lower imaging plate 2 on the holding member 5A.

Although the description above has focused on the case where N=2, even if N≥3, the image processing unit 281 can similarly identify the stacking order of the two imaging plates 2 from which the two read radiation images are read, for each of (N-1) pairs of radiation images to be combined, based on the mark images 350 corresponding to the stacking order identification marks 10 included in the two read radiation images constituting one pair of radiation images to be combined. In other words, the image processing unit 281 can similarly identify the stacking order of the N imaging plates 2 based on the mark images 350 corresponding to the stacking order identification marks 10 included in the N read radiation images.

Further, as in a third example, a fourth example, and a sixth example, when the holding member 5 includes a non-overlapping portion mark 10, by setting the non-overlapping portion mark 10 as a stacking order identification mark, the image processing unit 281 can similarly identify the stacking order of the N imaging plates 2 on the holding member 5 based on the mark image 350 corresponding to the stacking order identification mark.

### <Tenth Example (Identification of Stacking Order)>

Unlike the examples above, when the holding member 5A does not indicate stacking order information, the imaging plate 2 on the first side may be placed on top, or the imaging plate 2 on the second side may be placed on top in the holding member 5A. In such a case, there is a possibility that the low-quality overlapping portion 321 included in the 2-th radiation image 320 may be left, and the high-quality overlapping portion 311 included in the 1-th radiation image 310 may be deleted, resulting in the combination of the 1-th radiation image 310 and the 2-th radiation image 320. Furthermore, even when the holding member 5A indicates the stacking order information, there is a possibility that the two imaging plates 2 are stacked in the wrong order on the holding member 5A.

Therefore, in this example, the image processing unit 281 identifies the stacking order of the two imaging plates 2 based on a difference in intensity of the mark images 350 corresponding to an overlapping portion mark 10 between two read radiation images read from two adjacent imaging plates 2 in the holding member 5A.

In this example, as in a second example, a third example, a fifth example, and a seventh example, when the holding member 5 includes the overlapping portion mark 10, the image processing unit 281 can identify the stacking order of the two imaging plates 2 based on the intensity of the mark images 350 corresponding to the overlapping portion mark 10, which are included in two read radiation images read from the two imaging plates 2 held by the holding member 5A.

Here, an overlapping portion of the lower imaging plate 2 with the upper imaging plate 2 is irradiated with radiation that has passed through the upper imaging plate 2. Therefore, a difference arises between the intensity of the mark image 350 corresponding to the overlapping portion mark 10 read from the lower imaging plate 2 and the intensity of the mark image 350 corresponding to the overlapping portion mark 10 read from the upper imaging plate 2. Here, for example, it is assumed that the intensity of the mark image 350 corresponding to the overlapping portion mark 10 read from the upper imaging plate 2 is greater than the intensity of the mark image 350 corresponding to the overlapping portion mark 10 read from the lower imaging plate 2.

The image processing unit 281 compares the intensity (also referred to as a first intensity) of the mark image 350 corresponding to the overlapping portion mark 10, which is included in a 1-th read radiation image read from the imaging plate 2 on the first side, with the intensity (a second intensity) of the mark image 350 corresponding to the overlapping portion mark 10, which is included in a 2-th read radiation image read from the imaging plate 2 on the second side. Then, when the first intensity is greater than the second intensity, the image processing unit 281 determines that the imaging plate 2 on the first side is positioned above the imaging plate 2 on the second side. On the other hand, when the second intensity is greater than the first intensity, the image processing unit 281 determines that the imaging plate 2 on the second side is positioned above the imaging plate 2 on the first side. The processing after the image processing unit 281 identifies the stacking order of the two imaging plates 2 is the same as in a ninth example.

As described above, the overlapping portion of the lower imaging plate 2 with the upper imaging plate 2 is irradiated with radiation that has passed through the upper imaging plate 2. For this reason, this overlapping portion is irradiated with radiation after it has been scattered by the upper imaging plate 2. Furthermore, a distance from a radiation source to the lower imaging plate 2 is greater than a distance from the source to the upper imaging plate 2. For this reason, the size of the mark image 350 corresponding to the overlapping portion mark 10 read from the lower imaging plate 2 becomes larger than the size of the mark image 350 corresponding to the overlapping portion mark 10 read from the upper imaging plate 2.

Therefore, the image processing unit 281 may identify the stacking order of the imaging plate 2 on the first side and the imaging plate 2 on the second side based on a difference between a size (also referred to as a first size) of the mark image 350 corresponding to the overlapping portion mark 10 included in the first read radiation image read from the imaging plate 2 on the first side and a size (a second size) of the mark image 350 corresponding to the overlapping portion mark 10 included in the second read radiation image read from the imaging plate 2 on the second side. For example, when the first size is smaller than the second size, the image processing unit 281 determines that the imaging plate 2 on the first side is positioned above the imaging plate 2 on the second side. On the other hand, when the second size is smaller than the first size, the image processing unit 281 determines that the imaging plate 2 on the second side is positioned above the imaging plate 2 on the first side.

Even if N≥3, the image processing unit 281 can similarly identify the stacking order of the two imaging plates 2 from which the two read radiation images are read, for each of (N-1) pairs of radiation images to be combined, based on the intensity or size of the mark images 350 corresponding to the overlapping portion mark 10 included in the two read radiation images that constitute one pair of radiation images to be combined. In other words, even if N≥3, the image processing unit 281 can similarly identify the stacking order of the N imaging plates 2 on the holding member 5.

### <Identification of Anterior Side or Posterior Side of Composite Image>

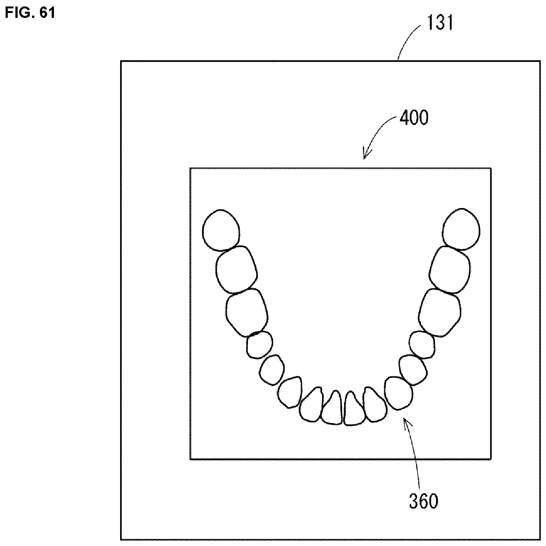

FIG. 61 is a schematic diagram illustrating a display example of the composite image 400 on a display unit 130. As in the example of FIG. 61, a display control unit 282 may, for example, cause the composite image 400 to be displayed on a display surface 131 of the display unit 130 with an anterior side facing downward. In other words, the display control unit 282 may cause the composite image 400 to be displayed on the display surface 131 with a posterior side facing upward.

Thus, when a display orientation of the composite image 400 (in other words, the display orientation) is automatically determined or set by default based on the anterior side or the posterior side of the composite image 400, it is necessary to identify the anterior side or the posterior side of the composite image 400.

Therefore, in this example, the plurality of marks 10 included in the holding member 5 include at least one identification mark (also referred to as a mark for identifying the anterior side/posterior side or a second identification mark) for identifying the anterior side or the posterior side of the composite image 400 based on a mark image. The image processing unit 281 identifies the anterior side or the posterior side of the composite image 400 based on the mark image 350 corresponding to the at least one mark for identifying the anterior side/posterior side. The display control unit 282 causes the composite image 400 to be displayed on the display unit 130 in a predetermined orientation (in other words, a predetermined direction), for example as shown in FIG. 61, based on the anterior side or the posterior side of the composite image 400 identified by the image processing unit 281. Note that the display orientation of the composite image 400 is not limited to this. Further, a display orientation of a composite image 400 showing a maxillary dental arch and a display orientation of a composite image 400 showing a mandibular dental arch may be different from each other.

In this example, for instance, when the holding member 5A shown in FIG. 49 described above is used in a first example, the anterior side or the posterior side of the composite image 400 can be identified using the marks 10 included in the holding member 5A. The image processing unit 281 uses the marks 10a, 10b, 10c, and 10d included in the holding member 5A shown in FIG. 49 as the marks 10 for identifying the anterior side/posterior side.

The marks 10a, 10b, 10c, and 10d are configured such that the anterior side or the posterior side of the composite image 400 is identified from an orientation of the corresponding mark images 350. When the holding member 5A shown in FIG. 49 is placed in the oral cavity, one vertex of each of the marks 10a, 10b, 10c, and 10d points toward the posterior side. The image processing unit 281 identifies the anterior side or the posterior side of the composite image 400 based on the orientation of the mark images 350a, 350b, 350c, and 350d corresponding to the marks 10a, 10b, 10c, and 10d. This allows the image processing unit 281 to easily and reliably identify the anterior side or the posterior side of the composite image 400.

Here, for convenience of explanation, a direction along the longitudinal direction of the 1-th radiation image 310 and the 2-th radiation image 320 included in the composite image 400 is referred to as a vertical direction of the composite image 400. When one vertex of each of the mark images 350a, 350b, 350c, and 350d included in the composite image 400 points toward one end side in the vertical direction of the composite image 400, the image processing unit 281 determines that this one end side is the posterior side. Alternatively, when one vertex of each of the mark images 350a, 350b, 350c, and 350d included in the composite image 400 points toward one end side in the longitudinal direction of the composite image 400, the image processing unit 281 determines that the other end side in the longitudinal direction of the composite image 400 is the anterior side. The display control unit 282 determines the display orientation of the composite image 400 based on the anterior side or the posterior side of the composite image 400 identified by the image processing unit 281. This allows the display control unit 282 to cause the display unit 130 to display the composite image 400 in a display orientation based on the anterior side or the posterior side of the composite image 400. For example, as shown in FIG. 61, the display control unit 282 causes the display unit 130 to display the composite image 400 such that the posterior side of the composite image 400 identified by the image processing unit 281 is positioned on the upper side, or the anterior side of the composite image 400 identified by the image processing unit 281 is positioned on the lower side. Note that the display control unit 282 may cause the display unit 130 to display the composite image 400 such that the posterior side of the composite image 400 identified by the image processing unit 281 is positioned on the lower side, or the anterior side of the composite image 400 identified by the image processing unit 281 is positioned on the upper side.

Even when the holding member 5A shown in FIGS. 51 to 54 is used, the image processing unit 281 can similarly identify the anterior side or the posterior side of the composite image 400. In this case, a triangular mark 10 included in the holding member 5A shown in FIGS. 51 to 54 is used as the mark 10 for identifying the anterior side/posterior side, and the image processing unit 281 can similarly identify the anterior side or the posterior side of the composite image 400 based on an orientation of a triangular mark image 350 corresponding to the mark 10 for identifying the anterior side/posterior side.

The image processing unit 281 may identify the anterior side or the posterior side of the composite image 400 based on a position of the mark image 350 corresponding to the at least one mark for identifying the anterior side/posterior side. For example, a case where the holding member 5A shown in FIG. 55 is used is considered. In this case, the marks 10a, 10b, 10c, and 10d included in the holding member 5A shown in FIG. 55 are used as the marks for identifying the anterior side/posterior side. The marks 10a, 10b, 10c, and 10d are configured such that the anterior side or the posterior side of the composite image 400 is identified from the position of the corresponding mark images 350. When two of four mark images 350 included in the composite image 400 are located at one end side in the vertical direction of the composite image 400, and the remaining two mark images 350 are located at a central portion in the vertical direction of the composite image 400, the image processing unit 281 identifies that one end side as the posterior side, or the other end side in the longitudinal direction of the composite image 400 as the anterior side.

Even when the holding member 5A shown in FIG. 56 is used, the image processing unit 281 can identify the anterior side or the posterior side of the composite image 400 based on the positions of mark images 350a and 350b corresponding to the marks 10a and 10b included in the holding member 5A. When two triangular mark images 350 included in the composite image 400 are located on one side in the vertical direction of the composite image 400, the image processing unit 281 identifies that one side as the posterior side, or the other end side in the longitudinal direction of the composite image 400 as the anterior side.

Further, when the holding member 5A shown in FIG. 56 is used, the image processing unit 281 may identify the anterior side or the posterior side of the composite image 400 based on the orientation and position of the mark images 350a and 350b corresponding to the marks 10a and 10b included in the holding member 5A. In this case, when the two triangular mark images 350 included in the composite image 400 are located on one side in the vertical direction of the composite image 400, and one vertex of each of the two mark images 350 points to that one side in the vertical direction of the composite image 400, the image processing unit 281 identifies that one side as the posterior side, or the other end side in the longitudinal direction of the composite image 400 as the anterior side.

### <Identification of Orientation of Plurality of Imaging Plates with Respect to Holding Member>

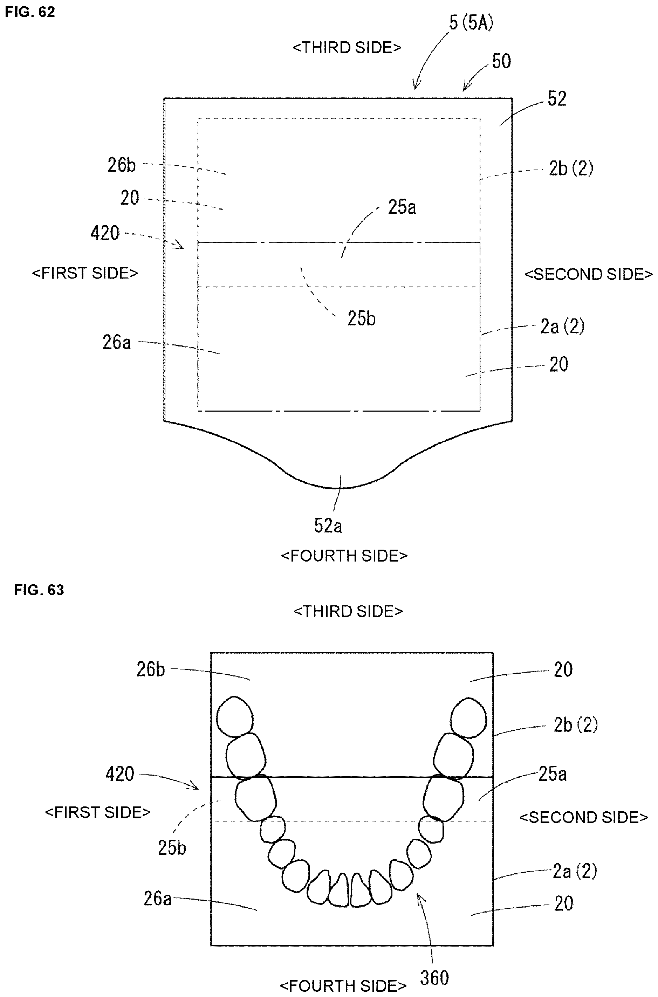

Unlike the above example, when the holding member 5A does not show placement location information 51c shown in FIG. 1, etc., as shown in FIG. 62, an IP assembly 1 may be used for intra-oral radiography with a plurality of imaging plates 2 arranged in the holding member 5A such that their longitudinal direction is aligned with a horizontal direction of the holding member 5A. In other words, in a holding member 5A that does not show the placement location information 51c, the plurality of imaging plates 2 may be arranged such that their longitudinal direction is aligned with a vertical direction of the holding member 5A, or the plurality of imaging plates 2 may be arranged such that their longitudinal direction is aligned with a horizontal direction of the holding member 5A. Hereinafter, such a holding member 5A may be referred to as a hybrid holding member 5A. In the example of FIG. 62, an upper imaging plate 2a is arranged on a fourth side of the holding member 5A, and a lower imaging plate 2b is arranged on a third side of the holding member 5A. Note that illustration of the marks 10 is omitted in FIG. 62.

Further, as shown in FIG. 16, etc., a manner of placing the plurality of imaging plates 2 with respect to the holding member 5A such that the longitudinal direction of each imaging plate 2 is aligned with the vertical direction of the holding member 5A is referred to as "vertical placement" for convenience of explanation. Further, as shown in FIG. 62, a manner of placing the plurality of imaging plates 2 with respect to the holding member 5A such that the longitudinal direction of each imaging plate 2 is aligned with the horizontal direction of the holding member 5A is referred to as "horizontal placement" for convenience of explanation.

In the holding member 5A shown in FIG. 1, etc., the placement location information 51c indicates that the imaging plate 2 is to be vertical placement with respect to the surface to be fixed 51b of the holding member 5A. Therefore, the holding member 5A shown in FIG. 1, etc., can be said to be a holding member 5A exclusively for vertical placement of the imaging plate 2. Hereinafter, the holding member 5A exclusively for vertical placement of the imaging plate 2 may be referred to as a vertical placement-dedicated holding member 5A.

In addition to the hybrid holding member 5A and the vertical placement-dedicated holding member 5A, a holding member 5A exclusively for horizontal placement of the imaging plate 2 (also referred to as a horizontal placement-dedicated holding member 5A) may also be used. Placement location information 51c indicated by the horizontal placement-dedicated holding member 5A indicates that the imaging plate 2 is to be horizontal placement with respect to the surface to be fixed 51b of the holding member 5A. When an operator assembling the imaging plate assembly 1 places a plurality of unit assemblies 7 on the surface to be fixed 51b of the horizontal placement-dedicated holding member 5A in accordance with the placement location information 51c indicated by the horizontal placement-dedicated holding member 5A, the plurality of imaging plates 2 are placed horizontally. When the vertical placement-dedicated holding member 5A and the horizontal placement-dedicated holding member 5A are prepared, a radiography technician uses either the vertical placement-dedicated holding member 5A or the horizontal placement-dedicated holding member 5A depending on a subject of radiography.

Thus, when the hybrid holding member 5A is used in intra-oral radiography, the imaging plate 2 may be placed vertically or horizontally in the holding member 5A. Further, even when the vertical placement-dedicated holding member 5A and the horizontal placement-dedicated holding member 5A are prepared, the imaging plate 2 may be placed vertically or horizontally in the holding member 5A.

FIG. 63 is a diagram schematically illustrating an example of a radiation image recorded on a combined imaging plate 420 composed of an imaging plate 2a and an imaging plate 2b placed horizontally with respect to the holding member 5A as shown in FIG. 62. In FIG. 63, illustration of the mark image 350 is omitted.

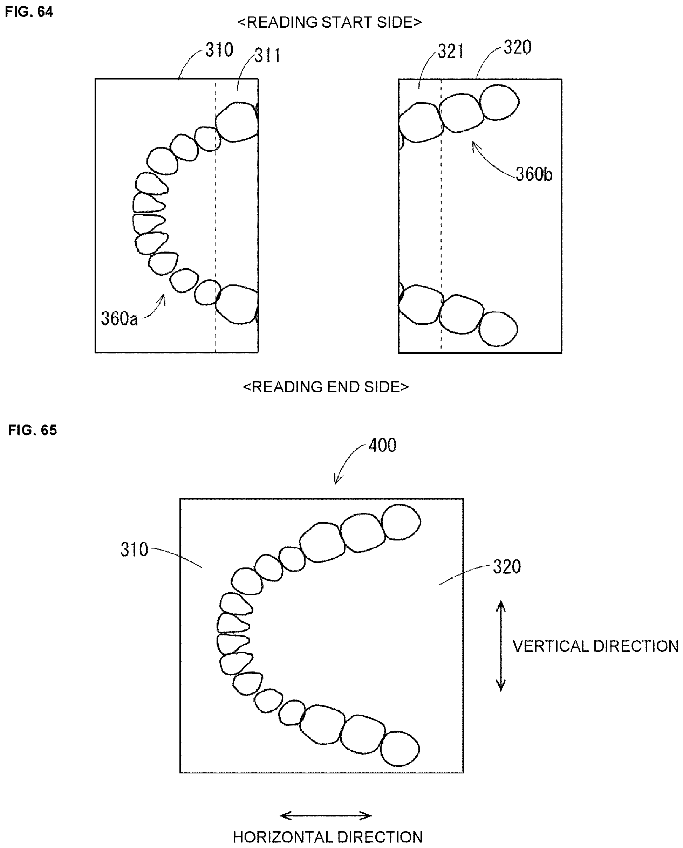

FIG. 64 is a diagram schematically illustrating an example of the 1-th radiation image 310 and the 2-th radiation image 320 read from the imaging plate 2a and the imaging plate 2b shown in FIG. 63. In FIG. 64, the 1-th radiation image 310 is shown such that a reading start side in a reading device 100 for the 1-th radiation image 310 is on an upper side of the figure. The same applies to the 2-th radiation image 320.

In this example, the imaging plate 2 placed horizontally in the holding member 5A is inserted into an insertion slot 121 of the reading device 100 from a short side on a first side when being held by the holding member 5A, for example. Then, the reading device 100 reads the radiation image from the short side on the first side of the imaging plate 2 when it is held by the holding member 5A. Therefore, in the 1-th radiation image 310, the side where an image read from the end portion on the first side of the imaging plate 2a is located becomes the reading start side in the reading device 100 for the 1-th radiation image 310. Similarly, in the 2-th radiation image 320, the side where an image read from the end portion on the first side of the imaging plate 2b is located becomes the reading start side in the reading device 100 for the 2-th radiation image 320. Note that the imaging plate 2 placed horizontally in the holding member 5A may be inserted into the insertion slot 121 of the reading device 100 from a short side on a second side when being held by the holding member 5A.

Even when N imaging plates 2 are placed horizontally in the holding member 5, the image processing unit 281 can perform registration of N read radiation images read from the N imaging plates 2 based on the mark image 350 corresponding to at least one mark 10 included in the holding member 5. Then, the image processing unit 281 combines the N read radiation images after registration to generate the composite image 400.

FIG. 65 is a schematic diagram illustrating an example of a composite image 400 obtained by performing registration on the 1-th radiation image 310 and the 2-th radiation image 320 shown in FIG. 64 in the same manner as described above, and combining the 1-th radiation image 310 and the 2-th radiation image 320 after the registration.

Here, a case where the imaging plate 2 is placed vertically in the holding member 5Ais considered. In this case, in the composite image 400, as shown in FIG. 22, etc., a dental arch appears to be convex toward one side in the vertical direction of the composite image 400. Therefore, when the display unit 130 displays the composite image 400 on the display surface 131 such that the vertical direction of the composite image 400 is aligned with, for example, the up-down direction of the display surface 131, a convex direction of the dental arch appearing in the composite image 400 displayed on the display surface 131 is a direction along the up and down direction of the display surface 131.

Next, a case where the imaging plate 2 is placed horizontally in the holding member 5A is considered. In this case, in the composite image 400, as shown in FIG. 65, the dental arch appears to be convex toward one side in a up and down direction of the composite image 400. Therefore, when the display unit 130 displays the composite image 400 on the display surface 131 such that the vertical direction of the composite image 400 is aligned with the up and down direction of the display surface 131, the convex direction of the dental arch appearing in the composite image 400 displayed on the display surface 131 is a direction along the left and right direction of the display surface 131.

Thus, the direction that the convex direction of the dental arch appearing in the composite image 400 displayed on the display surface 131 follows may differ between when the imaging plate 2 is placed vertically and when it is placed horizontally.

Therefore, in this example, the plurality of marks 10 included in the holding member 5 include at least one identification mark (also referred to as a placement orientation identification mark or a third identification mark) 10 for identifying an orientation of N imaging plates 2 with respect to the holding member 5, in other words, a placement orientation of the N imaging plates 2 on the holding member 5, based on the mark image 350. The placement orientation identification mark 10 is a mark 10 for identifying whether the placement orientation of the imaging plate 2 on the holding member 5 is vertical or horizontal based on the mark image 350. The image processing unit 281 identifies whether the placement orientation of the imaging plate 2 on the holding member 5 is vertical or horizontal based on the mark image 350 corresponding to the at least one placement orientation identification mark 10. This allows the image processing unit 281 to easily and accurately identify the placement orientation of the imaging plate 2 on the holding member 5. The display control unit 282 determines the display orientation of the composite image 400 according to the placement orientation identified by the image processing unit 281. This automatically sets the display orientation of the composite image 400. For example, it becomes possible to automatically match the direction that the convex direction of the dental arch appearing in the composite image 400 displayed on the display surface 131 follows, between when the imaging plate 2 is placed vertically and when it is placed horizontally. Therefore, a user of the reading device 100 can more easily check the composite image 400 displayed on the display unit 130.

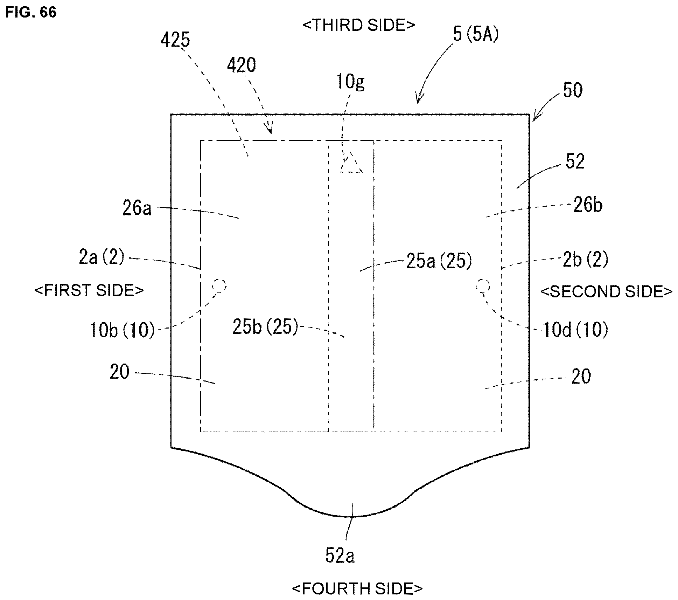

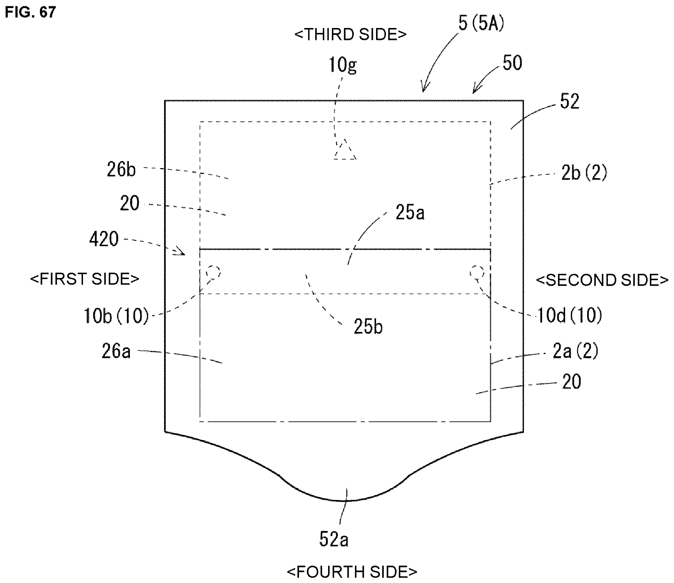

FIGS. 66 and 67 are schematic diagrams illustrating an example of the holding member 5A of this example. In FIG. 66, the imaging plate 2 is placed vertically in the holding member 5A, and in FIG. 67, the imaging plate 2 is placed horizontally in the holding member 5A. The holding member 5A shown in FIGS. 66 and 67 may be a hybrid holding member 5A. Alternatively, the holding member 5A shown in FIG. 66 may be a vertical placement-dedicated holding member 5A, and the holding member 5A shown in FIG. 67 may be a horizontal placement-dedicated holding member 5A.

The holding member 5A shown in FIGS. 66 and 67 is a modification of the holding member 5A shown in FIG. 33 described above, in which the shape of a mark 10g is changed to an equilateral triangle, and the positions of the marks 10b and 10d are changed. When the imaging plate 2 is placed vertically, the marks 10b and 10d become non-overlapping portion marks 10. On the other hand, when the imaging plate 2 is placed horizontally, the marks 10b and 10d become overlapping portion marks 10. In the examples of FIGS. 66 and 67, the mark 10g is used as the placement orientation identification mark 10.

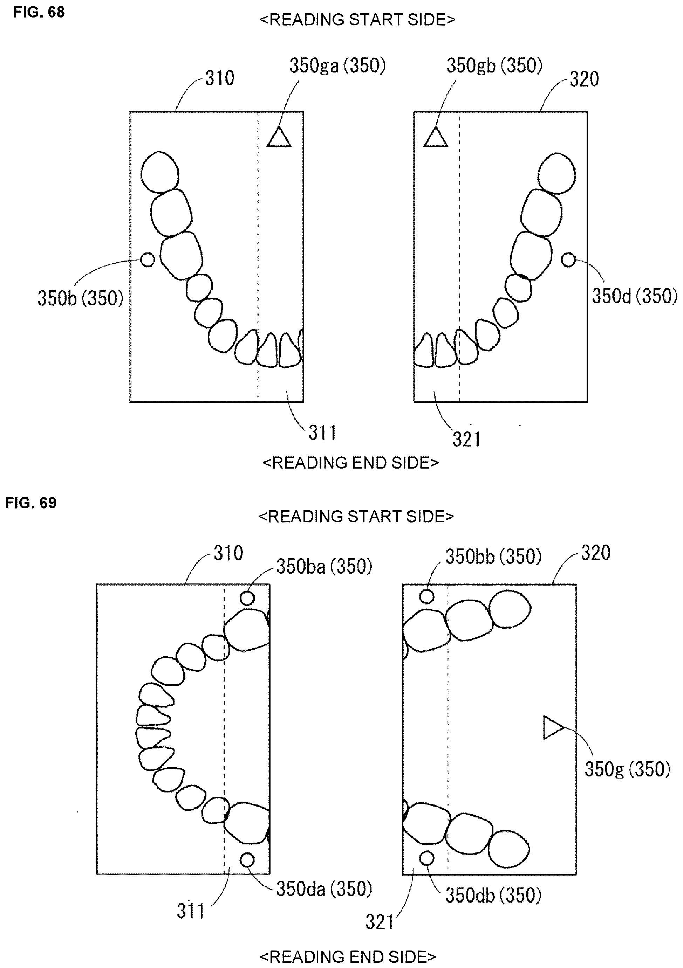

FIG. 68 is a schematic diagram illustrating an example of the 1-th radiation image 310 and the 2-th radiation image 320 read from the imaging plates 2a and 2b shown in FIG. 66. FIG. 69 is a schematic diagram illustrating an example of the 1-th radiation image 310 and the 2-th radiation image 320 read from the imaging plates 2a and 2b shown in FIG. 67.

When the imaging plate 2 is placed vertically, as shown in FIG. 68, the 1-th radiation image 310 includes a triangular mark image 350ga corresponding to the mark 10g and a circular mark image 350b corresponding to the mark 10b. The 2-th radiation image 320 includes a triangular mark image 350gb corresponding to the mark 10g and a circular mark image 350d corresponding to the mark 10d.

On the other hand, when the imaging plate 2 is placed horizontally, as shown in FIG. 69, the 1-th radiation image 310 includes a circular mark image 350ba corresponding to the mark 10b and a circular mark image 350da corresponding to the mark 10d. The 2-th radiation image 320 includes a circular mark image 350bb corresponding to the mark 10b, a circular mark image 350db corresponding to the mark 10d, and a triangular mark image 350gb corresponding to the mark 10g.

The mark 10g is configured such that the orientation of the imaging plates 2a and 2b with respect to the holding member 5A is identified from the shape of the corresponding mark image 350. The image processing unit 281 identifies whether the placement orientation of the imaging plate 2 is vertical or horizontal based on the shape of the mark image 350 corresponding to the mark 10g. Specifically, when each of the 1-th radiation image 310 and the 2-th radiation image 320 includes a triangular mark image 350, the image processing unit 281 determines that the placement orientation of the imaging plate 2 is vertical. On the other hand, when only the second radiation image 320 of the 1-th radiation image 310 and the 2-th radiation image 320 includes a triangular mark image 350, the image processing unit 281 determines that the placement orientation of the imaging plate 2 is horizontal.

When the image processing unit 281 determines that the placement orientation of the imaging plate 2 is vertical, it performs registration of the 1-th radiation image 310 and the 2-th radiation image 320 based on the mark images 350b, 350d, 350ga, and 350gb, in the same manner as in the third example described above, and then combines the 1-th radiation image 310 and the 2-th radiation image 320 after registration to generate the composite image 400.

On the other hand, when the image processing unit 281 determines that the placement orientation of the imaging plate 2 is horizontal, it performs registration of the 1-th radiation image 310 and the 2-th radiation image 320 based on the mark images 350ba, 350da, 350bb, and 350db, which correspond to the marks 10b and 10d that are overlapping portion marks 10, in the same manner as in the second example described above, and then combines the 1-th radiation image 310 and the 2-th radiation image 320 after registration to generate the composite image 400.

When the image processing unit 281 determines that the placement orientation of the imaging plate 2 is vertical, the display control unit 282 displays the composite image 400 on the display surface 131 such that the vertical direction of the composite image 400 generated by the image processing unit 281 is aligned with the up and down direction of the display surface 131, for example. This causes the convex direction of the dental arch appearing in the composite image 400 displayed on the display surface 131 to be aligned with the up and down direction of the display surface 131.

On the other hand, when the image processing unit 281 determines that the placement orientation of the imaging plate 2 is horizontal, the display control unit 282 displays the composite image 400 on the display surface 131 such that the horizontal direction of the composite image 400 generated by the image processing unit 281 is aligned with the up and down direction of the display surface 131, for example. This causes the convex direction of the dental arch appearing in the composite image 400 displayed on the display surface 131 to be aligned with the up and down direction of the display surface 131, similar to the case where the placement orientation of the imaging plate 2 is vertical.

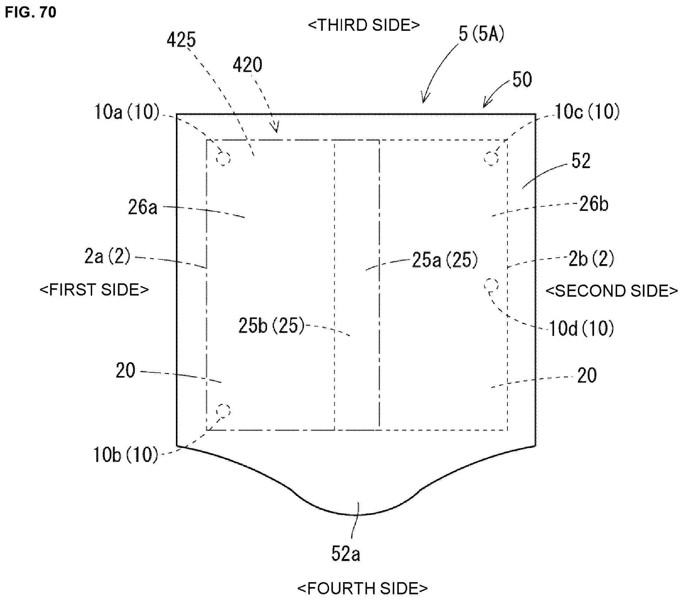

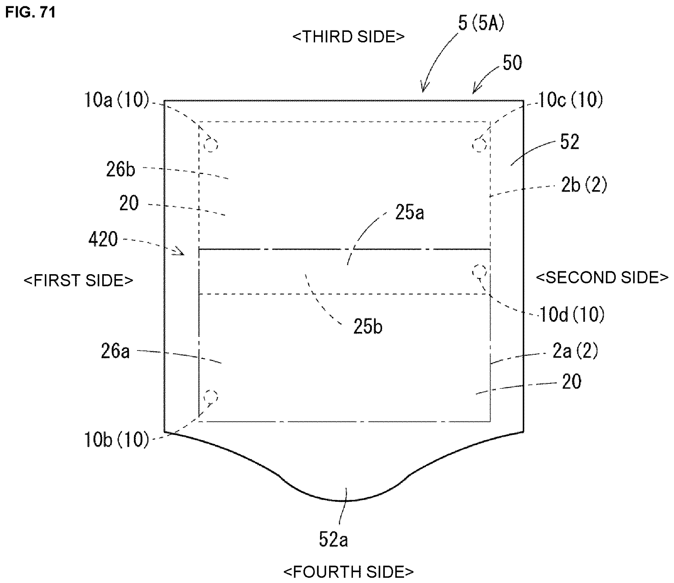

Note that the image processing unit 281 may identify whether the placement orientation of the imaging plate 2 is vertical or horizontal based on the position of the placement orientation identification mark 10. FIGS. 70 and 71 are schematic diagrams illustrating an example of the holding member 5A in this case. In FIG. 70, the imaging plate 2 is placed vertically in the holding member 5A, and in FIG. 71, the imaging plate 2 is placed horizontally in the holding member 5A.

The holding member 5A of FIGS. 70 and 71 is a modification of the holding member 5A shown in FIG. 16 described above, in which the position of the mark 10d is changed. When the imaging plate 2 is placed vertically, the mark 10d becomes a non-overlapping portion mark 10. On the other hand, when the imaging plate 2 is placed horizontally, the mark 10d becomes an overlapping portion mark 10. In the examples of FIGS. 70 and 71, the mark 10d is used as the placement orientation identification mark 10.

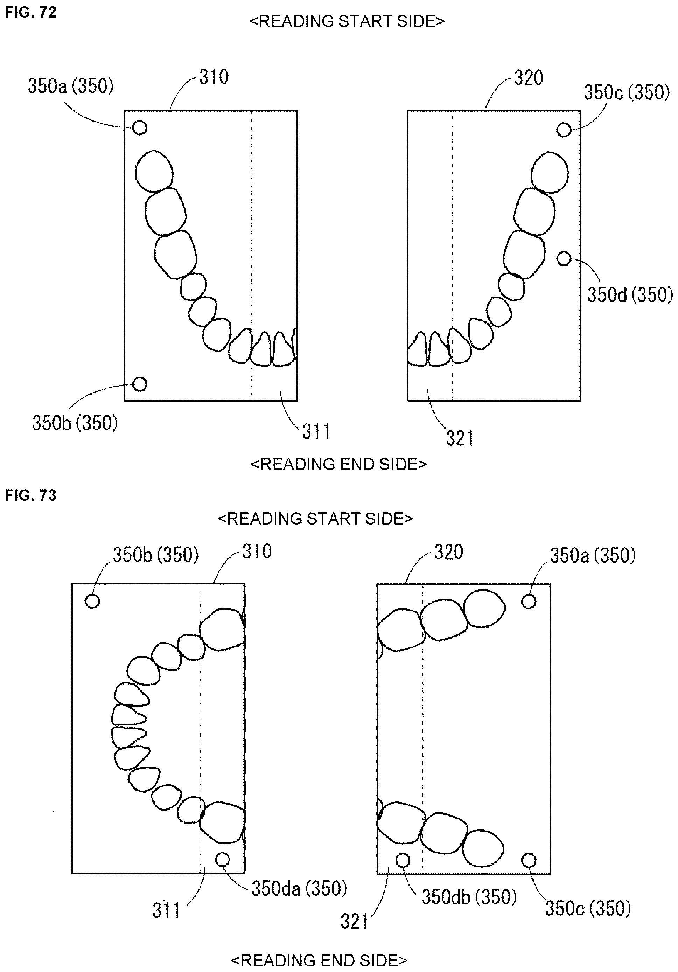

FIG. 72 is a schematic diagram illustrating an example of the 1-th radiation image 310 and the 2-th radiation image 320 read from the imaging plates 2a and 2b shown in FIG. 70. FIG. 73 is a schematic diagram illustrating an example of the 1-th radiation image 310 and the 2-th radiation image 320 read from the imaging plates 2a and 2b shown in FIG. 71.

When the imaging plate 2 is placed vertically, as shown in FIG. 72, the 1-th radiation image 310 includes mark images 350a and 350b corresponding to the marks 10a and 10b. The mark image 350a is located at one longitudinal end of the 1-th radiation image 310, and the mark image 350b is located at the other longitudinal end of the 1-th radiation image 310. The 2-th radiation image 320 includes mark images 350c and 350d corresponding to the marks 10c and 10d. The mark image 350c is located at one longitudinal end of the 2-th radiation image 320, and the mark image 350d is located at a central portion in the longitudinal direction of the 2-th radiation image 320.

On the other hand, when the imaging plate 2 is placed horizontally, as shown in FIG. 73, the 1-th radiation image 310 includes a mark image 350b corresponding to the mark 10b and a mark image 350da corresponding to the mark 10d. The mark image 350b is located at one longitudinal end of the 1-th radiation image 310, and the mark image 350da is located at the other longitudinal end of the 1-th radiation image 310. The second radiation image 320 includes a mark image 350ab corresponding to the mark 10a, a mark image 350c corresponding to the mark 10c, and a mark image 350db corresponding to the mark 10d. The mark image 350a is located at one longitudinal end of the 2-th radiation image 320, and the mark images 350c and 350db are located at one end in the longitudinal direction of the 2-th radiation image 320.

The mark 10d is configured such that the orientation of the imaging plates 2a and 2b with respect to the holding member 5A is identified from the position of the corresponding mark image 350. The image processing unit 281 identifies whether the placement orientation of the imaging plate 2 is vertical or horizontal based on the position of the mark image 350 corresponding to the mark 10d. Specifically, when no mark image 350 is present in the central portion in the longitudinal direction of the 1-th radiation image 310 and a mark image 350 is present in the central portion in the longitudinal direction of the 2-th radiation image 320, the image processing unit 281 determines that the placement orientation of the imaging plate 2 is vertical. On the other hand, when no mark image 350 is present in the central portion in the longitudinal direction of the 1-th radiation image 310 and no mark image 350 is present in the central portion in the longitudinal direction of the 2-th radiation image 320, the image processing unit 281 determines that the placement orientation of the imaging plate 2 is horizontal.

When the image processing unit 281 determines that the placement orientation of the imaging plate 2 is vertical, it performs registration of the 1-th radiation image 310 and the 2-th radiation image 320 based on the mark images 350a, 350b, 350c, and 350d in the same manner as in the first example described above, and then combines the 1-th radiation image 310 and the 2-th radiation image 320 after registration to generate the composite image 400.

On the other hand, when the image processing unit 281 determines that the placement orientation of the imaging plate 2 is horizontal, it performs registration of the 1-th radiation image 310 and the 2-th radiation image 320 based on the mark images 350a, 350b, 350da, and 350db in the same manner as in the third example described above, and then combines the 1-th radiation image 310 and the 2-th radiation image 320 after registration to generate the composite image 400. The operation of the display control unit 282 is the same as in the case of FIGS. 66 to 69.

The image processing unit 281 may identify whether the placement orientation of the imaging plate 2 is vertical or horizontal based on the number of mark images 350 corresponding to the placement orientation identification mark. For example, a case where the holding member 5A shown in FIGS. 70 and 71 is used is considered. In this case, the marks 10a, 10b, 10c, and 10d are configured such that the orientation of the imaging plates 2a and 2b with respect to the holding member 5A is identified from the number of the corresponding mark images 350. When the number of mark images 350 included in the first radiation image 310 and the number of mark images 350 included in the second radiation image 320 are both two, the image processing unit 281 determines that the placement orientation of the imaging plate 2 is vertical. On the other hand, when the number of mark images 350 included in the first radiation image 310 is two and the number of mark images 350 included in the second radiation image 320 is three, the image processing unit 281 determines that the placement orientation of the imaging plate 2 is horizontal.

Note that when the holding member 5A shown in FIGS. 70 and 71 is used, it is possible for the image processing unit 281 to identify whether the placement orientation of the imaging plate 2 is vertical or horizontal based on the position of the mark image 350 corresponding to the placement orientation identification mark, and it is possible to identify whether the placement orientation of the imaging plate 2 is vertical or horizontal based on the number of mark images 350 corresponding to the placement orientation identification mark. Therefore, it is also possible for the image processing unit 281 to identify whether the placement orientation of the imaging plate 2 is vertical or horizontal based on both the position and number of the mark images 350 corresponding to the placement orientation identification mark, by using in combination the identification based on the position of the mark image 350 corresponding to the placement orientation identification mark and the identification based on the number of mark images 350 corresponding to the placement orientation identification mark.

Further, in the holding member 5A shown in FIGS. 70 and 71, if the shape of the mark 10d is changed to a triangle, when the placement orientation of the imaging plate 2 is vertical, only the 2-th radiation image 320 of the 1-th radiation image 310 and the 2-th radiation image 320 includes a triangular mark image 350, and when the placement orientation of the imaging plate 2 is horizontal, each of the 1-th radiation image 310 and the 2-th radiation image 320 includes a triangular mark image 350. Therefore, in the holding member 5A shown in FIGS. 70 and 71, if the shape of the mark 10d is changed to a triangle, it is also possible for the image processing unit 281 to identify whether the placement orientation of the imaging plate 2 is vertical or horizontal based on the position and shape of the mark image 350 corresponding to the placement orientation identification mark, and it is also possible to identify whether the placement orientation of the imaging plate 2 is vertical or horizontal based on the number and shape of the mark image 350 corresponding to the placement orientation identification mark, and it is also possible to identify whether the placement orientation of the imaging plate 2 is vertical or horizontal based on the position, number, and shape of the mark image 350 corresponding to the placement orientation identification mark.

As can be understood from the above description, the image processing unit 281 can use the same mark 10 provided in the holding member 5 as at least two of a reading direction identification mark, a stacking order identification mark, a mark for identifying the anterior side/posterior side, and a placement orientation identification mark. For example, the marks 10a, 10b, 10c, and 10d shown in FIG. 58 can be used not only as stacking order identification marks, but also as reading direction identification marks and marks for identifying the anterior side/posterior side. Further, the mark 10g shown in FIGS. 66 and 67 can be used not only as a placement orientation identification mark, but also as a reading direction identification mark and a mark for identifying the anterior side/posterior side.

Further, when the image processing unit 281 identifies the anterior side or the posterior side of the composite image 400 based on the mark image 350 corresponding to the mark for identifying the anterior side/posterior side, the display control unit 282 can cause the display unit 130 to display the composite image 400 in a desired orientation, regardless of whether the placement orientation of the imaging plate 2 is vertical or horizontal. Therefore, when identifying the anterior side or the posterior side of the composite image 400, the image processing unit 281 does not need to identify whether the placement orientation of the imaging plate 2 is vertical or horizontal.

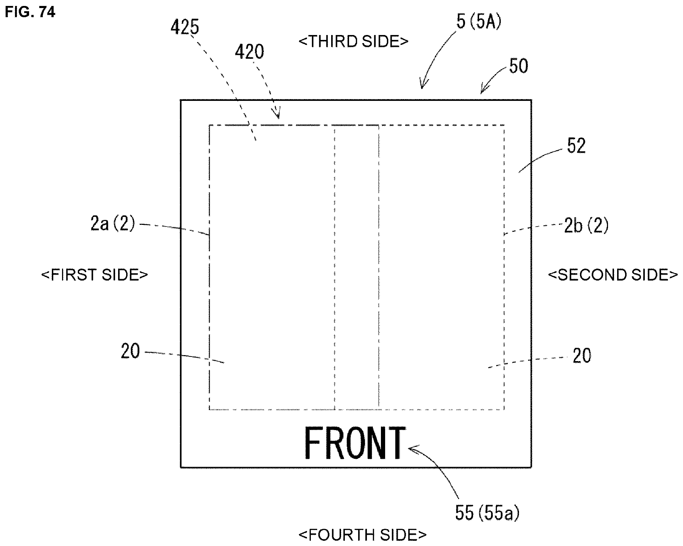

In the above example, an insertion orientation of the holding member 5 into the oral cavity was indicated by a pair of tab portions 51a and 52a of the holding member 5, but it may be indicated by other means. For example, as shown in FIG. 74, instead of the pair of tab portions 51a and 52a, insertion orientation information 55 indicating the insertion orientation of the holding member 5 into the oral cavity may be shown on the holding member 5. The insertion orientation information 55 is shown, for example, on an outer surface of a cover portion 52. In FIG. 74, vertically placed imaging plates 2a and 2b are shown. In FIG. 74, illustration of the marks 10 is omitted.

In the example of FIG. 74, the insertion orientation information 55 is represented by a character string 55a "FRONT". The character string 55a is shown at an end portion on a fourth side of the outer surface of the cover portion 52. The holding member 5A is inserted into the oral cavity such that the character string 55a is positioned at a front side of the oral cavity. Since the character string 55a determines the insertion orientation of the holding member 5 into the oral cavity, it can be said that the character string 55a indicates the insertion orientation of the holding member 5A into the oral cavity. The radiography technician can identify from which direction to insert the holding member 5A into the oral cavity from the character string 55a.

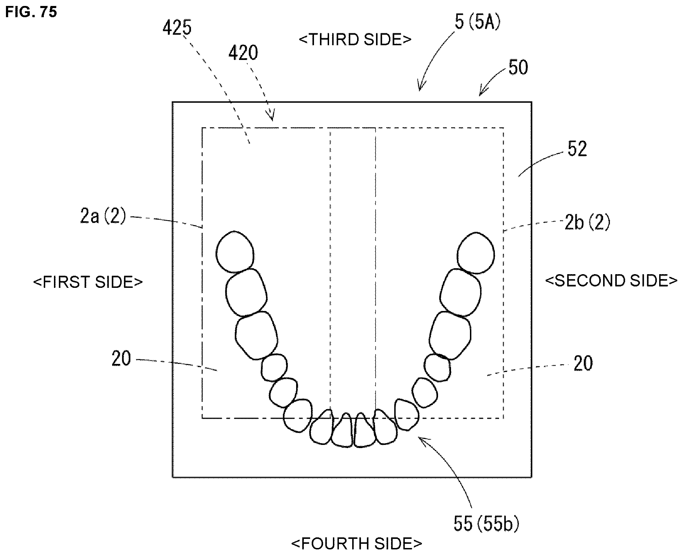

The insertion orientation information 55 is not limited to the above example. For example, as shown in FIG. 75, the insertion orientation information 55 may be a diagram 55b representing a dental arch. In the example of FIG. 75, the holding member 5 is inserted into the oral cavity such that an orientation of the dental arch indicated by the diagram 55b matches an orientation of the dental arch of the radiography subject in the oral cavity. The radiography technician can identify from which direction to insert the holding member 5A into the oral cavity from the diagram 55b. In FIG. 75, illustration of the marks 10 is omitted.

Note that at least one of the holding member 5 shown in FIG. 74 and the holding member 5 shown in FIG. 75 may include the pair of tab portions 51a and 52a. Further, the holding member 5 shown in FIG. 74 may show the diagram 55b shown in FIG. 75 as the insertion orientation information 55, in addition to the character string 55a. In this case, a material of the diagram 55b showing the insertion orientation information 55 is not captured in a radiation image.

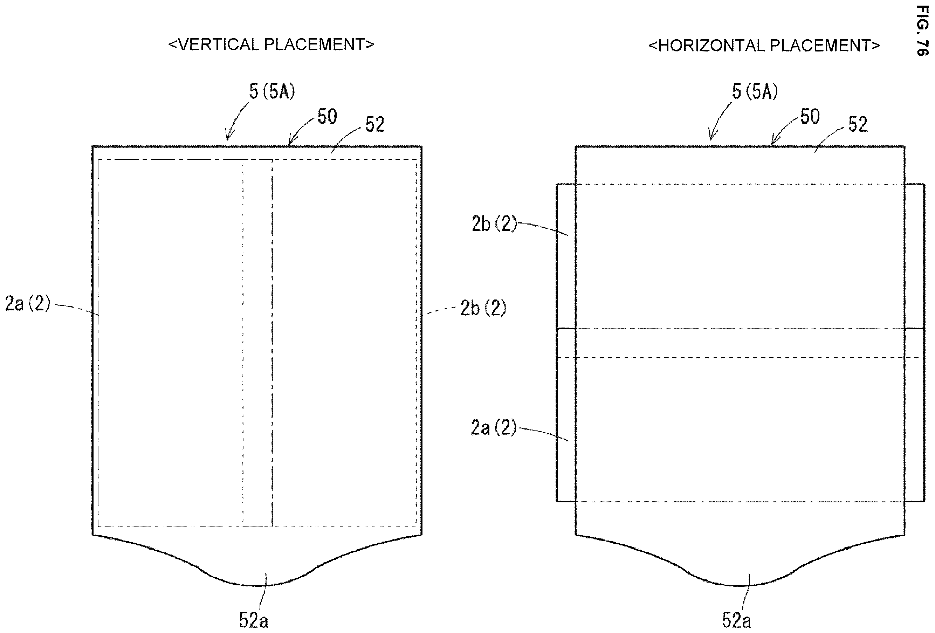

In the holding member 5 of the above example, an orientation that N imaging plates 2 should take with respect to the holding member 5 is indicated by a dotted line 51ca of the placement location information 51c, but it may be indicated by other means. For example, the orientation that the N imaging plates 2 should take with respect to the holding member 5 may be indicated by a shape of the holding member 5. FIG. 76 is a schematic diagram illustrating an example of the holding member 5 in this case. In FIG. 76, illustration of the marks 10 is omitted.

On a left side of FIG. 76, the holding member 5 holding the vertically placed imaging plates 2 is shown. On a right side of FIG. 76, the holding member 5 holding horizontally placed imaging plates 2 is shown.

In the example of FIG. 76, the holding member 5 indicates by its own shape that the N imaging plates 2 should be placed vertically in the holding member 5. In the example of FIG. 76, the shape of the holding member 5 is set such that when the imaging plates 2a and 2b are placed vertically, the imaging plates 2a and 2b do not protrude from the holding member 5 (see the left side of FIG. 76), and when the imaging plates 2a and 2b are placed horizontally, the imaging plates 2a and 2b protrude from the holding member 5 (see the right side of FIG. 76). An operator assembling the IP assembly 1 can identify from the shape of the holding member 5 that the orientation the N imaging plates 2 should take with respect to the holding member 5 is vertical placement.

Note that regardless of whether the plurality of imaging plates 2 are placed vertically or horizontally in the holding member 5, when the holding member 5 is placed in the oral cavity, a length of the holding member 5 in a front-rear direction of the oral cavity may be larger than, the same as, or smaller than a length of the holding member 5 in a left and right direction of the oral cavity.

A configuration of the holding member 5 is not limited to the above example. The holding member 5 may be, for example, a case type that can be opened and closed. For example, the holding member 5 may include a box-shaped resin body portion with an open upper surface, and a resin lid portion connected to the body portion and capable of covering the opening of the body portion, the lid portion may be fitted to the body portion so as to be openable and closable. In this case, an inner surface of the lid portion may be provided with a plurality of pressing protrusions that come into contact with each unit assembly 7 on the body portion when the lid portion is fitted to the body portion. Thereby, when the lid portion is closed and fitted to the body portion, the plurality of pressing protrusions come into contact with each unit assembly 7, and a position of each unit assembly 7 within the holding member 5 is fixed. This allows the holding member 5 to hold the plurality of unit assemblies 7 at predetermined positions. On an inner surface of the body portion, placement location information indicating placement locations of the plurality of unit assemblies 7 may be shown.

FIGS. 77 and 78 are schematic diagrams illustrating other configuration examples of the holding member 5. A holding member 5 (also referred to as a holding member 5B) shown in FIGS. 77 and 78 can hold a plurality of unit assemblies 7 at predetermined positions by sandwiching the plurality of unit assemblies 7. The holding member 5B can hold, for example, two unit assemblies 7 at predetermined positions. FIG. 77 shows the holding member 5B before holding a unit assembly 7. FIG. 78 shows the holding member 5B holding two unit assemblies 7.

The holding member 5B is made of a radiation-transmissive material. The holding member 5B is made of paper, for example. As shown in FIGS. 77 and 78, the holding member 5B has, for example, a substantially box shape with a very small thickness. The holding member 5B has an opening 500 on its end surface (also referred to as a side surface) through which the unit assembly 7 is inserted and removed. The opening 500 is connected to an internal space of the holding member 5A.

The holding member 5B has main wall portions 501 and 502 facing each other. The main wall portions 501 and 502 function as a pair of a first sandwiching portion 501 and a second sandwiching portion 502, respectively, capable of sandwiching the unit assembly 7. For example, each unit assembly 7 is inserted from the opening 500 into the internal space of the holding member 5B such that a non-reading surface 22 and a reading surface 21 of the imaging plate 2 face the first sandwiching portion 501 and the second sandwiching portion 502 of the holding member 5B, respectively. Each unit assembly 7 is inserted into the holding member 5A from a bottom side of a waterproof bag 4. The plurality of unit assemblies 7 inserted into the holding member 5B are sandwiched by the first sandwiching portion 501 and the second sandwiching portion 502. A large part of the unit assembly 7 is accommodated in the internal space of the holding member 5B. Note that all of the unit assembly 7 may be accommodated in the internal space.

The two unit assemblies 7 are held by the holding member 5B such that one end portion 25 in a short-side direction of the imaging plate 2 of one unit assembly 7 and one end portion 25 in the short-side direction of the imaging plate 2 of the other unit assembly 7 overlap each other in a thickness direction of the holding member 5B, similar to the case of being held by the holding member 5A described above.

During intra-oral radiography, radiation is irradiated onto the holding member 5B from, for example, the second sandwiching portion 502 side. The registration mark 10 is provided on the second sandwiching portion 502. The mark 10 may be provided on an outer surface of the second sandwiching portion 502, as in the example of FIGS. 77 and 78, or may be provided on an inner surface of the second sandwiching portion 502.

Note that the holding member 5B may be made of a material other than paper. For example, the holding member 5B may be made of a resin such as PVC or EVA. Further, the holding member 5B may be made of paper and a resin. In this case, for example, the holding member 5B may be configured by coating a base material (also referred to as a main body) made of paper with a resin such as vinyl. Furthermore, the internal space of the holding member 5B may have a plurality of partial spaces in which a plurality of unit assemblies 7 are respectively arranged and partitioned from each other.

The imaging plate protection assembly 3 may further comprise a covering bag 8 that covers the entirety of the holding member 5. FIG. 79 is a schematic diagram illustrating an example of a state in which the entirety of the holding member 5 holding a plurality of unit assemblies 7 is covered by the covering bag 8.

The covering bag 8 has, for example, a configuration similar to that of the waterproof bag 4. The holding member 5 is inserted into a main body portion 80 of the covering bag 8 through an opening of the main body portion 80. Then, after the holding member 5 is inserted in the main body portion 80, a lid portion 83 is attached to an adhesive member 88 provided on the main body portion 80. Thereby, the main body portion 80 is sealed. The covering bag 8 can also be referred to as a covering member that covers the imaging plate 2.

The covering bag 8 is made of, for example, a radiation-transmissive material. The adhesive member 88 may be a double-sided tape or an applied adhesive. The main body portion 80 may be made of, for example, a resin such as PVC or EVA. The material of the main body portion 80 may be the same as or different from the material of a main body portion 40 of the waterproof bag 4. When the main body portion 80 is made of a resin, it is possible to make it less likely for a subject to feel discomfort when the IP assembly 1 is placed in the oral cavity. In addition, since the covering bag 8 covers the entirety of the holding member 5, bodily fluids such as saliva are less likely to adhere to the holding member 5 and the like accommodated inside the covering bag 8. This makes it possible to improve the ease of handling when reusing the holding member 5 and the like inside the covering bag 8.

Note that, instead of the adhesive member 88, for example, a curable adhesive may be used. Alternatively, the main body portion 80 may be sealed by welding the folded lid portion 83. Further, similar to the main body portion 40 of the waterproof bag 4, at least a part of the main body portion 80 may have a light-shielding property against visible light. In this case, for example, since visible light or external light emitted from surrounding lighting devices is less likely to strike the imaging plate 2, information of the radiation image is less likely to be lost from the imaging plate 2. Furthermore, the configuration of the covering bag 8 may be different from the configuration of the waterproof bag 4.

When the IP assembly 1 includes the covering bag 8 that covers the holding member 5, it may not include the waterproof bag 4 that covers the imaging plate 2. In this case, the holding member 5 holds the imaging plate 2 that is not covered by the waterproof bag 4.

In the IP assembly 1 of the above example, the registration marks 10 were provided on the holding member 5, but the member on which the registration marks 10 are provided is not limited to the holding member 5. For example, the registration marks 10 may be provided on the covering bag 8 as a covering member that covers the N imaging plates 2. In this case, the IP assembly 1 may or may not include the waterproof bag 4. Further, unlike the above example, when one waterproof bag 4 collectively covers the N imaging plates 2, the marks 10 may be provided on that one waterproof bag 4. Further, unlike the above example, when one protection cover 6 collectively covers the N imaging plates 2, the marks 10 may be provided on that one protection cover 6.

In the above example, a composite image 400 generated by an image processing unit 281 is displayed on a display unit 130 included in a reading apparatus 100, but it may be displayed on a display unit 800 as an external device connected to the reading apparatus 100.

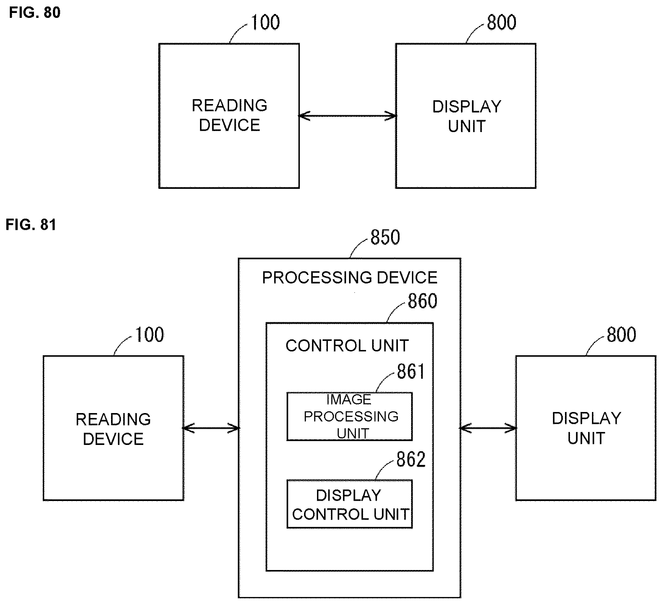

FIG. 80 is a schematic diagram illustrating an example of a state in which the reading device 100 and the display unit 800 are connected. The reading device 100 outputs an image signal indicating the composite image 400 generated by the image processing unit 281 to the display unit 800 through an interface unit 240. The display unit 800 displays the composite image 400 based on the image signal from the interface unit 240. The display unit 800 can also be referred to as a display device 800. The display unit 800 may be, for example, a liquid crystal display device or an organic EL display device. When the composite image 400 is displayed on the display unit 800, the display unit 130 of the reading device 100 may or may not display the composite image 400.

In the above example, the image processing unit 281 of the reading device 100 performs registration and combination of N read radiation images, but the registration and combination of the N read radiation images may be performed in a processing device 850 as an external device connected to the reading device 100.

FIG. 81 is a schematic diagram illustrating an example of a state in which the reading device 100 and the processing device 850 are connected. The processing device 850 is, for example, a computer device and includes a control unit 860. For example, the display unit 800 is connected to the processing device 850. The processing device 850 can control the display of the display unit 800.

The control unit 860 includes, for example, at least one processor and a storage unit, similar to a control unit 280 of the reading device 100. In the control unit 860, by the at least one processor executing a program in the storage unit, the control unit 860 includes, as functional blocks, for example, an image processing unit 861 and a display control unit 862.

The reading device 100 outputs N image signals, respectively indicating N read radiation images read from N imaging plates 2, to the processing device 850 through the interface unit 240. In the processing device 850, the image processing unit 861 performs registration of the N read radiation images, which are respectively indicated by the N image signals from the reading device 100, in the same manner as described above. Then, the image processing unit 861 combines the N radiation images after the registration to generate the composite image 400. The display control unit 862 controls the display unit 800 to cause the display unit 800 to display the composite image 400 generated by the image processing unit 861. Note that the display unit 800 may be included in the processing device 850.

Although the imaging plate assembly and a processing device such as a reading device have been described in detail as above, the above description is illustrative in all aspects, and this disclosure is not limited thereto. Further, the various examples described above can be applied in combination as long as they do not contradict each other. Then, it is to be understood that countless non-exemplified examples can be envisaged without departing from the scope of this disclosure.

### <Additional Notes>

The present specification and drawings disclose the following aspects.

A covering member according to a first aspect is a covering member that covers a plurality of imaging plates used for intra-oral radiography and is placed in an oral cavity together with the plurality of imaging plates, the covering member comprising at least one mark for registration when combining a plurality of radiation images respectively read from the plurality of imaging plates, wherein when the plurality of imaging plates are irradiated with radiation through the covering member, a mark image corresponding to the at least one mark is included in the plurality of radiation images.

According to the first aspect, registration upon combination of a plurality of radiation images can be performed easily, in a short time, accurately, and appropriately based on the mark images included in the plurality of radiation images. Therefore, it becomes possible to reliably generate an image of a subject with no clinical problems.

The covering member according to a second aspect is the covering member according to the first aspect, wherein the plurality of imaging plates include a first imaging plate and a second imaging plate that are covered by the covering member in a state of being partially overlapped, the first imaging plate has a first non-overlapping portion that does not overlap with the second imaging plate, the second imaging plate has a second non-overlapping portion that does not overlap with the first imaging plate, and the at least one mark includes at least one first non-overlapping portion mark for recording a mark image on the first non-overlapping portion, and at least one second non-overlapping portion mark for recording a mark image on the second non-overlapping portion.

The covering member according to a third aspect is the covering member according to the first aspect, wherein the plurality of imaging plates include a first imaging plate and a second imaging plate that are covered by the covering member in a state of being partially overlapped, the first imaging plate has a first overlapping portion that overlaps with the second imaging plate, the second imaging plate has a second overlapping portion that overlaps with the first imaging plate, and the at least one mark includes at least one overlapping portion mark for recording a mark image on the first overlapping portion and the second overlapping portion.

The covering member according to a fourth aspect is the covering member according to the second aspect, wherein the first imaging plate has a first overlapping portion that overlaps with the second imaging plate, the second imaging plate has a second overlapping portion that overlaps with the first imaging plate, and the at least one mark includes at least one overlapping portion mark for recording a mark image on the first overlapping portion and the second overlapping portion.

The covering member according to a fifth aspect is the covering member according to any one of the first to fourth aspects, wherein the at least one mark includes at least one edge mark for recording a mark image on an edge of a combined imaging plate composed of the plurality of imaging plates. In this case, the mark image is less likely to overlap with the subject image recorded on the combined imaging plate, and an appropriate subject image is more easily obtained.

The covering member according to a sixth aspect is the covering member according to the fifth aspect, wherein the at least one edge mark includes at least one corner mark for recording a mark image on at least one corner of the plurality of imaging plates. In this case, the mark image is less likely to overlap with the subject image recorded on the combined imaging plate, and an appropriate subject image is more easily obtained.

The covering member according to a seventh aspect is the covering member according to the fifth or sixth aspect, wherein the at least one mark is configured such that all mark images corresponding to the at least one mark are recorded on the edge. In this case, the mark image is less likely to overlap with the subject image recorded on the combined imaging plate, and an appropriate subject image is more easily obtained.

The covering member according to an eighth aspect is the covering member according to any one of the first to seventh aspects, wherein the at least one mark includes at least one first identification mark for identifying, based on a mark image, a reading direction of a radiation image from each of the plurality of imaging plates. In this case, the reading direction of the radiation image from each of the plurality of imaging plates can be easily and appropriately identified based on the mark image corresponding to the at least one first identification mark included in the plurality of radiation images read from the plurality of imaging plates.

The covering member according to a ninth aspect is the covering member according to the eighth aspect, wherein the at least one first identification mark is configured such that the reading direction is identified from at least one of an orientation and a position of the mark image corresponding to the at least one first identification mark.

The covering member according to a tenth aspect is the covering member according to any one of the first to ninth aspects, wherein the at least one mark includes at least one second identification mark for identifying, based on a mark image, an anterior side or a posterior side of a composite image obtained by combining the plurality of radiation images. In this case, the anterior side or the posterior side of the composite image obtained by combining the plurality of radiation images can be easily and appropriately identified based on the mark image corresponding to the at least one second identification mark included in the plurality of radiation images read from the plurality of imaging plates.

The covering member according to an eleventh aspect is the covering member according to the tenth aspect, wherein the at least one second identification mark is configured such that the anterior side or the posterior side of the composite image is identified from at least one of an orientation and a position of the mark image corresponding to the at least one second identification mark.

The covering member according to a twelfth aspect is the covering member according to the tenth or eleventh aspect, wherein the covering member indicates an insertion orientation of the covering member into the oral cavity. In this case, an operator can more easily insert the covering member into the oral cavity in an appropriate orientation.

The covering member according to a thirteenth aspect is the covering member according to any one of the first to twelfth aspects, wherein the at least one mark includes at least one third identification mark for identifying, based on a mark image, an orientation of the plurality of imaging plates with respect to the covering member. In this case, the orientation of the plurality of imaging plates with respect to the covering member can be easily and appropriately identified based on the mark image corresponding to the at least one third identification mark included in the plurality of radiation images read from the plurality of imaging plates.

The covering member according to a fourteenth aspect is the covering member according to the thirteenth aspect, wherein the at least one third identification mark is configured such that the orientation of the plurality of imaging plates with respect to the covering member is identified from at least one of a shape, a position, and a number of the mark image corresponding to the at least one third identification mark.

The covering member according to a fifteenth aspect is the covering member according to the thirteenth or fourteenth aspect, wherein the covering member indicates an orientation that the plurality of imaging plates should take with respect to the covering member. In this case, an operator can more easily set the orientation of the plurality of imaging plates with respect to the covering member to an appropriate orientation.

The covering member according to a sixteenth aspect is the covering member according to any one of the first to fifteenth aspects, wherein the plurality of imaging plates include a first imaging plate and a second imaging plate that are covered by the covering member in a state of being partially overlapped, and the at least one mark includes at least one fourth identification mark for identifying, based on a mark image, a stacking order of the first imaging plate and the second imaging plate. In this case, the stacking order of the first imaging plate and the second imaging plate can be easily and appropriately identified based on the mark image corresponding to the at least one fourth identification mark included in a first radiation image and a second radiation image read from the first imaging plate and the second imaging plate, respectively.

The covering member according to a seventeenth aspect is the covering member according to the sixteenth aspect, wherein the at least one fourth identification mark is configured such that the stacking order is identified from at least one of a shape, a position, and a number of the mark image corresponding to the at least one fourth identification mark.

The covering member according to an eighteenth aspect is the covering member according to the sixteenth or seventeenth aspect, wherein the covering member indicates a stacking order that the first imaging plate and the second imaging plate should take. In this case, an operator can more easily set the stacking order of the first imaging plate and the second imaging plate to an appropriate stacking order.

The covering member according to a nineteenth aspect is the covering member according to any one of the first to eighteenth aspects, wherein the covering member holds the first imaging plate and the second imaging plate at predetermined positions. In this case, the imaging plates can be held at appropriate positions during radiography.

An imaging plate assembly according to a twentieth aspect comprises the covering member according to any one of the first to nineteenth aspects, and the plurality of imaging plates covered by the covering member.

A processing device according to a twenty-first aspect comprises an image processing unit configured to perform registration of the plurality of radiation images read from the plurality of imaging plates covered by the covering member according to any one of the first to nineteenth aspects, based on a mark image included in the plurality of radiation images, and to combine the plurality of radiation images after the registration.

According to the twenty-first aspect, the image processing unit can easily and appropriately perform registration of a plurality of radiation images read from a plurality of imaging plates based on the mark images included in the plurality of radiation images.

The processing device according to a twenty-second aspect is the processing device according to the twenty-first aspect, wherein the plurality of imaging plates include a first imaging plate and a second imaging plate, the first imaging plate and the second imaging plate are covered by the covering member in a state of being partially overlapped such that the first imaging plate is positioned above the second imaging plate, the plurality of radiation images include a first radiation image and a second radiation image read from the first imaging plate and the second imaging plate, respectively, the second radiation image includes a boundary image corresponding to a boundary between the first imaging plate and the second imaging plate on the second imaging plate, and the image processing unit performs registration of the first radiation image and the second radiation image based on the boundary image and the mark images included in the first radiation image and the second radiation image. In this case, the image processing unit can easily and appropriately perform the registration of the first radiation image and the second radiation image based on the boundary image included in the second radiation image and the mark images included in the first radiation image and the second radiation image.

The processing device according to a twenty-third aspect comprises an image processing unit configured to perform registration of the plurality of radiation images read from the plurality of imaging plates covered by the covering member according to the eighth or ninth aspect, based on a mark image included in the plurality of radiation images, and to combine the plurality of radiation images after the registration, wherein the image processing unit identifies, based on the mark image corresponding to the at least one first identification mark included in the plurality of radiation images, a reading direction of the radiation image from each of the plurality of imaging plates, and performs the registration of the plurality of radiation images based on the identified reading direction and the mark images included in the plurality of radiation images.

According to the twenty-third aspect, the image processing unit can easily and appropriately perform the registration of the plurality of radiation images based on the reading direction of the radiation image from each of the plurality of imaging plates and the mark images included in the plurality of radiation images read from the plurality of imaging plates.

The processing device according to a twenty-fourth aspect comprises an image processing unit configured to perform registration of the plurality of radiation images read from the plurality of imaging plates covered by the covering member according to any one of the tenth to twelfth aspects, based on a mark image included in the plurality of radiation images, and to combine the plurality of radiation images after the registration, wherein the image processing unit identifies an anterior side or a posterior side of a composite image obtained by the combination of the plurality of radiation images, based on the mark image corresponding to the at least one second identification mark included in the plurality of radiation images.

According to the twenty-fourth aspect, the image processing unit can easily and appropriately perform registration of a plurality of radiation images read from a plurality of imaging plates based on the mark images included in the plurality of radiation images. Furthermore, the image processing unit can easily and accurately identify the anterior side or the posterior side of the composite image obtained by the combination of the plurality of radiation images, based on the mark image corresponding to the at least one second identification mark included in the plurality of radiation images.

The processing device according to a twenty-fifth aspect is the processing device according to the twenty-fourth aspect, further comprising a display control unit configured to cause a display unit to display the composite image, wherein the display control unit determines a display orientation of the composite image based on the anterior side or the posterior side of the composite image identified by the image processing unit. In this case, the composite image can be displayed on the display unit in a display orientation based on the anterior side or the posterior side of the composite image.

The processing device according to a twenty-sixth aspect comprises an image processing unit configured to perform registration of the plurality of radiation images read from the plurality of imaging plates covered by the covering member according to any one of the thirteenth to fifteenth aspects, based on a mark image included in the plurality of radiation images, and to combine the plurality of radiation images after the registration, wherein the image processing unit identifies an orientation of the plurality of imaging plates with respect to the covering member, based on the mark image corresponding to the at least one third identification mark included in the plurality of radiation images.

According to the twenty-sixth aspect, the image processing unit can easily and appropriately perform registration of a plurality of radiation images read from a plurality of imaging plates based on the mark images included in the plurality of radiation images. Furthermore, the image processing unit can easily and accurately identify the orientation of the plurality of imaging plates with respect to the covering member based on the mark image corresponding to the at least one third identification mark included in the plurality of radiation images.

The processing device according to a twenty-seventh aspect is the processing device according to the twenty-sixth aspect, further comprising a display control unit configured to cause a display unit to display a composite image obtained by the combination of the plurality of radiation images in the image processing unit, wherein the display control unit determines a display orientation of the composite image based on the orientation of the plurality of imaging plates with respect to the covering member, which is identified by the image processing unit. In this case, the display orientation of the composite image, which is obtained by combining the plurality of radiation images read from the plurality of imaging plates, can be automatically set according to the orientation of the plurality of imaging plates with respect to the covering member.

The processing device according to a twenty-eighth aspect comprises an image processing unit configured to perform registration of the plurality of radiation images read from the plurality of imaging plates covered by the covering member according to any one of the sixteenth to eighteenth aspects, based on a mark image included in the plurality of radiation images, and to combine the plurality of radiation images after the registration, wherein the plurality of radiation images include a first radiation image and a second radiation image read from the first imaging plate and the second imaging plate included in the plurality of imaging plates, respectively, and the image processing unit identifies a stacking order of the first imaging plate and the second imaging plate, based on the mark image corresponding to the at least one fourth identification mark included in the first radiation image and the second radiation image, and performs the registration of the first radiation image and the second radiation image based on the identified stacking order and the mark images included in the first radiation image and the second radiation image.

The processing device according to a twenty-ninth aspect comprises an image processing unit configured to perform registration of the plurality of radiation images read from the plurality of imaging plates covered by the covering member according to the third or fourth aspect, based on a mark image included in the plurality of radiation images, and to combine the plurality of radiation images after the registration, wherein the plurality of radiation images include a first radiation image and a second radiation image read from the first imaging plate and the second imaging plate included in the plurality of imaging plates, respectively, and the image processing unit identifies a stacking order of the first imaging plate and the second imaging plate, based on the mark image corresponding to the at least one overlapping portion mark included in the first radiation image and the second radiation image, and performs the registration of the first radiation image and the second radiation image based on the identified stacking order and the mark images included in the first radiation image and the second radiation image.

According to the twenty-eighth and twenty-ninth aspects, the image processing unit can generate a high-quality composite image while easily and appropriately performing the registration of the first radiation image and the second radiation image based on the stacking order of the first imaging plate and the second imaging plate and the mark images included in the first radiation image and the second radiation image read from the first imaging plate and the second imaging plate.

The processing device according to a thirtieth aspect is the processing device according to the twenty-ninth aspect, wherein the image processing unit identifies the stacking order based on a difference between an intensity of the mark image corresponding to the at least one overlapping portion mark included in the first radiation image and an intensity of the mark image corresponding to the at least one overlapping portion mark included in the second radiation image.

The processing device according to a thirty-first aspect is the processing device according to the twenty-ninth or thirtieth aspect, wherein the image processing unit identifies the stacking order based on a difference between a size of the mark image corresponding to the at least one overlapping portion mark included in the first radiation image and a size of the mark image corresponding to the at least one overlapping portion mark included in the second radiation image.

The processing device according to a thirty-second aspect is the processing device according to any one of the twenty-first to thirty-first aspects, further configured to read the plurality of radiation images from the plurality of imaging plates. In this case, it is possible to realize a processing device that can easily, in a short time, accurately, and appropriately perform a process for reliably generating an image of a subject with no clinical problems, thereby improving usability.

A program according to a thirty-third aspect is a program for causing a computer device to execute a process of performing registration of the plurality of radiation images read from the plurality of imaging plates covered by the covering member according to any one of the first to nineteenth aspects, based on a mark image included in the plurality of radiation images, and a process of combining the plurality of radiation images after the registration.

An image combination method according to a thirty-fourth aspect performs registration of the plurality of radiation images read from the plurality of imaging plates covered by the covering member according to any one of the first to nineteenth aspects, based on a mark image included in the plurality of radiation images, and combines the plurality of radiation images after the registration.

According to the thirty-third and thirty-fourth aspects, it becomes possible to easily and appropriately perform registration of a plurality of radiation images read from a plurality of imaging plates based on the mark images included in the plurality of radiation images.

### Reference Signs List

1 imaging plate assembly (IP assembly)
2, 2a, 2b imaging plate
4 waterproof bag (covering member)
5, 5A, 5B holding member (covering member)
6 protection cover (covering member)
8 covering bag (covering member)
10, 10a, 10b, 10c, 10d, 10e, 10f, 10g, 10h, 10i mark
28a, 28b corner portion
100 reading device (processing device)
280, 860 control unit (computer device)
280c program
281, 861 image processing unit
282, 862 display control unit
310 first radiation image
320 second radiation image
325 boundary image
330 third radiation image
350, 350a, 350b, 350ba, 350bb, 350c, 350d, 350da, 350db, 350e, 350f, 350g, 350ga, 350gb,
350ha, 350hb mark image
400 composite image
420 combined imaging plate
425 end portion
850 processing device

## Claims

1. A processing device, comprising:
an image processing unit,
wherein the image processing unit is configured to:
when a plurality of imaging plates used for intra-oral radiography are covered by a covering member placed in the oral cavity, the covering member comprising at least one mark used for registration when combining a plurality of radiation images respectively read from the plurality of imaging plates, and when the plurality of imaging plates are irradiated with radiation through the covering member, a mark image corresponding to the at least one mark is included in the plurality of radiation images,
perform registration of the plurality of radiation images, which are read from the plurality of imaging plates and include the mark image corresponding to the mark, based on the mark image, and
combine the plurality of radiation images after the registration.

2. The processing device according to claim 1, wherein
the plurality of imaging plates include a first imaging plate and a second imaging plate,
the first imaging plate and the second imaging plate are covered by the covering member in a state of being partially overlapped such that the first imaging plate is positioned above the second imaging plate,
the plurality of radiation images include a first radiation image and a second radiation image read from the first imaging plate and the second imaging plate, respectively,
the second radiation image includes a boundary image corresponding to a boundary between the first imaging plate and the second imaging plate on the second imaging plate, and
the image processing unit performs the registration of the first radiation image and the second radiation image based on the boundary image and the mark images included in the first radiation image and the second radiation image.

3. The processing device according to claim 1, wherein
the at least one mark includes at least one first identification mark for identifying, based on the mark image, a reading direction of the radiation image from each of the plurality of imaging plates, and
the image processing unit is configured to:
identify the reading direction of the radiation image from each of the plurality of imaging plates, based on the mark image corresponding to the at least one first identification mark included in the plurality of radiation images, and
perform the registration of the plurality of radiation images based on the identified reading direction and the mark images included in the plurality of radiation images.

4. The processing device according to claim 1, wherein
the at least one mark includes at least one second identification mark for identifying, based on the mark image, an anterior side or a posterior side of a composite image obtained by combining the plurality of radiation images, and
the image processing unit identifies the anterior side or the posterior side of the composite image obtained by the combination of the plurality of radiation images, based on the mark image corresponding to the at least one second identification mark included in the plurality of radiation images.

5. The processing device according to claim 4, further comprising:
a display control unit,
wherein the display control unit is configured to:
cause a display unit to display the composite image, and
determine a display orientation of the composite image based on the anterior side or the posterior side of the composite image identified by the image processing unit.

6. The processing device according to claim 1, wherein
the at least one mark includes at least one third identification mark for identifying, based on the mark image, an orientation of the plurality of imaging plates with respect to the covering member, and
the image processing unit identifies the orientation of the plurality of imaging plates with respect to the covering member, based on the mark image corresponding to the at least one third identification mark included in the plurality of radiation images.

7. The processing device according to claim 6, further comprising:
a display control unit,
wherein the display control unit is configured to:
cause a display unit to display a composite image obtained by the combination of the plurality of radiation images in the image processing unit, and
determine a display orientation of the composite image based on the orientation of the plurality of imaging plates with respect to the covering member, which is identified by the image processing unit.

8. The processing device according to claim 1, wherein
the plurality of imaging plates include a first imaging plate and a second imaging plate that are covered by the covering member in a state of being partially overlapped,
the at least one mark includes at least one fourth identification mark for identifying, based on the mark image, a stacking order of the first imaging plate and the second imaging plate,
the plurality of radiation images include a first radiation image and a second radiation image read from the first imaging plate and the second imaging plate included in the plurality of imaging plates, respectively, and
the image processing unit is configured to:
identify the stacking order of the first imaging plate and the second imaging plate, based on the mark image corresponding to the at least one fourth identification mark included in the first radiation image and the second radiation image, and
perform the registration of the first radiation image and the second radiation image based on the identified stacking order and the mark images included in the first radiation image and the second radiation image.

9. The processing device according to claim 1, wherein
the plurality of imaging plates include a first imaging plate and a second imaging plate that are covered by the covering member in a state of being partially overlapped,
the first imaging plate has a first overlapping portion that overlaps with the second imaging plate,
the second imaging plate has a second overlapping portion that overlaps with the first imaging plate,
the at least one mark includes at least one overlapping portion mark for recording a mark image on the first overlapping portion and the second overlapping portion,
the plurality of radiation images include a first radiation image and a second radiation image read from the first imaging plate and the second imaging plate included in the plurality of imaging plates, respectively, and
the image processing unit is configured to:
identify a stacking order of the first imaging plate and the second imaging plate, based on the mark image corresponding to the at least one overlapping portion mark included in the first radiation image and the second radiation image, and
perform the registration of the first radiation image and the second radiation image based on the identified stacking order and the mark images included in the first radiation image and the second radiation image.

10. The processing device according to claim 9, wherein
the image processing unit identifies the stacking order based on a difference between an intensity of the mark image corresponding to the at least one overlapping portion mark included in the first radiation image and an intensity of the mark image corresponding to the at least one overlapping portion mark included in the second radiation image.

11. The processing device according to claim 9, wherein
the image processing unit identifies the stacking order based on a difference between a size of the mark image corresponding to the at least one overlapping portion mark included in the first radiation image and a size of the mark image corresponding to the at least one overlapping portion mark included in the second radiation image.

12. The processing device according to claim 1, further configured to:
read the plurality of radiation images from the plurality of imaging plates.

13. An image combination method,
wherein, when a plurality of imaging plates used for intra-oral radiography are covered by a covering member placed in the oral cavity, the covering member comprising at least one mark used for registration when combining a plurality of radiation images respectively read from the plurality of imaging plates, and when the plurality of imaging plates are irradiated with radiation through the covering member, a mark image corresponding to the at least one mark is included in the plurality of radiation images,
the method comprises:
performing registration of the plurality of radiation images, which include the mark image corresponding to the mark, based on the mark image; and
combining the plurality of radiation images after the registration.

14. A program for causing a computer to execute the image combination method according to claim 13.

15. A computer-readable recording medium on which the program according to claim 14 is recorded.
